# EUROPEAN PATENT APPLICATION

(11) **EP 4 159 725 A1**
(43) Date of publication of application: **05.04.2023**
(21) Application number: 21812765.2
(22) Date of filing: 05.04.2021
(51) Int. Cl.: C07D 295/13, A61K 31/495, A61K 31/4439, A61P 25/00, A61P 25/18, C07D 211/18, C07D 401/04, A23L 33/10

(54) **CARBOXAMIDE DERIVATIVE AND PHARMACEUTICAL COMPOSITION COMPRISING SAME AS ACTIVE INGREDIENT FOR PREVENTING OR TREATING MENTAL ILLNESS**

(30) Priority: 29.05.2020 KR 20200065504; 31.03.2021 KR 20210041817
(71) Applicant: Daegu-Gyeongbuk Medical Innovation Foundation, Daegu 41061 (KR); Trineuro, Seoul 05029 (KR)
(72) Inventor: SONG, Minsoo, Daegu 41061 (KR); IM, Chun Young, Daegu 41061 (KR); PARK, Ga Young, Daegu 41061 (KR); KO, Eun Bi, Daegu 41061 (KR); KANG, Jihee, Daegu 41061 (KR); BAE, Seri, Daegu 41061 (KR); KIM, Soong-Hyun, Daegu 41061 (KR); PARK, Yoojin, Daegu 41061 (KR); LEE, Eunhye, Daegu 41061 (KR); CHOI, Yujeong, Daegu 41061 (KR); CHA, Eunju, Daegu 41061 (KR); SEO, Chang Hoon, Daegu 41061 (KR); SHIN, Chan Young, Seoul 05029 (KR); KWON, Kyoung Ja, Seoul 05029 (KR); MABUNGA, Darine Froy, Chungju-si Chungcheongbuk-do 27478 (KR); PARK, Donghyun, Chungju-si Chungcheongbuk-do 27478 (KR); HWANG, Hee Jong, Daegu 41106 (KR); KIM, Ryeong Eun, Chungju-si Chungcheongbuk-do 27478 (KR); PAUDEL, Suresh, Seoul 05029 (KR); JEON, Se Jin, Seoul 05029 (KR); CHO, Kyu Suk, Seoul 05029 (KR)
(74) Representative: EP&C
(86) International application number: PCT/KR2021/004209
(87) International publication number: WO 2021/241876

(57) **Abstract**

Disclosed are a carboxamide derivative and a pharmaceutical composition comprising same as an active ingredient for use in preventing or treating mental illness. The derivative is superbly effective in a triple reuptake inhibitor of serotonin, norepinephrine, and dopamine, and thus can be effectively used in treating mental illness.

## Description

### TECHNICAL FIELD

The present invention relates to a carboxamide derivative and a pharmaceutical composition for use in preventing or treating mental illness, containing same as an active ingredient.

### BACKGROUND ART

Mental illness refers to pathological abnormalities of the brain characterized by identifiable symptoms resulting in abnormalities of cognition, emotion, mood, or emotion. Representative mental illness includes attention deficit hyperactivity disorder (ADHD), panic disorder, bipolar disorder, depression, schizophrenia, eating disorder, dissociative disorder, posttraumatic stress disorder, etc. and these disorders are characterized by various dysfunctions, severity and duration of symptoms, etc. Factors that cause mental illness include: 1) brain secretion disorder caused by an excess or deficiency of neurotransmitters affecting cerebral nerve functions, 2) brain damage and degeneration of brain functions, 3) abnormal secretion of hormones, 4) stress, etc.

Numerous neurons constituting the brain regulate nerve functions by communicating with different neurons, and these neurons have two main transmission methods. One method is an electrical transmission, which is a method of transmission within neurons, and the other method is a chemical transmission, which is a method of extraneuronal transmission between neurons. The chemical transmission occurs at the synapse (the junction between the axon terminal of one neuron and the dendrite of the next neuron), and it is a method of secreting neurotransmitters (i.e., chemical substances) in the presynaptic process and transferring them to another synapse. As a postsynaptic process, the neuron which has received neurotransmitters through the synapse is activated and excited when the threshold is exceeded according to the amount of the neurotransmitters. The thus activated neuron generates an electrical signal in the dendrite and transmits the signal to the axon, and the axon secretes neurotransmitters through the synapse, and this whole process is repeated. Neurotransmitters secreted from neurons to regulate synaptic responses with neurons and eventually to control neural circuits are known to be the most important factors regulating human behavior.

When neurotransmitters are secreted from a neuron, specific receptors in a postsynaptic neuron forming a synapse with the neuron binds to the neurotransmitters to be activated, postsynaptic neurons and synaptic activation can be regulated depending on the signaling of receptors. Human behavior is presumed to be regulated by the regulation of activation of neurons and synapses by neurotransmitters, and mutations in genes of neurotransmitters (when the neurotransmitters are peptides), enzymes that produce neurotransmitters, or genes of neurotransmitter receptors have been reported to be associated with behavioral control disorders. Additionally, it is known that drugs that regulate the amount of neurotransmitters or signal transmission through receptors can regulate human behavior.

Neurotransmitters that regulate behavior as described above include monovalent amines (*e.g.*, catecholamine and serotonin), acetylcholine, excitatory or inhibitory amino acids, and various neuropeptides and receptors thereof are largely classified into ionotropic receptors, G-protein-coupled receptors, and tyrosine kinase receptors.

Among various neurotransmitters in the brain, dopamine, which is a catecholamine-based neurotransmitter, may be an excitatory signal or inhibitory signal depending on the type of postsynaptic dopamine receptors. Dopamine is associated with behavior, attention, learning, motivation, reward system, reinforcing effect, etc. in our body. When dopamine activity is reduced, depressive symptoms (*e.g*., lethargy, decreased motivation, decreased energy, etc.) appear. In addition, dopamine excites neurons in the frontal lobe and affects mental activities such as short-term memory, planning, and strategy. In particular, the reward given by dopamine plays a very important role in the construction of neural pathways in the early brain because the reward makes humans repeat the behavior and learning in order to feel the pleasure of this reward.

Norepinephrine, which is a catecholamine-based neurotransmitter found in the autonomic nervous system, is known as a secondary neurotransmitter of dopamine, which is secreted by the release action of dopamine. Norepinephrine is associated with arousal, which makes the body tense, pays attention and alerts to its surroundings, and involved in fear or stress response. In particular, norepinephrine is closely associated with emotions and is considered a cause of mood disorders (*e.g*., depression and bipolar disorder). That is, there is a hypothesis that depression is a state in which the function of norepinephrine or dopamine is reduced, and mania is a state in which the function of norepinephrine is excessively accelerated.

Serotonin, which is a neurotransmitter that usually acts in an inhibitory way, is associated with many basic human physiological phenomena (*e.g*., sleep, appetite, pain, regulation of body temperature, cardiovascular response, sexual desire, anxiety, depression, etc.). Clinically, serotonin is considered to be involved in the causes of various neuropsychiatric disorders (*e.g*., depression, schizophrenia, obsessive-compulsive disorder, anxiety disorder, eating disorder, sleep disorder, sexual disorder, impulse control disorder, developmental disorders, degenerative brain diseases, stress disorder, motor disorder, *etc*.), and in particular, the association with depression is very important.

Neurotransmitters are contained in synaptic vesicles of a neuron, and when stimulation is transferred to the neuron, calcium ion channels open before the synapse between neurons, leading to an influx of calcium into the neuron to move synaptic vesicles to the cell membrane of the neuron. The vesicles migrated to the cell membrane are released into the synapse by exocytosis at the axon terminal. The released neurotransmitters interact with the receptors to excite or inhibit the neuron that has received the neurotransmitters, and the remaining neurotransmitters after acting on the receptors are reuptaken at the axon terminal of the presynaptic neuron, degraded by degradation enzymes present in the postsynaptic membrane, or diffused outward and removed by glial cells.

The reuptake process occurs by a neurotransmitter transporter. Depending on the type of neurotransmitters, specific neurotransmitter transporters exist, which are present in the synaptic membrane and are responsible for reuptake of neurotransmitters present in the synaptic cleft into the presynaptic neuron. The regulation of the concentration of neurotransmitters through this process would ultimately affect the intensity and duration of activation of various receptors present in the postsynaptic neuron.

As described above, the reuptake of neurotransmitters refers to the process in which neurotransmitters are inactivated by the reuptake into the presynaptic neuron by specific neurotransmitter transporters in the membrane of the presynaptic neuron from which the neurotransmitters have been released. The increase in reuptake of neurotransmitters causes deficiency and imbalance of neurotransmitters, resulting in diseases associated with reuptake of neurotransmitters. Treatments for these reuptake-related diseases improve symptoms by increasing the concentration of neurotransmitters in the synapse by blocking the reuptake of these substances.

Neurotransmitter reuptake inhibitors include selective serotonin reuptake inhibitors, serotonin-norepinephrine reuptake inhibitors, selective norepinephrine reuptake inhibitors, dopamine-norepinephrine reuptake inhibitors, serotonin receptor antagonists-serotonin reuptake inhibitors, etc., and these inhibitors are used to treat depression, mood disorder, ADHD, chronic neuropathic pain, obsessive-compulsive disorder, panic disorder, anxiety disorder, menopausal symptoms, etc.

Drugs for treating mental illness are continuously under development, and among them, as related art associated with neurotransmitter reuptake inhibitors, Korean Patent Laid-open No. 10-2009-0089439 discloses chromen-2-one derivatives, a use thereof as monoamine neurotransmitter reuptake inhibitors, and a use for the treatment of mental illness, and Korean Patent Laid-open No. 10-2009-0117736, which is directed to a composition for selective serotonin reuptake inhibition and a method for producing the same, discloses a composition containing asiaticoside and madecassoside.

However, currently-used treatments for mental illness in the field of cranial nerves act merely on one nervous system among the various neurotransmitters suggested as related etiological mechanisms or have insufficient selectivity for neurotransmitters, and thus have many problems in therapeutic effects and compliance of patients, and cause serious adverse effects (e.g., growth retardation, sleep disorder, and dependence); therefore, there is a need to develop drugs that can flexibly regulate multiple targets with various differential selectivity for neurotransmitters.

In this regard, development on drugs that regulate multiple targets is in progress, in which Korean Patent Laid-open No. 10-2013-0026292, which is directed to novel azetidine derivatives and antidepressant composition containing the same, discloses an antidepressant composition containing an azetidine derivative capable of simultaneously inhibiting reuptake of dopamine, serotonin, and norepinephrine; Korean Patent Laid-open No. 2002-0079730 discloses a use of aryl- and heteroaryl-substituted tetrahydroisoquinolines to block reuptake of norepinephrine, dopamine, and serotonin; and Korean Patent Laid-open No. 10-2010019982, which is directed to phenyl substituted cycloalkylamines as monoamine reuptake inhibitors, discloses phenyl substituted cycloalkylamines which inhibit reuptake of endogenous monoamines, such as dopamine, serotonin and norepinephrine from the synaptic cleft and modulate one or more monoamine transporter.

The present inventors have made efforts to develop drugs that could flexibly regulate multiple targets by securing the differential selectivity of neuronal function control thereof, based on substances that simultaneously regulate dopamine, norepinephrine, and serotonin nervous system, and confirmed that a carboxamide derivative is excellent in the triple reuptake inhibitory effect of serotonin, norepinephrine, and dopamine, and thus can be useful in the treatment of mental illness, thereby completing the present invention.

### DISCLOSURE OF THE INVENTION

### TECHNICAL PROBLEM

One object of the present invention is to provide a carboxamide derivative.

Another object of the present invention is to provide a pharmaceutical composition for use in preventing or treating mental illness, containing a carboxamide derivative as an active ingredient.

Still another object of the present invention is to provide a triple reuptake inhibitor of serotonin, norepinephrine, and dopamine for use in preventing or treating mental illness, containing a carboxamide derivative as an active ingredient.

Still another object of the present invention is to provide a health functional food composition for use in preventing or improving mental illness, containing a carboxamide derivative as an active ingredient.

### TECHNICAL SOLUTION

In order to accomplish the objects,
an aspect of the present invention provides a compound represented by the following Formula 1, an isomer thereof, a solvate thereof, a hydrate thereof, or a pharmaceutically acceptable salt thereof:

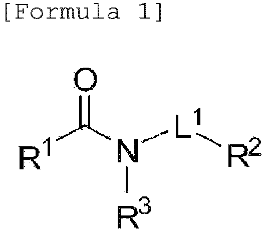

In Formula 1, R¹, R², R³ and L¹ are the same as defined in the description.

Another aspect of the present invention provides a pharmaceutical composition for use in preventing or treating mental illness, containing the compound represented by Formula 1 above, an isomer thereof, a solvate thereof, a hydrate thereof, or a pharmaceutically acceptable salt thereof as an active ingredient.

Still another aspect of the present invention provides a triple reuptake inhibitor of serotonin, norepinephrine, and dopamine for use in preventing or treating mental illness, containing the compound represented by Formula 1 above, an isomer thereof, a solvate thereof, a hydrate thereof, or a pharmaceutically acceptable salt thereof as an active ingredient.

Still another aspect of the present invention provides a health functional food composition for use in preventing or improving mental illness, containing the compound represented by Formula 1 above, an isomer thereof, a solvate thereof, a hydrate thereof, or a pharmaceutically acceptable salt thereof as an active ingredient.

Still another aspect of the present invention provides a method for use in preventing or treating mental illness, which includes administering a pharmaceutical composition or health functional food composition containing the compound represented by Formula 1 above, an isomer thereof, a solvate thereof, a hydrate thereof, or a pharmaceutically acceptable salt thereof as an active ingredient to a subject in need thereof.

Still another aspect of the present invention provides a use of a pharmaceutical composition or health functional food composition containing the compound represented by Formula 1 above, an isomer thereof, a solvate thereof, a hydrate thereof, or a pharmaceutically acceptable salt thereof as an active ingredient in the prevention or treatment of mental illness.

### ADVANTAGEOUS EFFECTS

The compound represented by Formula 1 according to the present invention is excellent in the triple reuptake inhibitory effect of serotonin, norepinephrine, and dopamine, and thus can be useful in the treatment of mental illness.

### BRIEF DESCRIPTION OF DRAWINGS

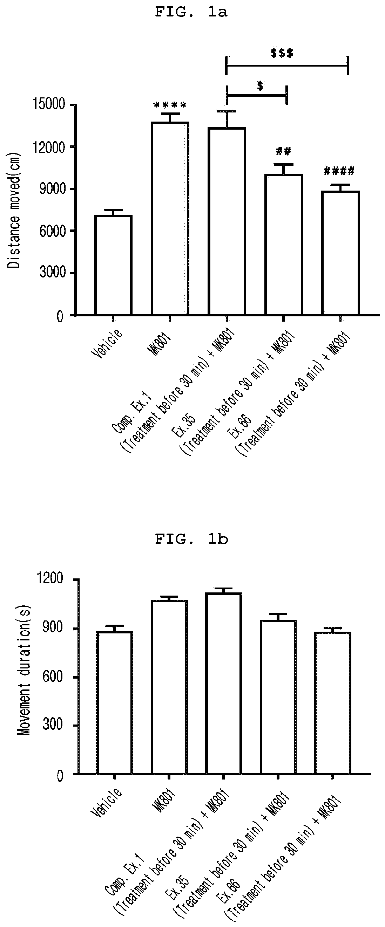

FIG. 1 shows graphs showing measured results of the behavior patterns of mice administered with drugs corresponding to a vehicle and each group for observing and analyzing the hyperactivity of the mice using an Ethovision System that is one type of an open field test, where graph a measured distance moved, and graph b measured movement duration.

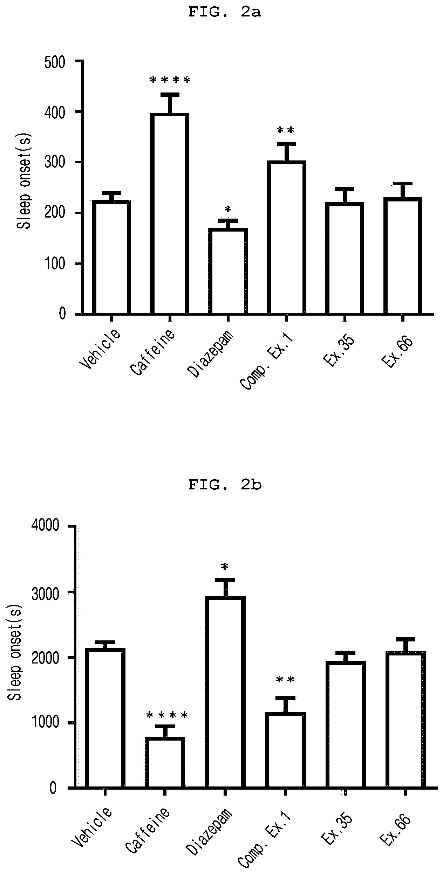

FIG. 2 shows graphs showing measured results of sleep onset time (graph a) and sleep duration (graph b) after inducing sleeping of mice administered with drugs corresponding to a vehicle and each group for evaluating the causing of sleep disorder or not, by intraperitoneal administration of pentobarbital sodium.

### MODE FOR CARRYING OUT THE INVENTION

Hereinafter, the present invention will be described in detail.

Meanwhile, the embodiments of the present invention may be modified in various forms, and the scope of the present invention is not limited to the embodiments described below. In addition, the embodiments of the present invention are provided to more completely explain the present invention to those skilled in the art. Furthermore, "including" a certain component throughout the specification means that other components may be further included instead of excluding other components unless otherwise stated.

An aspect of the present invention provides a compound represented by Formula 1, an isomer thereof, a solvate thereof, a hydrate thereof, or a pharmaceutically acceptable salt thereof. 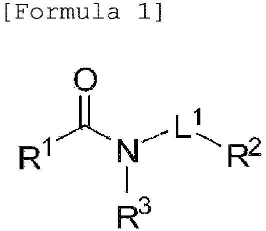 In Formula 1,
R¹ is unsubstituted or substituted cycloalkyl of C₃₋₆, unsubstituted or substituted aryl of C₆₋₁₀, unsubstituted or substituted heteroaryl of 5 to 14 atoms, containing one or more heteroatoms selected from the group consisting of N, S and O, substituted linear or branched C₁₋₈ alkyl, or substituted cycloalkenyl of C₄₋₆ to form aryl of C₆₋₁₀ together with substituted carbon atoms,
the substituted cycloalkyl is substituted with linear or branched C₁₋₈ alkyl, the substituted aryl is substituted with one or more substituents selected from the group consisting of linear or branched C₁₋₁₀ alkoxy and halogen, or substituted to form heterocycloalkenyl of C₃₋₆, containing one or more O together with substituted carbon atoms, the substituted heteroaryl is substituted with one or more substituents selected from the group consisting of unsubstituted or substituted linear or branched C₁₋₈ alkyl with one or more halogens, unsubstituted or substituted linear or branched C₁₋₁₀ alkoxy with aryl of C₆₋₁₀, C₁₋₁₀ carboxy, C₁₋₁₀ aminocarbonyl, benzyloxy, hydroxy, nitrile and halogen, or substituted to form heterocycloalkenyl of C₃₋₆, containing one or more O together with substituted carbon atoms, and the substituted alkyl is substituted with one or more substituents selected from the group consisting of substituted heterocycloalkyl with benzyl of 4 to 8 atoms, containing one or more heteroatoms selected from the group consisting of N, S and O, and oxo;
L¹ is a single bond, unsubstituted or substituted linear or branched C₁₋₈ alkylene or substituted cycloalkenylene of C4-6,
the substituted alkylene is substituted with one or more substituents selected from the group consisting of oxo and linear or branched C₁₋₅ alkyl, or the substituted alkylene is substituted to form cycloalkyl of C₃₋₆ together with substituted carbon, and the substituted cycloalkenylene is substituted to form aryl of C₆₋₁₀ together with substituted carbon atoms;
R² is unsubstituted or substituted heterocycloalkyl of 4 to 8 atoms, containing one or more heteroatoms selected from the group consisting of N, S and O, unsubstituted or substituted heteroaryl of 5 to1 14 atoms, containing one or more heteroatoms selected from the group consisting of N, S and O, substituted aryl of C₆₋₁₀ with one or more halogens, unsubstituted or substituted cycloalkyl of C₃₋₆, or NR^{2a}R^{2b},
the substituted heterocycloalkyl, substituted heteroaryl and substituted cycloalkyl are each independently substituted with one or more substituents selected from the group consisting of unsubstituted or substituted heteroaryl of 5 to 14 atoms with halogen, containing one or more heteroatoms selected from the group consisting of N, S and O, substituted linear or branched C₁₋₁₀ alkyl and substituted aryl of C₆₋₁₀, where the substituted alkyl is substituted with one or more substituents selected from the group consisting of heterocycloalkyl of 4 to 8 atoms, containing one or more heteroatoms selected from the group consisting of N, S and O, hydroxy and unsubstituted or substituted aryl of C₆₋₁₀ with one or more halogens, and the substituted aryl is substituted with one or more substituents selected from the group consisting of substituted linear or branched C₁₋₈ alkyl with one or more halogens and halogen,
in this case, R^{2a} and R^{2b} are each independently hydrogen, unsubstituted or substituted linear or branched C₁₋₅ alkyl, where the substituted alkyl is substituted with one or more substituents selected from the group consisting of oxo and aryl of C₆₋₁₀; and
R³ is hydrogen or linear or branched C₁₋₅ alkyl;
or if L¹ is a single bond, R² and R³ may form unsubstituted or substituted heterocycloalkyl of 4 to 8 atoms, containing one or more heteroatoms selected from the group consisting of N, S and O together with a nitrogen atom bonded, and the substituted heterocycloalkyl is substituted with substituted linear or branched C₁₋₁₀ alkyl with aryl of C₆₋₁₀.

In Formula 1,
R¹ is unsubstituted or substituted cycloalkyl of C₃₋₅, unsubstituted or substituted aryl of C₆₋₁₀, unsubstituted or substituted heteroaryl of 5 to 10 atoms, containing one or more heteroatoms selected from the group consisting of N, S and O, substituted linear or branched C₁₋₃ alkyl, or substituted cycloalkenyl of C₅₋₆ to form aryl of C₆₋₈ together with substituted carbon atoms,
the substituted cycloalkyl is substituted with linear or branched C₁₋₃ alkyl, the substituted aryl is substituted with one or more substituents selected from the group consisting of linear or branched C₁₋₅ alkoxy and halogen, or substituted to form heterocycloalkenyl of C₄₋₆ containing one or more O together with substituted carbon atoms, the substituted heteroaryl is substituted with one or more substituents selected from the group consisting of unsubstituted or substituted linear or branched C₁₋₃ alkyl with one or more halogens, unsubstituted or substituted linear or branched C₁₋₅ alkoxy with aryl of C₆₋₈, C₁₋₅ carboxy, C₁₋₅ aminocarbonyl, benzyloxy, hydroxy, nitrile and halogen, or substituted to form heterocycloalkenyl of C₄₋₆, containing one or more O together with substituted carbon atoms, and the substituted alkyl is substituted with one or more substituents selected from the group consisting of substituted heterocycloalkyl with benzyl of 4 to 6 atoms, containing one or more heteroatoms selected from the group consisting of N, S and O, and oxo;
L¹ is a single bond, unsubstituted or substituted linear or branched C₁₋₃ alkylene or substituted cycloalkenylene of C4-6,
the substituted alkylene is substituted with one or more substituents selected from the group consisting of oxo and linear or branched C₁₋₃ alkyl, or the substituted alkylene is substituted to form cycloalkyl of C₃₋₅ together with substituted carbon, and the substituted cycloalkenylene is substituted to form aryl of C₆₋₈ together with substituted carbon atoms;
R² is unsubstituted or substituted heterocycloalkyl of 4 to 6 atoms, containing one or more heteroatoms selected from the group consisting of N, S and O, unsubstituted or substituted heteroaryl of 5 to 10 atoms, containing one or more heteroatoms selected from the group consisting of N, S and O, substituted aryl of C₆₋₈ with one or more halogens, unsubstituted or substituted cycloalkyl of C₃₋₆, or NR^{2a}R^{2b},
the substituted heterocycloalkyl, substituted heteroaryl and substituted cycloalkyl are each independently substituted with one or more substituents selected from the group consisting of unsubstituted or substituted heteroaryl of 5 to 10 atoms with halogen, containing one or more heteroatoms selected from the group consisting of N, S and O, substituted linear or branched C₁₋₅ alkyl and substituted aryl of C₆₋₈, where the substituted alkyl is substituted with one or more substituents selected from the group consisting of heterocycloalkyl of 4 to 6 atoms, containing one or more heteroatoms selected from the group consisting of N, S and O, hydroxy and unsubstituted or substituted aryl of C₆₋₈ with one or more halogens, and the substituted aryl is substituted with one or more substituents selected from the group consisting of substituted linear or branched C₁₋₃ alkyl with one or more halogens and halogen,
in this case, R^{2a} and R^{2b} are each independently hydrogen, unsubstituted or substituted linear or branched C₁₋₃ alkyl, where the substituted alkyl is substituted with one or more substituents selected from the group consisting of oxo and aryl of C₆₋₁₀; and
R³ is hydrogen or linear or branched C₁₋₃ alkyl;
or if L¹ is a single bond, R² and R³ may form unsubstituted or substituted heterocycloalkyl of 4 to 6 atoms, containing one or more heteroatoms selected from the group consisting of N, S and O together with a nitrogen atom bonded, and the substituted heterocycloalkyl may be substituted with linear or branched C₁₋₅ alkyl which is substituted with aryl of C₆₋₈.

In Formula 1,
R¹ is unsubstituted or substituted cyclopropyl, unsubstituted or substituted phenyl, unsubstituted or substituted naphthyl, unsubstituted or substituted heteroaryl of 5 to 9 atoms, containing one or more heteroatoms selected from the group consisting of N, S and O, substituted linear or branched C₁₋₃ alkyl, or substituted cyclopentenyl to form phenyl together with substituted carbon atoms,
the substituted cyclopropyl is substituted with methyl, the substituted phenyl and the substituted phenyl are each independently substituted with one or more substituents selected from the group consisting of linear or branched C₁₋₄ alkoxy, chlorine and bromine, or the substituted phenyl is substituted to form heterocyclopentenyl containing one or more O together with substituted carbon atoms, the substituted heteroaryl is substituted with one or more substituents selected from the group consisting of unsubstituted or substituted methyl with one or more halogens, unsubstituted or substituted linear or branched C₁₋₄ alkoxy with phenyl, C₁₋₃ carboxy, C₁₋₃ aminocarbonyl, benzyloxy, hydroxy, nitrile and halogen, or substituted to form heterocycloalkenyl of C₅₋₆, containing one or more O together with substituted carbon atoms, and the substituted alkyl is substituted with one or more substituents selected from the group consisting of substituted piperidinyl with benzyl and oxo;
L¹ is a single bond, unsubstituted or substituted ethylene or substituted cyclohexenylene of C₄₋₆,
the substituted ethylene is substituted with one or more substituents selected from the group consisting of oxo and methyl, or the substituted ethylene is substituted to form cyclopropyl together with substituted carbon, and the substituted cyclohexenylene is substituted to form phenyl together with substituted carbon atoms;
R² is substituted piperidinyl, substituted piperazinyl, unsubstituted or substituted pyrazol, indole, substituted phenyl with one or more chlorine atoms, substituted cyclohexyl with benzyl, or NR^{2a}R^{2b},
the substituted piperidinyl, substituted piperazinyl and substituted pyrazole are each independently substituted with one or more substituents selected from the group consisting of indole, substituted pyridine with fluorine, substituted methyl and substituted phenyl, where the substituted methyl is substituted with one or more substituents selected from the group consisting of tetrahydropyran, hydroxy and unsubstituted or substituted phenyl with one or more halogens, and the substituted phenyl is substituted with one or more substituents selected from the group consisting of substituted methyl with one or more fluorine atoms and chlorine,
in this case, R^{2a} and R^{2b} are each independently hydrogen, or unsubstituted or substituted methyl, where the substituted methyl is substituted with one or more substituents selected from the group consisting of oxo and naphthyl; and
R³ is hydrogen;
or if L¹ is a single bond, R² and R³ may form piperidinyl together with a nitrogen atom bonded, and the substituted piperidinyl may be substituted with substituted phenyl.

In Formula 1,
R¹ is 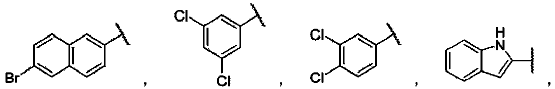 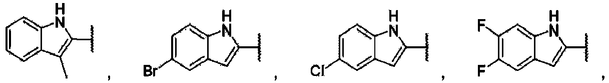 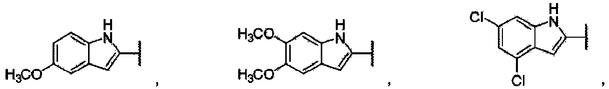 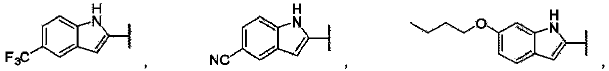 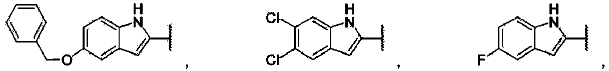 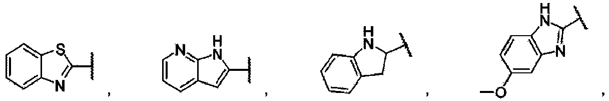 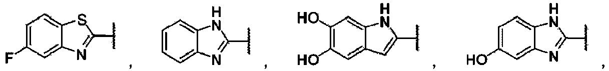 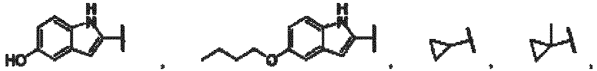 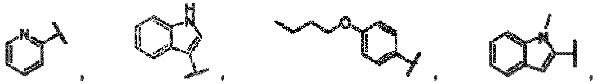 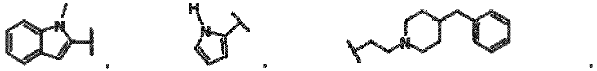 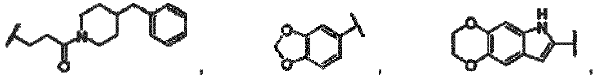 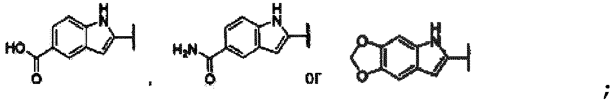
L¹ is a single bond, 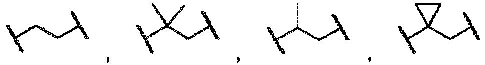 or 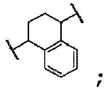
R² is 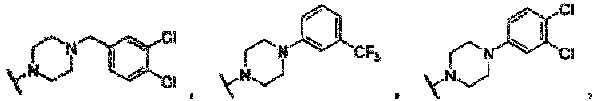 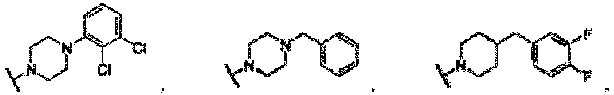 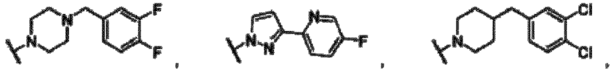 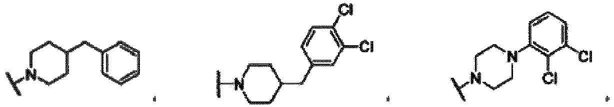 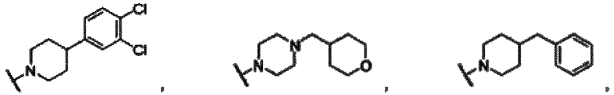 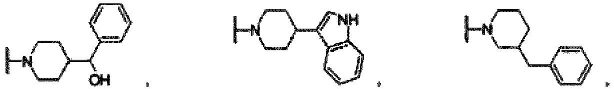 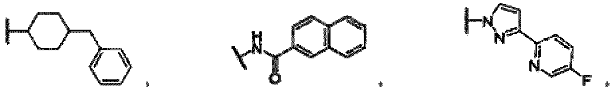 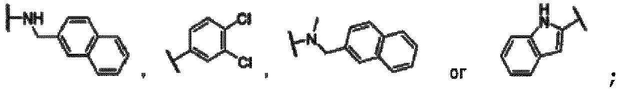 and
R³ is hydrogen; or
if L¹ is a single bond, -NR²R³ may be

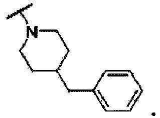

Examples of the compound represented by Formula 1 according to the present invention may include the following compound group:
<1> 6-bromo-N-(2-(4-(3,4-dichlorobenzyl)piperazin-1-yl)ethyl)-2-naphthamide;
<2> 6-bromo-N-(2-(4-(3-(trifluoromethyl)phenyl)piperazin-1-yl)ethyl)-2-naphthamide;
<3> 6-bromo-N-(2-(4-(3,4-dichlorophenyl)piperazin-1-yl)ethyl)-2-naphthamide;
<4> 6-bromo-N-(2-(4-(2,3-dichlorophenyl)piperazin-1-yl)ethyl)-2-naphthamide;
<5> N-(2-(4-benzylpiperazin-1-yl)ethyl)-6-bromo-2-naphthamide;
<6> 6-bromo-N-(2-(4-(3,4-difluorobenzyl)piperidin-1-yl)ethyl)-2-naphthamide;
<7> 6-bromo-N-(2-(4-(3,4-difluorobenzyl)piperazin-1-yl)ethyl)-2-naphthamide;
<8> 6-bromo-N-(2-(3-(5-fluoropyridin-2-yl)-1H-pyrazol-1-yl)ethyl)-2-naphthamide;
<9> 6-bromo-N-(2-(4-(3,4-dichlorobenzyl)piperidin-1-yl)ethyl)-2-naphthamide;
<10> 3,5-dichloro-N-(2-(4-(3,4-dichlorobenzyl)piperidin-1-yl)ethyl)benzamide;
<11> 3,4-dichloro-N-(2-(4-(3,4-dichlorobenzyl)piperazin-1-yl)ethyl)benzamide;
<12> 3,4-dichloro-N-(2-(4-(3-(trifluoromethyl)phenyl)piperazin-1-yl)ethyl)benzamide;
<13> 3,4-dichloro-N-(2-(4-(2,3-dichlorophenyl)piperazin-1-yl)ethyl)benzamide;
<14> 3,4-dichloro-N-(2-(4-(3,4-dichlorophenyl)piperazin-1-yl)ethyl)benzamide;
<15> N-(2-(4-benzylpiperazin-1-yl)ethyl)-3,4-dichlorobenzamide;
<16> 3,4-dichloro-N-(2-(4-(3,4-difluorobenzyl)piperazin-1-yl)ethyl)benzamide;
<17> 3,4-dichloro-N-(2-(4-(3,4-dichlorobenzyl)piperidin-1-yl)ethyl)benzamide;
<18> N-(2-(4-benzylpiperidin-1-yl)ethyl)-3,4-dichlorobenzamide;
<19> N-(2-(4-(3,4-dichlorobenzyl)piperidin-1-yl)ethyl)-1H-indol-2-carboxamide;
<20> N-(2-(4-benzylpiperidin-1-yl)ethyl)-3-methyl-1H-indol-2-carboxamide;
<21> N-(2-(4-(3-(trifluoromethyl)phenyl)piperazin-1-yl)ethyl)-1H-indol-2-carboxamide;
<22> N-(2-(4-(3,4-dichlorobenzyl)piperazin-1-yl)ethyl)-1H-indol-2-carboxamide;
<23> N-(2-(4-(2,3-dichlorophenyl)piperazin-1-yl)ethyl)-1H-indol-2-carboxamide;
<24> N-(2-(4-(3,4-dichlorophenyl)piperazin-1-yl)ethyl)-1H-indol-2-carboxamide;
<25> N-(2-(4-benzylpiperazin-1-yl)ethyl)-1H-indol-2-carboxamide;
<26> (4-benzylpiperidin-1-yl)(1H-indol-2-yl)methanone;
<27> N-(2-(4-(3,4-difluorobenzyl)piperidin-1-yl)ethyl)-1H-indol-2-carboxamide;
<28> N-(2-(4-(3,4-dichlorophenyl)piperidin-1-yl)ethyl)-1H-indol-2-carboxamide;
<29> N-(2-(4-((tetrahydro-2H-pyran-4-yl)methyl)piperazin-1-yl)ethyl)-1H-indol-2-carboxamide;
<30> N-(2-(4-benzylpiperidin-1-yl)ethyl)-5-bromo-1H-indol-2-carboxamide;
<31> N-(2-(4-benzylpiperidin-1-yl)ethyl)-5-chloro-1H-indol-2-carboxamide;
<32> N-(2-(4-benzylpiperidin-1-yl)ethyl)-5,6-difluoro-1H-indol-2-carboxamide;
<33> N-(2-(4-benzylpiperidin-1-yl)ethyl)-5-methoxy-1H-indol-2-carboxamide;
<34> N-(1-(4-benzylpiperidin-1-yl)-2-methylpropan-2-yl)-1H-indol-2-carboxamide;
<35> N-(2-(4-benzylpiperidin-1-yl)ethyl)-5,6-dimethoxy-1H-indol-2-carboxamide;
<36> N-(2-(4-(hydroxy(phenyl)methyl)piperidin-1-yl)ethyl)-1H-indol-2-carboxamide;
<37> N-(2-(4-(1H-indol-3-yl)piperidin-1-yl)ethyl)-1H-indol-2-carboxamide;
<38> N-(2-(3-benzylpiperidin-1-yl)ethyl)-1H-indol-2-carboxamide;
<39> N-(2-(4-benzylpiperidin-1-yl)ethyl)-4,6-dichloro-1H-indol-2-carboxamide;
<40> N-(2-(4-benzylpiperidin-1-yl)ethyl)-5-(trifluoromethyl)-1H-indol-2-carboxamide;
<41> N-(2-(4-benzylpiperidin-1-yl)ethyl)-5-cyano-1H-indol-2-carboxamide;
<42> N-(2-(4-benzylpiperidin-1-yl)ethyl)-6-butoxy-1H-indol-2-carboxamide;
<43> N-(1-(4-benzylpiperidin-1-yl)propan-2-yl)-1H-indol-2-carboxamide;
<44> N-(4-benzylcyclohexyl)-1H-indol-2-carboxamide;
<45> N-(2-(2-naphthamido)ethyl)-1H-indol-2-carboxamide;
<46> 5-(benzyloxy)-N-(2-(4-benzylpiperidin-1-yl)ethyl)-1H-indol-2-carboxamide;
<47> N-(2-(4-benzylpiperidin-1-yl)ethyl)-5,6-dichloro-1H-indol-2-carboxamide;
<48> N-(2-(4-(3,4-dichlorobenzyl)piperidin-1-yl)ethyl)-5,6-dimethoxy-1H-indol-2-carboxamide;
<49> N-(2-(4-benzylpiperidin-1-yl)ethyl)-5-fluoro-1H-indol-2-carboxamide;
<50> N-(2-(4-benzylpiperidin-1-yl)-2-oxoethyl)-1H-indol-2-carboxamide;
<51> N-(2-(4-(3,4-dichlorobenzyl)piperidin-1-yl)ethyl)-5-fluoro-1H-indol-2-carboxamide;
<52> N-(2-(3-(5-fluoropyridin-2-yl)-1H-pyrazol-1-yl)ethyl)-1H-indol-2-carboxamide;
<53> N-(1-((4-benzylpiperidin-1-yl)methyl)cyclopropyl)-1H-indol-2-carboxamide;
<54> N-(2-((naphthalen-2-ylmethyl)amino)ethyl)-1H-indol-2-carboxamide;
<55> N-(2-(4-benzylpiperidin-1-yl)ethyl)benzo[d]thiazol-2-carboxamide;
<56> N-(2-(4-benzylpiperidin-1-yl)ethyl)-1H-pyrrolol[2,3-b]pyridin-2-carboxamide;
<57> N-(2-(4-benzylpiperidin-1-yl)ethyl)indolin-2-carboxamide;
<58> (R)-N-(2-(4-benzylpiperidin-1-yl)ethyl)indolin-2-carboxamide;
<59> N-(2-(4-benzylpiperidin-1-yl)ethyl)-5-methoxy-1H-benzo[d]imidazol-2-carboxamide;
<60> N-(2-(4-benzylpiperidin-1-yl)ethyl)-5-fluorobenzo[d]thiazol-2-carboxamide;
<61> N-(2-(4-benzylpiperidin-1-yl)ethyl)-1H-benzo[d]imidazol-2-carboxamide;
<62> N-((1R,4S)-4-(3,4-dichlorophenyl)-1,2,3,4-tetrahydronaphthalen-1-yl)-1H-indol-2-carboxamide;
<63> N-(2-(4-benzylpiperidin-1-yl)ethyl)-5,6-dihydroxy-1H-indol-2-carboxamide;
<64> N-(2-(4-benzylpiperidin-1-yl)ethyl)-5-hydroxy-1H-benzo[d]imidazol-2-carboxamide;
<65> N-(2-(4-(3,4-dichlorobenzyl)piperidin-1-yl)ethyl)-5,6-dihydroxy-1H-indol-2-carboxamide;
<66> N-(2-(4-benzylpiperidin-1-yl)ethyl)-5-hydroxy-1H-indol-2-carboxamide;
<67> N-(2-(4-benzylpiperidin-1-yl)ethyl)-5-butoxy-1H-indol-2-carboxamide;
<68> N-(2-(methyl(naphthalen-2-ylmethyl)amino)ethyl)-1H-indol-2-carboxamide;
<69> N-(2-(4-benzylpiperidin-1-yl)ethyl)cyclopropanecarboxamide;
<70> N-(2-(4-benzylpiperidin-1-yl)ethyl)-1-methylcyclopropanecarboxamide;
<71> N-(2-(4-benzylpiperidin-1-yl)ethyl)picolinamide;
<72> N-(2-(4-(1H-indol-3-yl)piperidin-1-yl)ethyl)-4-butoxybenzamide;
<73> N-(2-(4-benzylpiperidin-1-yl)ethyl)-1H-indol-3-carboxamide;
<74> N-(2-(4-benzylpiperidin-1-yl)ethyl)-1-methyl-1H-indol-2-carboxamide;
<75> N-(2-(4-benzylpiperidin-1-yl)ethyl)-1H-pyrrol-2-carboxamide;
<76> 3-(4-benzylpiperidin-1-yl)-N-(1H-indol-2-yl)propanamide;
<77> 4-(4-benzylpiperidin-1-yl)-N-(1H-indol-2-yl)-4-oxobutanamide;
<78> N-(2-(4-benzylpiperidin-1-yl)ethyl)benzo[d] [1,3]dioxol-5-carboxamide;
<79> N-(2-(4-benzylpiperidin-1-yl)ethyl)-2,3-dihydro-6H-[1,4]dioxyno[2,3-f]indol-7-carboxamide;
<80> N-(2-(4-benzylpiperidin-1-yl)ethyl)-5H-[1,3]dioxo[4,5-f]indol-6-carboxamide;
<81> 2-((2-(4-benzylpiperidin-1-yl)ethyl)carbamoyl)-1H-indol-5-carboxylic acid; and
<82> N2-(2-(4-benzylpiperidin-1-yl)ethyl)-1H-indol-2,5-dicarboxamide.

The compound represented by Formula 1 of the present invention can be used in the form of a pharmaceutically acceptable salt, and an acid addition salt formed by a pharmaceutically acceptable free acid is useful as the salt. The acid addition salt is obtained from inorganic acids (*e.g*., hydrochloric acid, nitric acid, phosphoric acid, sulfuric acid, hydrobromic acid, hydroiodic acid, nitrous acid, phosphorous acid, *etc*.); non-toxic organic acids (*e.g*., aliphatic mono- and di-carboxylates, phenyl-substituted alkanoates, hydroxy alkanoates and alkanedioates, aromatic acids, aliphatic and aromatic sulfonic acids, etc.; organic acids (*e.g*., trifluoroacetic acid, acetate, benzoic acid, citric acid, lactic acid, maleic acid, gluconic acid, methanesulfonic acid, 4-toluenesulfonic acid, tartaric acid, fumaric acid, etc.). These types of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, maleate, tartrate, methanesulfonate, propanesulfonate, naphthalen-1-sulfonate, naphthalen-2-sulfonate, mandelate, etc.

The acid addition salt according to the present invention may be prepared by a conventional method, for example, may be prepared by dissolving the derivative of Formula 1 in an organic solvent (e.g., methanol, ethanol, acetone, methylene chloride, acetonitrile, etc.), and adding an organic or inorganic acid to filter and dry the resultant precipitate; or may be prepared by distilling the solvent and excess acid under reduced pressure, drying, and crystallizing in an organic solvent.

In addition, a pharmaceutically acceptable metal salt may be prepared using a base. An alkali metal or alkaline earth metal salt is obtained, for example, by dissolving a compound in an excess alkali metal hydroxide or alkaline earth metal hydroxide solution, filtering the undissolved compound salt, and evaporating and drying the filtrate. In this case, it is pharmaceutically suitable to prepare a sodium, potassium, or calcium salt as the metal salt. In addition, the corresponding salt is obtained by reacting an alkali metal or alkaline earth metal salt with a suitable negative salt (*e.g*., silver nitrate).

Furthermore, the present invention includes not only the compound represented by Formula 1 and a pharmaceutically acceptable salt thereof, but also solvates, optical isomers, hydrates, etc., which can be prepared therefrom.

The term "hydrate" refers to a compound of the present invention, which contains a stoichiometric or non-stoichiometric amount of water bound by a non-covalent intermolecular force, or a salt thereof. The hydrate of the compound represented by Formula 1 of the present invention may contain a stoichiometric or non-stoichiometric amount of water bound by a non-covalent intermolecular force. The hydrate may contain 1 equivalent or more, preferably 1-5 equivalents of water. Such a hydrate may be prepared by crystallizing the compound represented by Formula 1 of the present invention, an isomer thereof, or a pharmaceutically acceptable salt thereof of the present invention from water or a water-containing solvent.

The term "solvate" refers to the compound of the present invention or a salt thereof that contains a stoichiometric or non-stoichiometric amount of a solvent bound by a non-covalent intermolecular force. Preferred solvents as such include solvents that are volatile, non-toxic, and/or suitable for administration to humans.

The term "isomer" refers to compounds of the present invention or salts thereof that have the same chemical formula or molecular formula but differ structurally or sterically. These isomers include structural isomers (*e.g*., tautomers, *etc*.), R or S isomers having an asymmetric carbon center, stereoisomers (*e.g*., geometric isomers (*trans, cis*)), and optical isomers (enantiomers). All of these isomers and mixtures thereof are also included within the scope of the present invention.

The compound represented by Formula 1 according to the present invention may be prepared, as shown in Reaction 1 below,
may be prepared according to a preparation method which includes preparing a compound represented by Formula 1 by reacting a compound represented by Formula 2 with a compound represented by Formula 3.

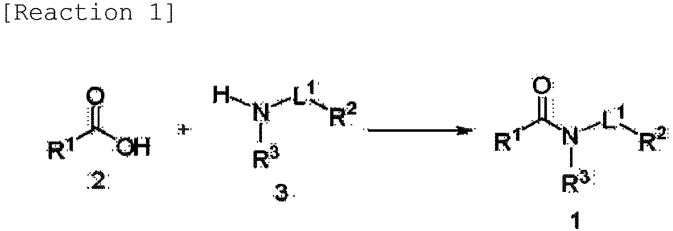

In Formula 1, R¹, R², R³ and L¹ are the same as defined in the present disclosure.

The preparation method of Reaction 1 is a method of reacting the carboxy of the compound represented by Formula 2 and the amine of the compound represented by Formula 3 to form a carboxamide bond and to prepare the compound represented by Formula 1. The preparation method may use reaction conditions well-known in the art and the preparation may be performed according to the Examples of the present invention, but this is only an illustration and is not limited thereto.

Another aspect of the present invention provides a pharmaceutical composition for use in preventing or treating mental illness, containing the compound represented by Formula 1, an optical isomer thereof or a pharmaceutically acceptable salt thereof, as an active ingredient.

The compound can inhibit the reuptake of serotonin, norepinephrine, and dopamine.

The mental illness may be at least one selected from the group consisting of bulimia nervosa, mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substance-induced mood disorder, pseudo dementia, Ganger syndrome, obsessive-compulsive disorder, panic disorder, panic disorder without agoraphobia, panic disorder with agoraphobia, agoraphobia without history of panic disorder, panic attacks, memory deficit, memory loss, attention deficit hyperactivity disorder, obesity, anxiety, generalized anxiety disorder, eating disorder, Parkinson's disease, Parkinson's symptoms, dementia, aging dementia, senile dementia, Alzheimer's disease, Down syndrome, acquired immunodeficiency syndrome dementia complex, memory dysfunction in aging, specific phobia, social phobia, social anxiety disorder, post-traumatic stress disorder, acute stress disorder, chronic stress disorder, drug addiction, drug abuse, drug abuse tendency, cocaine abuse, nicotine abuse, tobacco abuse, alcohol addiction, alcoholism, pathological kleptomaniac, withdrawal syndrome due to withdrawal of intoxicating substances, pain, chronic pain, inflammatory pain, neuropathic pain, diabetic neuropathic pain, migraine, tension-type headache, chronic tension-type headache, depression-related pain, fibromyalgia, arthritis, osteoarthritis, rheumatoid arthritis, back pain, cancer pain, irritable bowel pain, irritable bowel syndrome, postoperative pain, postmastectomy pain syndrome (PMPS), poststroke pain, drug-induced neuropathy, diabetic neuropathy, pain sustained by the sympathetic nervous system, trigeminal neuralgia, toothache, facial muscle pain, phantom limb pain, anorexia, premenstrual syndrome, premenstrual dysphoric disorder, late luteal phase syndrome, posttraumatic syndrome, chronic fatigue syndrome, persistent vegetative state, urinary incontinence, stress incontinence, urge incontinence, nocturnal incontinence, sexual dysfunction, premature ejaculation, erectile difficulty, erectile dysfunction, female premature orgasm, restless legs syndrome, periodic limb movement disorder, eating disorder, anorexia nervosa, sleep disorder, pervasive developmental disorder, autism, Asperger's disorder, Rett disorder, childhood disintegrative disorder, learning disorder, motor ability disorder, mutism, trichotillomania, narco-lepsy, post-stroke depression, stroke-induced brain injury, stroke-induced nerve injury, Tourette syndrome, tinnitus, tic disorder, body dysmorphic disorder, oppositional defiant disorder, and post-stroke disorder.

The compound represented by Formula 1 according to the present invention is excellent in the reuptake inhibitory effect of serotonin, norepinephrine, and dopamine, and thus can be useful in the treatment of mental illness (see Experimental Example 1 and Table 15).

In an experimental embodiment of the present invention, it was confirmed that the hyperactivity of mice induced by MK801 was not inhibited by the treatment with the compound of Comparative Example 1, but was significantly inhibited by the treatment with the compound of Example 35 or 66 (see Experimental Example 2 and FIG. 1).

In an experimental embodiment of the present invention, it was confirmed that the sleep onset time of mice was increased, sleep duration was decreased by the treatment with the compound of Comparative Example 1, and sleep disorder wash shown, but no change was induced on the sleep onset time and the sleep duration, and sleep disorder was not shown by the treatment with the compound of Example 35 or 66 (see Experimental Example 3 and FIG. 2).

In the pharmaceutical composition according to the present invention, the compound represented by Formula 1 above or a pharmaceutically acceptable salt thereof may be administered in various oral and parenteral dosage forms during clinical administration, and more preferably, parenteral dosage forms. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, etc., and such solid formulations are prepared by mixing at least one excipient, for example, starch, calcium carbonate, sucrose or lactose, gelatin, *etc*. with one or more compounds. In addition to simple excipients, lubricants such as magnesium stearate and talc are also used. Liquid formulations for oral administration include suspensions, solutions for internal use, emulsions, syrups, *etc*., and in addition to water and liquid paraffin, which are commonly used simple diluents, various excipients such as wetting agents, sweeteners, fragrances, and preservatives may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (*e.g*., olive oil), injectable esters (*e.g*., ethyl oleate), *etc*. may be used.

The compound represented by Formula 1 or a pharmaceutically acceptable salt thereof may be administered in various oral and parenteral dosage form during clinical administration. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, *etc*., and such solid formulations are prepared by mixing at least one excipient, for example, starch, calcium carbonate, sucrose or lactose, gelatin, *etc*. with one or more compounds. In addition to simple excipients, lubricants such as magnesium stearate and talc are also used. Liquid formulations for oral administration include suspensions, solutions for internal use, emulsions, syrups, *etc*., and in addition to water and liquid paraffin, which are commonly used simple diluents, various excipients such as wetting agents, sweeteners, fragrances, and preservatives may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (*e.g*., olive oil), injectable esters (*e.g*., ethyl oleate), *etc*. may be used.

The pharmaceutical composition containing the compound represented by Formula 1 or a pharmaceutically acceptable salt thereof, as an active ingredient may be administered parenterally, and the parenteral administration is performed by subcutaneous injection, intravenous injection, intramuscular injection, or intrathoracic injection.

In this case, to be formulated into a dosage form for parenteral administration, the compound represented by Formula 1 above or a pharmaceutically acceptable salt thereof may be mixed in water along with a stabilizer or buffer to prepare a solution or suspension, which may be prepared in unit dosage form in an ampoule or vial. The composition may be sterilized and/or contain preservatives, stabilizers, wetting agents or emulsification accelerators, salts and/or buffers for the control of osmotic pressure, and other therapeutically useful substances, and may be formulated according to the conventional method such as mixing, granulation, or coating method.

Dosage form for oral administration include, for example, tablets, pills, hard/soft capsules, liquids, suspensions, emulsifiers, syrups, granules, elixirs, troches, etc., and these dosage forms contain diluents (*e.g*., lactose, dextrose, sucrose, mannitol, sorbitol, cellulose and/or glycine), lubricants (*e.g*., silica, talc, stearic acid and a magnesium or calcium salt thereof and/or polyethylene glycol) in addition to active ingredients. Tablets may contain binders (*e.g*., magnesium aluminum silicate, starch paste, gelatin, methylcellulose, sodium carboxymethylcellulose and/or polyvinylpyrrolidine); optionally contain disintegrants (*e.g*., starch, agar, alginic acid or a sodium salt thereof) or effervescent mixtures, and/or absorbents, colorants, flavors, and sweeteners.

The pharmaceutical composition for use in preventing or treating mental illness containing the compound represented by Formula 1, an optical isomer thereof or pharmaceutically acceptable salt thereof, as an active ingredient may be administered as an individual therapeutic agent or used in combination with other therapeutic agents in use.

Still another aspect of the present invention provides a triple reuptake inhibitor of serotonin, norepinephrine, and dopamine for use in preventing or treating mental illness, containing the compound represented by Formula 1 above, an isomer thereof, a solvate thereof, a hydrate thereof, or a pharmaceutically acceptable salt thereof, as an active ingredient.

Yet another aspect of the present invention provides a health functional food composition for use in preventing or improving mental illness containing the compound represented by Formula 1 above, an isomer thereof, a solvate thereof, a hydrate thereof, or a pharmaceutically acceptable salt thereof, as an active ingredient.

Further another aspect of the present invention provides a method for use in preventing or treating mental illness, the method including administering a pharmaceutical composition or health functional food composition containing the compound represented by Formula 1 above, an isomer thereof, a solvate thereof, a hydrate thereof, or a pharmaceutically acceptable salt thereof, as an active ingredient to a subject in need thereof.

Yet still another aspect of the present invention provides a use of a pharmaceutical composition or health functional food composition containing the compound represented by Formula 1 above, an isomer thereof, a solvate thereof, a hydrate thereof, or a pharmaceutically acceptable salt thereof, as an active ingredient in the prevention or treatment of mental illness.

### MODES FOR CARRYING OUT THE INVENTION

Hereinafter, the present invention will be described in detail by Examples and Experimental Examples.

However, the following Examples and Experimental Examples are merely illustrative of the present invention, and the contents of the present invention are not limited to the Examples and Experimental Examples.

### <Preparation Example 1> Preparation of 2-(4-benzylpiperidin-1-yl)ethan-1-amine

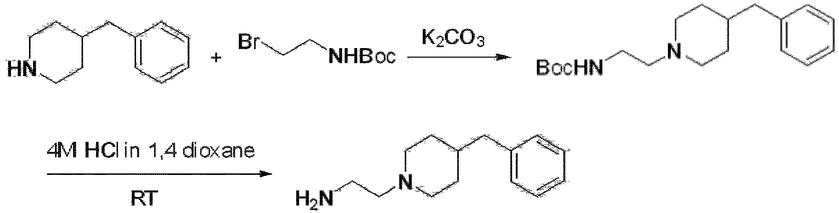

Step 1: 4-Benzylpiperidine (1 eq) and K₂CO₃ (1.5 eq) were dissolved in DMF, followed by stirring for about 5 minutes, and then, tert-butyl-(2-bromoethyl)carbamate (1.2 eq) was added. The reaction mixture was stirred at 60°C for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain tert-butyl 2-(4-benzylpiperidin-1-yl)ethyl)carbamate.

Step 2: The tert-butyl 2-(4-benzylpiperidin-1-yl)ethyl)carbamate (1 eq) of Step 1 was dissolved in an excessive amount of a 4 M HCl in 1,4-dioxane, and the reactant was stirred at room temperature for 1-2 hours. After finishing the reaction, the resultant was extracted with dichloromethane, an organic layer was washed with a NaHCO₃ saturated aqueous solution and brine in order, and dried over anhydrous Na₂SO₄, and the solvent was removed under a reduced pressure. Then, the reaction mixture was separated and purified by MPLC to obtain the compound of Preparation Example 1.

The compounds of Preparation Examples 2 to 14 were obtained by the same method as Preparation Example 1 except for using R listed in Table 1 instead of 4-benzylpiperidine in Step 1.

**[Table 1]**

| Preparation Example | R | Structure |
|---|---|---|
| 2 | 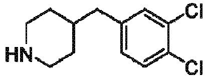 | 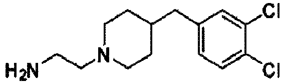 |
| 3 | 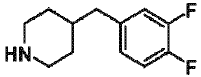 | 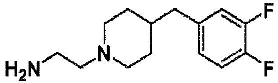 |
| | HCl salt form | |
| 4 | 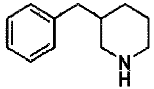 | 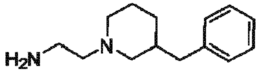 |
| 5 | 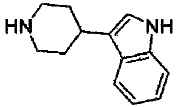 | 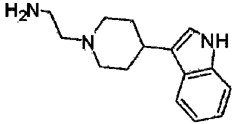 |
| 6 | 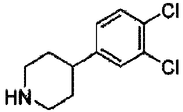 | 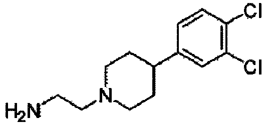 |
| | HCl salt form | |
| 7 | 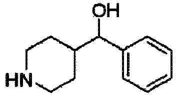 | 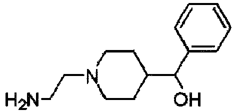 |
| 8 | 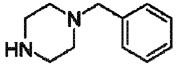 | 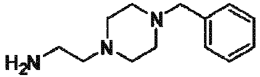 |
| | HCl salt form | |
| 9 | 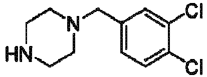 | 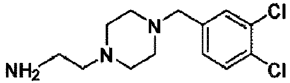 |
| 10 | 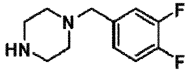 | 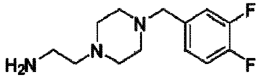 |
| 11 | 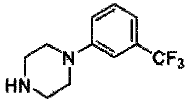 | 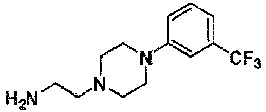 |
| 12 | 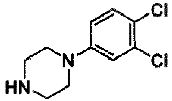 | 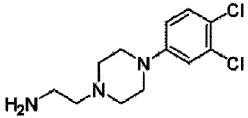 |
| 13 | 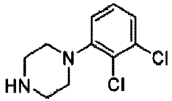 | 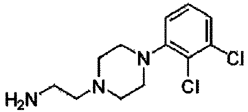 |
| 14 | 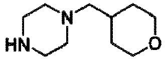 | 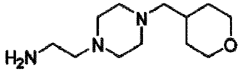 |

### <Preparation Example 15> Preparation of N-(2-aminoethyl)-2-naphthamide

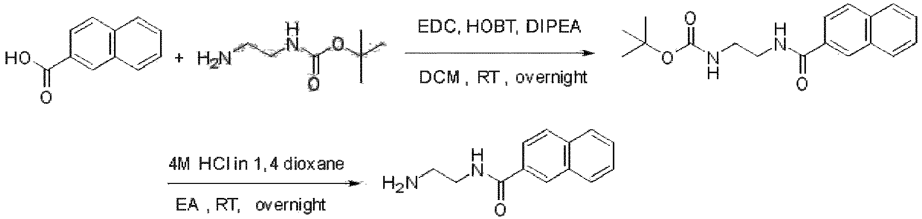

Step 1: 2-Naphthoic acid (1 eq), tert-butyl (2-aminoethyl) carbamate (1 eq), EDC (2.5 eq), HOBT (1.5 eq) and DIPEA (3 eq) were dissolved in DCM, and the reactants were stirred at room temperature for 12 hours. After finishing the reaction, the resultant was extracted with dichloromethane, an organic layer was washed with a NaHCO₃ saturated aqueous solution and brine in order, and dried over anhydrous Na₂SO₄, and the solvent was removed under a reduced pressure. Then, the reaction mixture was separated and purified by MPLC to obtain tert-butyl (2-(2-naphthamido)ethyl)carbamate.

Step 2: The tert-butyl (2-(2-naphthamido)ethyl)carbamate (1 eq) obtained in Step 1 and 4 M HCl in 1,4-dioxane (3.25 eq) were dissolved in EA, and the reactants were stirred at room temperature for 12 hours. After finishing the reaction, the solvent was removed under a reduced pressure. The product was not subjected to additional purification to obtain the compound of Preparation Example 15.

### <Preparation Example 16> Preparation of 1-(4-benzylpiperidin-1-yl)-2-methylpropan-2-amine

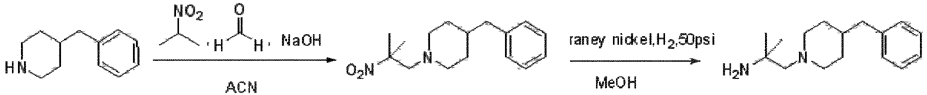

Step 1: 4-Benzylpiperidine (1 eq) and 2-nitropropane (1 eq) were dissolved in ACN, and the reactants were cooled to 0°C. Then, formaldehyde (3 eq) and 0.5 M NaOH (0.1 eq) was added thereto, and the reaction mixture was stirred at 50°C for 2 hours. Formaldehyde (3 eq) and 0.5 M NaOH (0.1 eq) were added thereto, and the reactants were stirred at 50°C for 12 hours. After finishing the reaction, the solvent was removed under a reduced pressure, and the reaction mixture was separated and purified by MPLC to obtain 4-benzyl-1-(2-methyl-2-nitropropyl)piperidine.

Step 2: The 4-benzyl-1-(2-methyl-2-nitropropyl)piperidine obtained in Step 1 was dissolved in MeOH, and the reaction mixture was stirred under raney nickel cartridge and 50 psi conditions for 12 hours using a H-CUBE hydrogenation reactor. After finishing the reaction, the solvent was removed under a reduced pressure. The product was not subjected to additional purification to obtain the compound of Preparation Example 16.

### <Preparation Example 17> Preparation of 1-(4-benzylpiperidin-1-yl)propan-2-amine

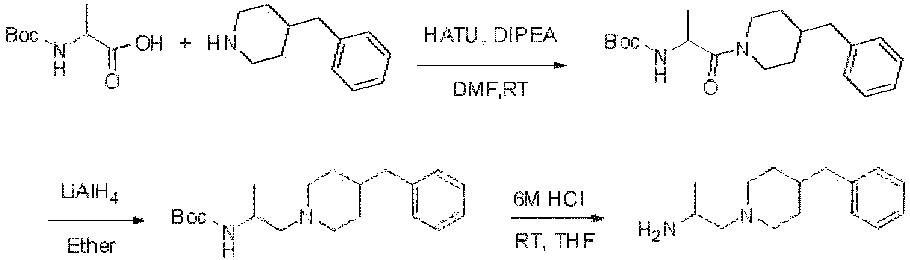

Step 1: (Tert-butoxycarbonyl)alanine (1 eq), 4-benzylpiperidine (1 eq), HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain tert-butyl-(1-(4-benzylpiperidin-1-yl)-1-oxopropan)carbamate.

Step 2: The tert-butyl-(1-(4-benzylpiperidin-1-yl)-1-oxopropan)carbamate (1 eq) obtained in Step 1 was dissolved in ether, and the reactant was cooled to 0°C. Then, LiAlH₄ (2 eq) was slowly added thereto, and the reactants were stirred under a nitrogen balloon stream at room temperature for 4 hours. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain tert-butyl-(1-(4-benzylpiperidin-1-yl)propan-2-yl)carbamate.

Step 3: The tert-butyl-(1-(4-benzylpiperidin-1-yl)propan-2-yl)carbamate (1 eq) obtained in Step 2 was dissolved in an excessive amount of 6 M HCl, followed by stirring at room temperature for 12 hours. After finishing the reaction, the solvent was removed under a reduced pressure. The product was not subjected to additional purification to obtain the compound of Preparation Example 17.

### <Example 1> Preparation of 6-bromo-N-(2-(4-(3,4-dichlorobenzyl)piperazin-1-yl)ethyl)-2-naphthamide

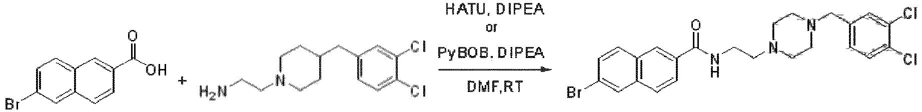

6-Bromo-2-naphthoic acid (1 eq), 2-(4-(3,4-dichlorobenzyl)piperidin-1-yl)ethan-1-amine (1.2 eq), DIPEA (3 eq) and HATU (2.5 eq) or PyBOB (1.1 eq) were dissolved in DMF, and the reactants were stirred at room temperature for 12 hours. After finishing the reaction, the solvent was removed under a reduced pressure. The resultant was extracted with dichloromethane, an organic layer was washed with a H₂O and brine in order, and dried over anhydrous Na₂SO₄, and the solvent was removed under a reduced pressure. Then, the reaction mixture was separated and purified by MPLC to obtain the compound of Example 1.

The compounds of Examples 2 to 7 were obtained by the same method as Example 1 except for using R listed in Table 2 instead of the 2-(4-(3,4-dichlorobenzyl)piperidin-1-yl)ethan-1-amine.

**[Table 2]**

| Example | R | Structure |
|---|---|---|
| 2 | 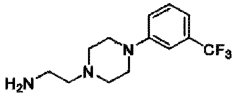 | 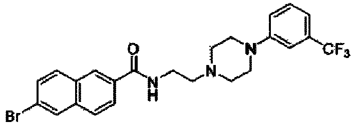 |
| 3 | 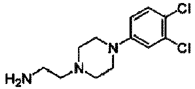 | 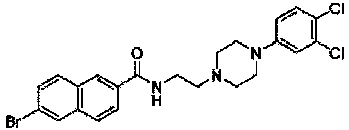 |
| 4 | 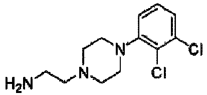 | 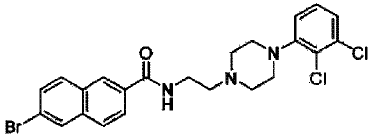 |
| 5 | 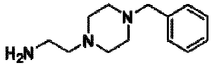 | 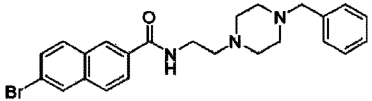 |
| 6 | 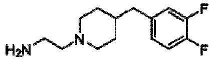 | 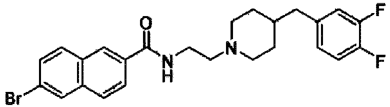 |
| 7 | 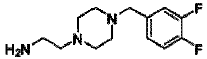 | 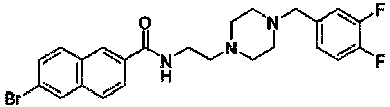 |

### <Example 8> Preparation of 6-bromo-N-(2-(3-(5-fluoropyridin-2-yl)-1H-pyrazol-1-yl)ethyl)-2-naphthamide

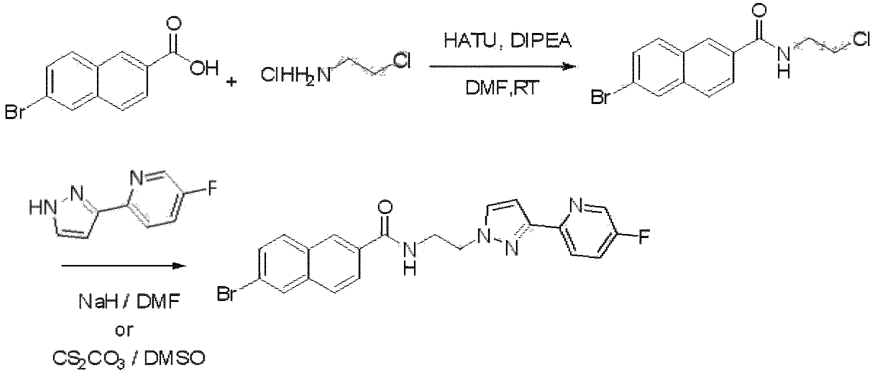

Step 1: 6-Bromo-2-naphthoic acid (1 eq), 2-chloroethan-1-amine hydrochloride (1.2 eq), HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain 6-bromo-N-(2-chloroethyl)-2-naphthamide.

Step 2: 5-Fluoro-2-(1H-pyrazol-3-yl)pyridine (1-1.5 eq) was dissolved in DMF or DMSO, and NaH (60% oil dispersion; 1.2 eq) or CS₂CO₃ (1.5 eq) was added thereto at 0°C. After stirring at room temperature for 5 minutes, the 6-bromo-N-(2-chloroethyl)-2-naphthamide (1 eq) prepared in Step 1 was added, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 8.

The compound of Example 9 was obtained by the same method as Example 8 except for using R listed in Table 3 instead of the 5-fluoro-2-(1H-pyrazol-3-yl)pyridine obtained in Step 1.

**[Table 3]**

| Example | R | Structure |
|---|---|---|
| 9 | 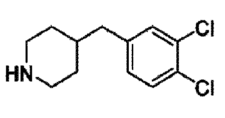 | 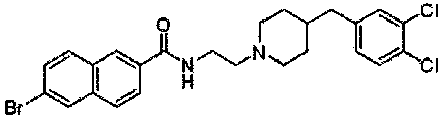 |

### <Example 10> Preparation of 3,5-dichloro-N-(2-(4-(3,4-dichlorobenzyl)piperidin-1-yl)ethyl)benzamide

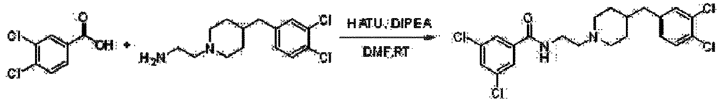

3,4-Dichlorobenzoic acid (1 eq), 2-(4-(3,4-dichlorobenzyl)piperidin-1-yl)ethan-1-amine (1.2 eq), HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 10.

The compounds of Examples 11 to 16 were obtained by the same method as Example 10 except for using 3,5-dichlorobenzoic acid instead of the 3,4-dichlorobenzoic acid and using R listed in Table 4 instead of the 2-(4-(3,4-dichlorobenzyl)piperidin-1-yl)ethan-1-amine.

**[Table 4]**

| Example | R | Structure |
|---|---|---|
| 11 | 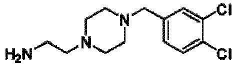 | 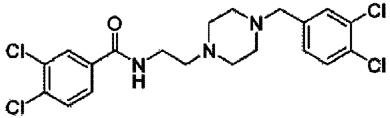 |
| 12 | 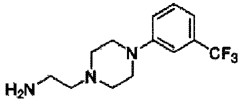 | 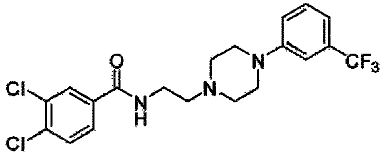 |
| 13 | 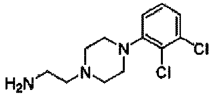 | 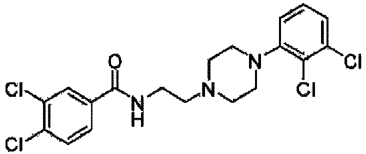 |
| 14 | 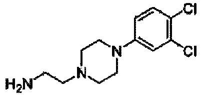 | 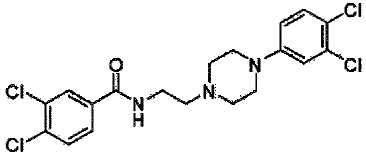 |
| 15 | 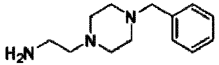 | 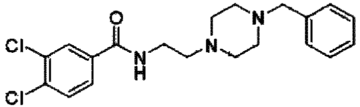 |
| 16 | 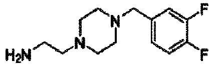 | 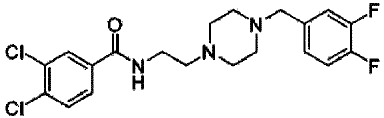 |

### <Example 17> Preparation of 3,4-dichloro-N-(2-(4-(3,4-dichlorobenzyl)piperidin-1-yl)ethyl)benzamide

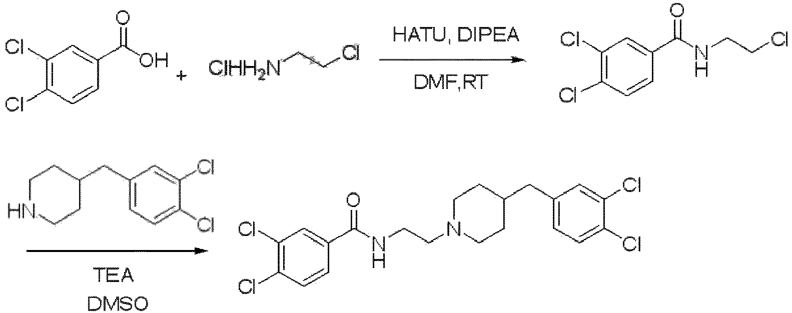

Step 1: 3,4-Dichlorobenzoic acid (1 eq), 2-chloroethan-1-amine hydrochloride (1.2 eq), HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain 3,4-dichloro-N-(2-chloroethyl)benzamide.

Step 2: 4-(3,4-Dichlorobenzyl)piperidine (1-1.5 eq) and TEA (4 eq) were dissolved in DMF, 3,4-dichloro-N-(2-chloroethyl) benzamide (1 eq) prepared in Step 1 was added thereto, and the reactants were stirred at 60°C for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 17.

The compound of Example 18 was obtained by the same method as Example 17 except for using R listed in Table 5 instead of the 4-(3,4-dichlorobenzyl)piperidine.

**[Table 5]**

| Example | R | Structure |
|---|---|---|
| 18 | 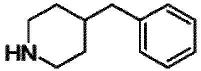 | 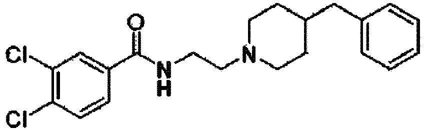 |

### <Example 19> Preparation of N-2-(4-(3,4-dichlorobenzyl)piperidin-1-yl)ethyl)-1H-indol-2-carboxamide

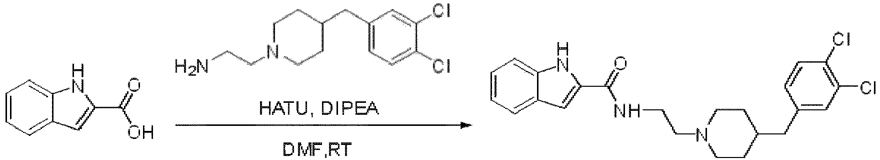

A carboxylic acid derivative (1 eq), an amine derivative (1.1 eq), HATU (2.5 eq), and DIPEA (1.5 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 19.

The compounds of Examples 20 to 51 were obtained by the same method as Example 19 except for using R^{a} listed in Table 6 instead of the 1H-indol-2-carboxylic acid and using R^{b} listed in Table 6 instead of 2-(4-(3,4-dichlorobenzyl)piperidin-1-yl)ethaneamine.

**[Table 6]**

| Example | **R^{a}** | **R^{b}** | Structure |
|---|---|---|---|
| 20 | 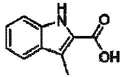 | 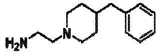 | 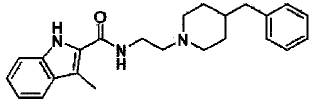 |
| 21 | 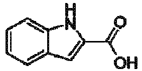 | 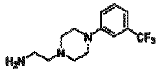 | 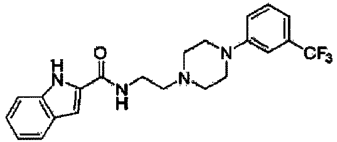 |
| 22 | 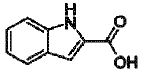 | 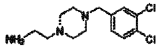 | 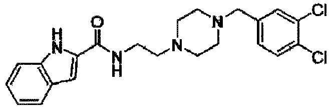 |
| 23 | 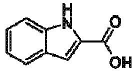 | 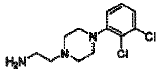 | 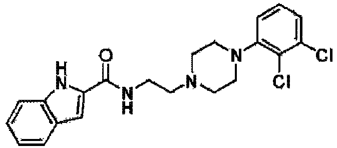 |
| 24 | 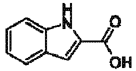 | 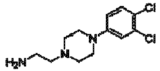 | 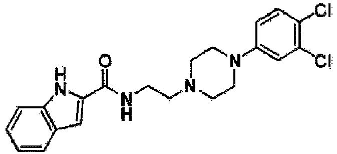 |
| 25 | 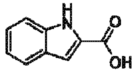 | 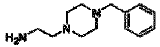 | 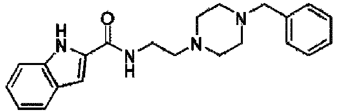 |
| **26** | 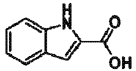 | 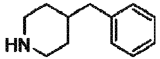 | 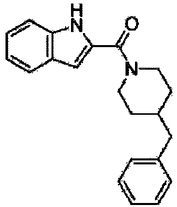 |
| 27 | 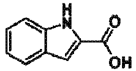 | 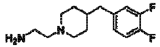 | 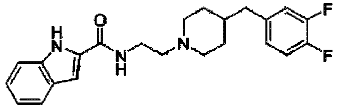 |
| 28 | 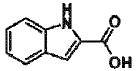 | 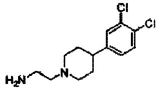 | 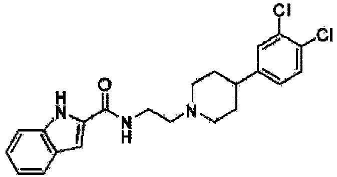 |
| 29 | 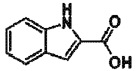 | 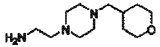 | 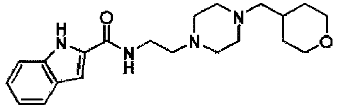 |
| 30 | 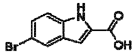 | 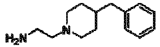 | 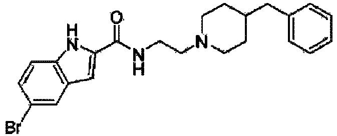 |
| 31 | 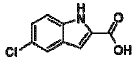 | 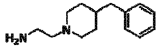 | 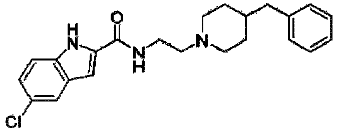 |
| 32 | 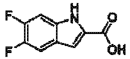 | 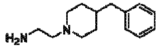 | 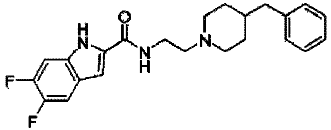 |
| 33 | 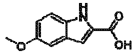 | 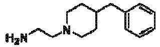 | 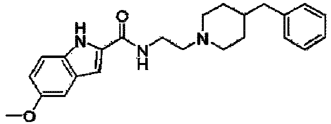 |
| 34 | 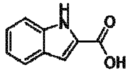 | 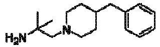 | 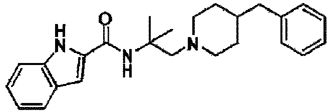 |
| 35 | 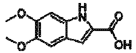 | 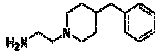 | 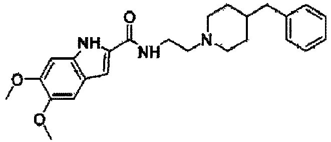 |
| 36 | 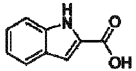 | 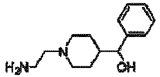 | 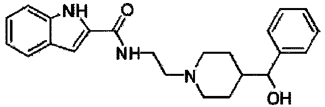 |
| 37 | 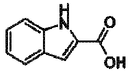 | 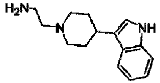 | 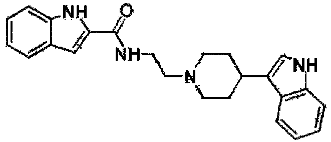 |
| 38 | 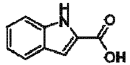 | 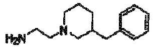 | 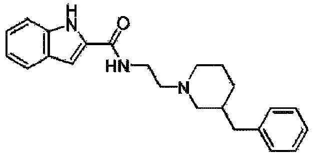 |
| 39 | 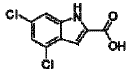 | 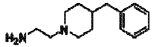 | 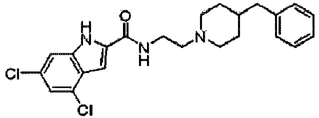 |
| 40 | 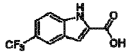 | 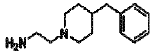 | 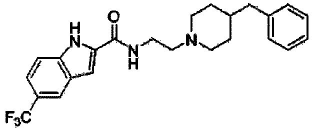 |
| 41 | 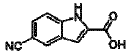 | 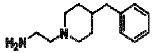 | 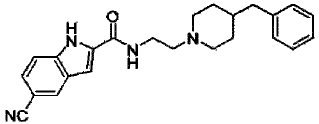 |
| 42 | 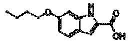 | 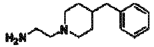 | 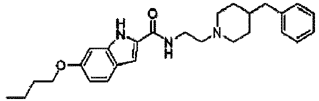 |
| 43 | 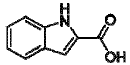 | 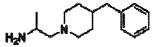 | 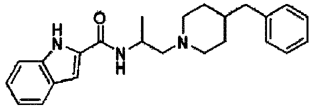 |
| | | HCl salt form | |
| 44 | 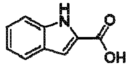 | 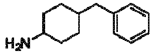 | 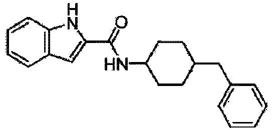 |
| 45 | 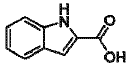 | 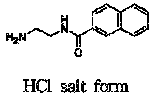 | 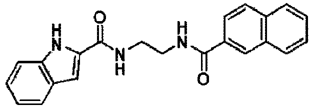 |
| 46 | 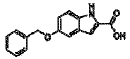 | 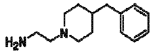 | 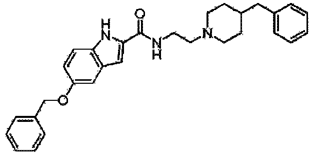 |
| 47 | 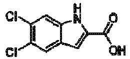 | 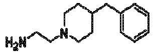 | 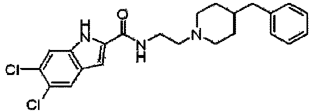 |
| 48 | 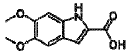 | 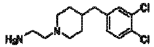 | 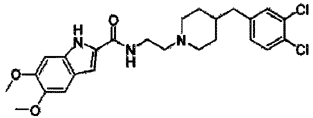 |
| 49 | 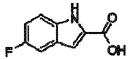 | 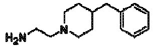 | 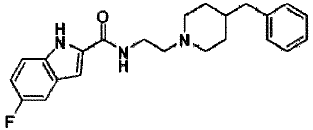 |
| | | | |
| 50 | 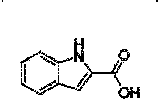 | 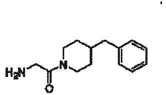 | 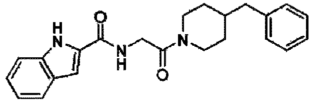 |
| 51 | 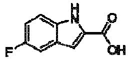 | 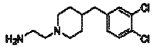 | 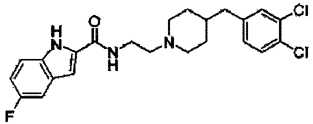 |

### <Example 52> Preparation of N-(2-(3-(5-fluoropyridin-2-yl)-1H-pyrazol-1-yl)ethyl)-1H-indol-2-carboxamide

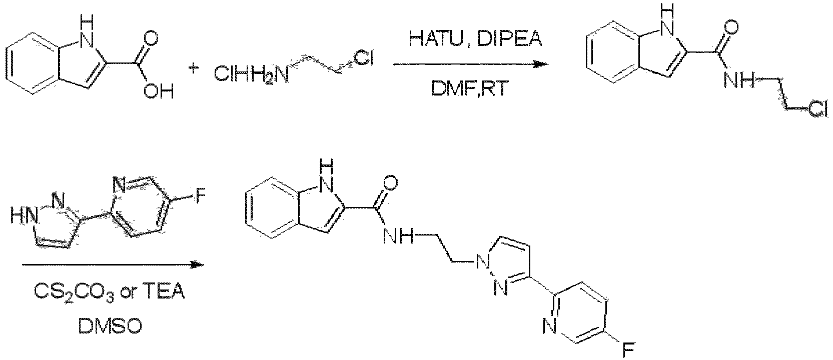

Step 1: 1H-indol-2-carboxylic acid (1 eq), 2-chloroethan-1-amine hydrochloride (1.2 eq), HATU (2.5 eq) and DIPEA (3 eq) were dissolved in DMF, and the reactants were stirred at room temperature for 12 hours. After finishing the reaction, the resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain a 1H-indol-2-carboxamide derivative.

Step 2: 5-Fluoro-2-(1H-pyrazol-3-yl)pyridine (1-1.5 eq) and Cs₂CO₃ (1.5 eq); or 5-Fluoro-2-(1H-pyrazol-3-yl)pyridine (1-1.5 eq) and TEA (4 eq) were dissolved in DMSO, the 1H-indol-2-carboxamide derivative (1 eq) prepared in Step 1 was added thereto, and the reactants were stirred at 60°C for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 52.

The compound of Example 53 was obtained by the same method as Example 52 except for using 1-(bromomethyl)cyclopropan-1-amine hydrobromide instead of 2-chloroethan-1-amine hydrochloride in Step 1, not using CS₂CO₃ but using TEA, and using R listed in Table 7 instead of the 5-(fluoro-2-(1H-pyrazol-3-yl)pyridine in Step 2.

**[Table 7]**

| **Example** | **R** | **Structure** |
|---|---|---|
| **53** | 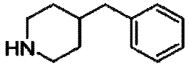 | 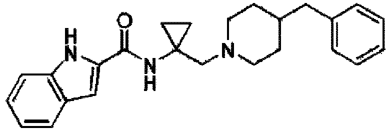 |

The compound of Example 54 was obtained by the same method as Example 52 except for using R listed in Table 8 instead of the 5-(fluoro-2-(1H-pyrazol-3-yl)pyridine in Step 2.

**[Table 8]**

| **Example** | **R** | **Structure** |
|---|---|---|
| **54** | 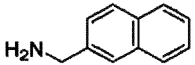 | 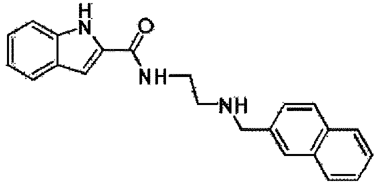 |

### <Example 55> Preparation of N-(2-(4-benzylpiperidin-1-yl)ethyl)benzo[d]thiazol-2-carboxamide

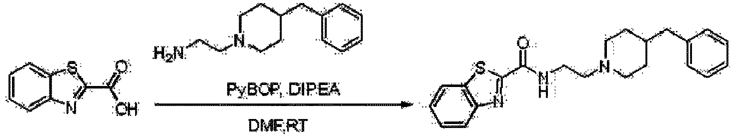

A carboxylic acid derivative (1 eq), an amine derivative (1.1 eq), PyBOP (1.1 eq) and DIPEA (3 eq) were dissolved in DMF, and the reactants were stirred at room temperature for 12 hours. After finishing the reaction, the resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 55.

The compounds of Examples 56 to 60 were obtained by the same method as Example 52 except for using R listed in Table 9 instead of the benzo[d]thiazol-2-carboxylic acid.

**[Table 9]**

| **Example** | **R** | **Structure** |
|---|---|---|
| **56** | 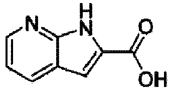 | 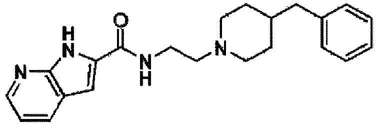 |
| **57** | 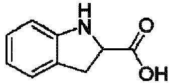 | 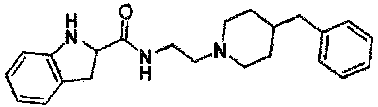 |
| **58** | 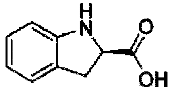 | 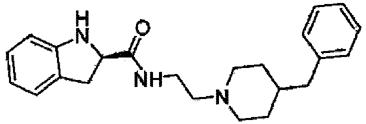 |
| **59** | 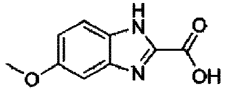 | 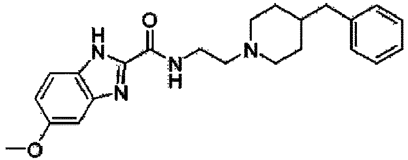 |
| **60** | 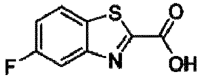 | 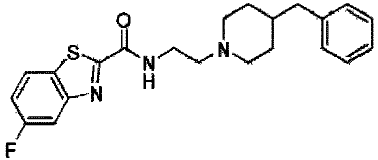 |

### <Example 61> Preparation of N-(2-(4-benzylpiperidin-1-yl)ethyl)-1H-benzo[d]imidazol-2-carboxamide

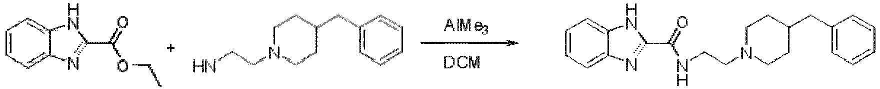

An amine derivative (1.2 eq) was dissolved in DCM, and 2 M trimethylaluminum in toluene (1.25 eq) was added thereto at 0°C. After stirring at room temperature for 5 minutes, ethyl 1H-benzo[d]imidazol-2-carboxylate (1 eq) dissolved in DCM was slowly added thereto, and the reactants were stirred at 40°C for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 61.

The compound of Example 62 was obtained by the same method as Example 61 except for using ethyl 1H-indol-2-carboxylate instead of ethyl 1H-benzo[d]imidazol-2-carboxylate and using R listed in Table 10 instead of the 2-(4-benzylpiperidin-1-yl)ethaneamine.

**[Table 10]**

| **Example** | **R** | **Structure** |
|---|---|---|
| **62** | 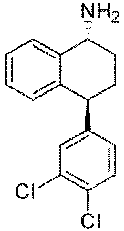 | 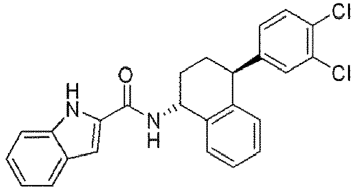 |

### <Example 63> Preparation of N-(2-(4-benzylpiperidin-1-yl)ethyl)-5,6-dihydroxy-1H-indol-carboxamide

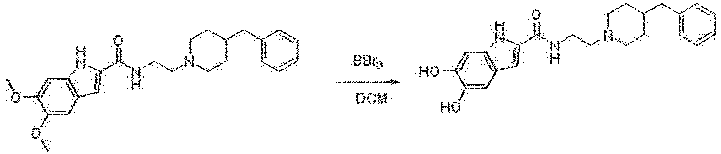

Example 35 (N-(2-(4-benzylpiperidin-1-yl)ethyl)-5,6-dimethoxy-1H-indol-2-carboxamide) was dissolved in DCM, and 1 M BBr₃ (2 eq) was slowly added thereto at 0°C under a nitrogen balloon stream. The reactant was stirred at room temperature for 12 hours. After finishing the reaction, the solvent was removed under a reduced pressure. The resultant was extracted with methanol, and the solvent in an organic layer was removed under a reduced pressure. Then, the reaction mixture was separated and purified by MPLC to obtain the compound of Example 63.

The compounds of Examples 64 and 65 were obtained by the same method as Example 63 except for using R listed in Table 11 instead of the Example 35 (N-(2-(4-benzylpiperidin-1-yl)ethyl)-5,6-dimethoxy-1H-indol-2-carboxamide).

**[Table 11]**

| **Example** | R | **Structure** |
|---|---|---|
| **64** | 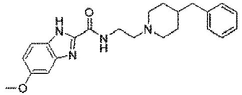 | 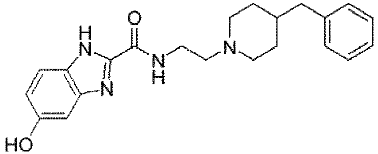 |
| | **(Example 59)** | |
| **65** | 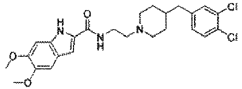 | 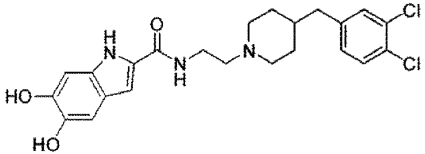 |
| | **(Example 48)** | |

### <Example 66> Preparation of N-(2-(4-benzylpiperidin-1-yl)ethyl)-5-hydroxy-1H-indol-2-carboxamide

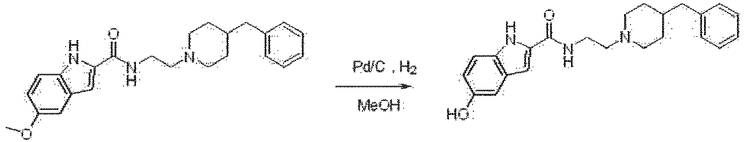

Example 33 (N-(2-(4-benzylpiperidin-1-yl)ethyl)-5-methoxy-1-H-indol-2-carboxamide) was dissolved in MeOH, and palladium on carbon (10% wt) was added thereto. The reactants were stirred under a hydrogen balloon stream at room temperature for 3 hours. After finishing the reaction, the reaction mixture was filtered using celite, and the solvent of an organic layer was removed under a reduced pressure. Then, the reaction mixture was separated and purified by MPLC to obtain the compound of Example 66.

### <Example 67> Preparation of N-(2-(4-benzylpiperidin-1-yl)ethyl)-5-butoxy-1H-indol-2-carboxamide

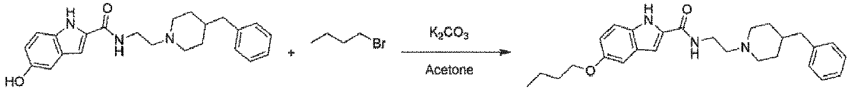

The N-(2-(4-benzylpiperidin-1-yl)ethyl-5-hydroxy-1H-indol-2-carboxamide (1 eq) obtained in Example 66, 1-bromobutane (1.2 eq) and k₂CO₃ (1.5 eq) were dissolved in acetone, and the reactants were refluxed and stirred for 3 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 67.

### <Example 68> Preparation of N-(2-(methyl(naphthalen-2-ylmethyl)amino)ethyl)-1H-indol-2-carboxamide

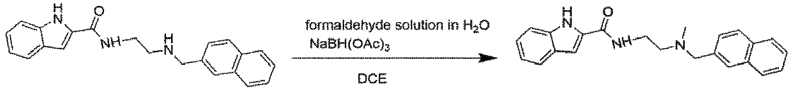

N-(2-((naphthalen-2-yl-methyl)amino)ethyl)-1H-indol-2-carboxamide (1 eq) obtained in Example 54, a formaldehyde solution (1.5 eq) and NaBH(Oac)₃ (5 eq) were dissolved in DCE, and the reactants were stirred at room temperature for 12 hours. After finishing the reaction, the resultant was extracted with 1 M NaOH and dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 68.

### <Example 69> Preparation of N-(2-(4-benzylpiperidin-1-yl)ethyl)cyclopropancarboxamide

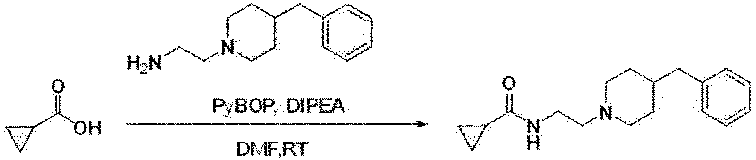

Cyclopropancarboxylic acid (1 eq), 2-(4-benzylpiperidin-1-yl)ethan-1-amine (1.1 eq), PyBOB (1.1 eq), and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 69.

The compounds of Examples 70 to 74 were obtained by the same method as Example 69 except for using R^{a} listed in Table 13 instead of the cyclopropanecarboxylic acid and using R^{b} listed in Table 13 instead of the 2-(4-benzylpiperidin-1-yl)ethan-1-amine.

**[Table 12]**

| **Example** | **R^{a}** | **R^{b}** | **Structure** |
|---|---|---|---|
| 70 | 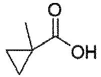 | 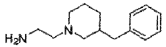 | 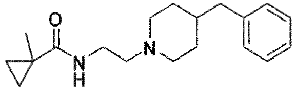 |
| 71 | 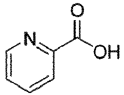 | 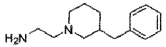 | 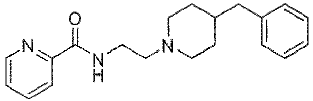 |
| 72 | 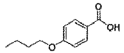 | 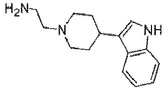 | 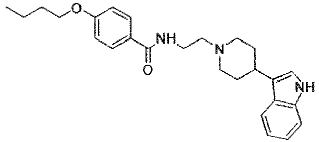 |
| 73 | 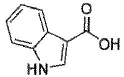 | 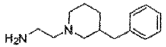 | 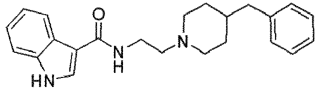 |
| 74 | 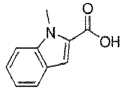 | 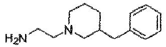 | 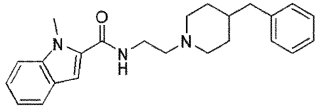 |

### <Example 75> Preparation of N-(2-(4-benzylpiperidin-1-yl)ethyl)-1H-pyrrol-2-carboxamide

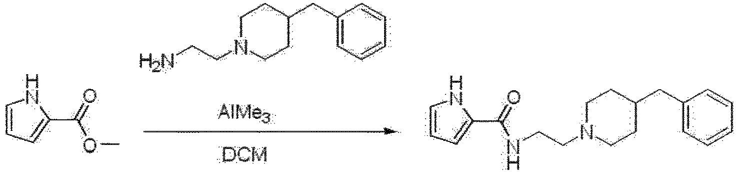

2-(4-Benzylpiperidin-1-yl)ethaneamine (1.2 eq) was dissolved in DCM, and 2 M trimethylaluminum in toluene (1.25 eq) was added thereto at 0°C. After stirring at room temperature for 5 minutes, methyl 1H-pyrrol-2-carboxylate (1 eq) dissolved in DCM was slowly added thereto, and the reactants were stirred at 40°C for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 75.

### <Example 76> Preparation of 3-(4-benzylpiperidin-1-yl)-N-(1H-indol-2-yl)propanamide

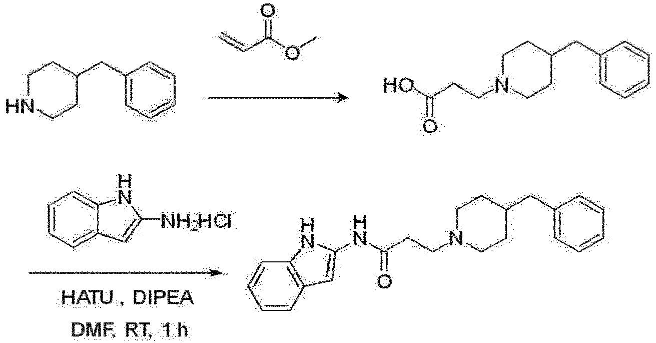

Step 1: 4-Benzylpiperidine (0.301 ml, 1.712 mmol) and methyl acrylate (0.186 ml, 2.054 mmol) were dissolved in DCM, and the reactants were stirred at room temperature for 12 hours. After finishing the reaction, the solvent was removed under a reduced pressure to obtain methyl 3-(4-benzylpiperidin-1-yl)propanoate, and the resultant was dissolved in a 5% NaOH aqueous solution (0.5 ml), followed by stirring at 40°C for 30 minutes. After finishing the reaction, the reaction mixture was cooled to room temperature and acidified using hydrochloric acid. The solid thus obtained was filtered and dried to obtain 3-(4-benzylpiperidin-1-yl)propanoic acid.

Step 2: 1H-indol-2-amine hydrochloride (1 eq), 3-(4-benzylpiperidin-1-yl)propanoic acid (1.1 eq) obtained in Step 1, HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by prep HPLC to obtain the compound of Example 76.

### <Example 77> Preparation of 4-(4-benzylpiperidin-1-yl)-N-(1H-indol-2-yl)-4-oxobutanamide

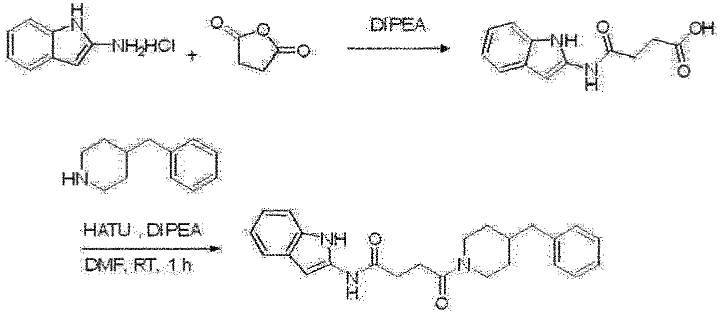

Step 1: 1H-indol-2-amine hydrochloride (1 eq), dihydro-furan-2,5-dione (1.2 eq) and DIPEA (3 eq) were dissolved in EtOH, and the reactants were stirred at 90°C for 3-4 hours. After finishing the reaction, the resultant was diluted with 1 M HCl and extracted with dichloromethane-methanol (20%), and the solvent was removed under a reduced pressure. Then, the reaction mixture was separated and purified by MPLC to obtain a carboxylic acid derivative.

Step 2: The carboxylic acid derivative (1 eq) obtained in Step 1, a piperidine derivative (1.1 eq), HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 77.

### <Example 78> Preparation of N-(2-(4-benzylpiperidin-1-yl)ethyl)benzo[d][1,3]dioxol-5-carboxamide

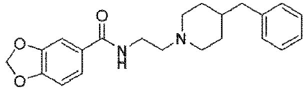

The compound of Example 78 was obtained by the same method as Example 19 except for using benzo[d] [1,3]dioxol-5-carboxylic acid 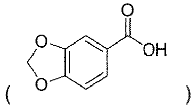 instead of the 1H-indol-2-carboxylic acid and using 2-(4-benzylpiperidin-1-yl)ethan-1-amine 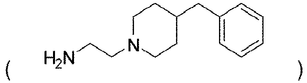 instead of the 2-(4-(3,4-dichlorobenzyl)piperidin-1-yl)ethaneamine.

### <Example 79> Preparation of N-(2-(4-benzylpiperidin-1-yl)ethyl)-2,3-dihydro-6H-[1,4]dioxyno[2,3-f]indol-7-carboxamide

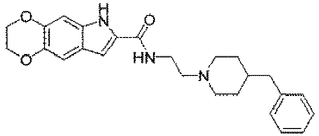

The compound of Example 79 was obtained by the same method as Example 19 except for using 2,3-dihydro-6H-[1,4]dioxyno[2,3-f]indol-7-carboxylic acid 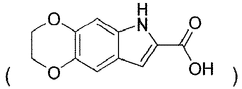 instead of the 1H-indol-2-carboxylic acid and using 2-(4-benzylpiperidin-1-yl)ethan-1-amine 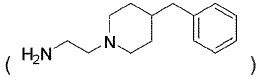 instead of the 2-(4-(3,4-dichlorobenzyl)piperidin-1-yl)ethaneamine.

### <Example 80> Preparation of N-(2-(4-benzylpiperidin-1-yl)ethyl)-2,3-dihydro-6H-[1,4]dioxyno[2,3-f]indol-7-carboxamide

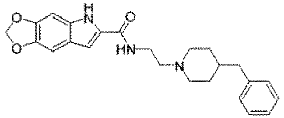

The compound of Example 80 was obtained by the same method as Example 19 except for using 5H-[1,3]dioxolo[4,5-f]indol-6-carboxylic acid 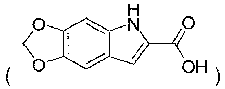 instead of the 1H-indol-2-carboxylic acid and using 2-(4-benzylpiperidin-1-yl)ethan-1-amine 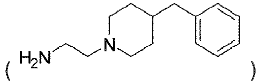 instead of the 2-(4-(3,4-dichlorobenzyl)piperidin-1-yl)ethaneamine.

### <Example 81> Preparation of 2-((2-(4-benzylpiperidin-1-yl)ethyl)carbamoyl)-1H-indol-5-carboxylic acid

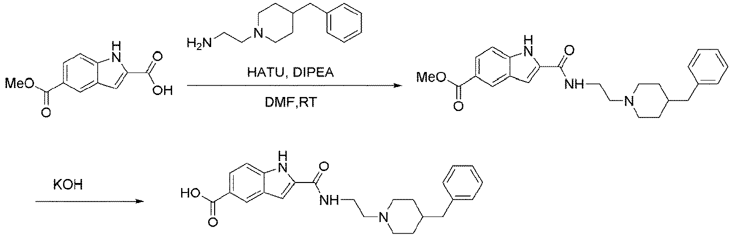

Step 1: 5-(Methoxycarbonyl)-1H-indol-2-carboxylic acid (1 eq), 2-(4-benzylpiperidin-1-yl)ethan-1-amine (1.1 eq), HA-TU (2.5 eq), and DIPEA (1.5 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over Na₂SO₄, and the solvent was removed under a reduced pressure. Then, the reaction mixture was separated and purified by MPLC to obtain methyl 2-((2-(4-benzylpiperidin-1-yl)ethyl)carbamoyl)-1H-indol-5-carboxylate.

Step 2: The methyl 2-((2-(4-benzylpiperidin-1-yl)ethyl)carbamoyl)-1H-indol-5-carboxylate (1 eq) obtained in Step 1 was dissolved in methanol, and a 4 M potassium hydroxide aqueous solution (150 eq, excess) was added thereto dropwisely, followed by reacting at 50°C for 12 hours. After finishing the reaction, the resultant was neutralized with a 1 M hydrochloric acid aqueous solution, extracted with dichloromethane and dried over Na₂SO₄, and the solvent was removed under a reduced pressure. Then, the reaction mixture was separated and purified by MPLC to obtain the title compound of 2-((2-(4-benzylpiperidin-1-yl)ethyl)carbamoyl)-1H-indol-5-carboxylic acid.

### <Example 82> Preparation of N2-(2-(4-benzylpiperidin-1-yl)-1H-indol-2,5-dicarboxamide

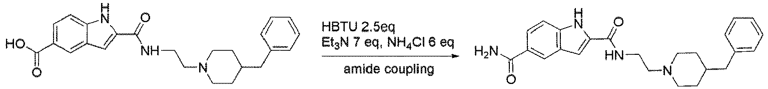

The 2-((2-(4-benzylpiperidin-1-yl)ethyl)carbamoyl)-1H-indol-5-carboxylic acid (1 eq) of Example 81 was dissolved in DMF, and HBTU (2.5 eq), ammonium chloride (7 eq), and triethylamine (6 eq) were added thereto dropwisely at 0°C. The reactants were stirred at room temperature for 12 hours.

After finishing the reaction, the resultant was extracted with ethyl acetate, an organic layer was washed with a saturated NaHCO₃ aqueous solution and brine in order, and dried over Na₂SO₄, and the solvent was removed under a reduced pressure. Then, the reaction mixture was separated and purified by MPLC and prep TLC to obtain the title compound of N2-(2-(4-benzylpiperidin-1-yl)ethyl)-1H-indol-2,5-dicarboxamide.

The compound names, NMR analysis results and Mass analysis results of Example 1 to Example 82 are summarized and shown in Tables 13 and 14.

**[Table 13]**

| Example | Compound name | ¹H NMR | Mass |
|---|---|---|---|
| 1 | 6-bromo-N-(2-(4-(3,4-dichloro-benzyl)pipera zin-1-yl)ethyl)-2-naphthamide | ¹H NMR (400 MHz, CDCl₃) δ 8.30 (s, 1H), 8.30 (s, 1H), 8.08 (s, 1H), 8.08 (s, 1H), 7.90 - 7.79 (m, 3H), 7.90 - 7.80 (m, 3H), 7.64 (d, J = 8.7 Hz, 1H), 7.64 (d, J = 8.7 Hz, 1H), 7.46 (d, J = 1.6 Hz, 1H), 7.46 (d, J = 1.6 Hz, 1H), 7.41 (d, J = 8.2 Hz, 1H), 7.41 (d, J = 8.2 Hz, 1H), 7.18 (dd, J = 8.2, 1.7 Hz, 1H), 7.18 (dd, J = 8.2, 1.7 Hz, 1H), 6.99 (s, 1H), 6.99 (s, 1H), 3.62 (dd, J = 11.1, 5.5 Hz, 2H), 3.49 (s, 2H), 2.68 (t, J = 6.0 Hz, 2H), 2.56 (d, J = 25.8 Hz, 8H). | 521.95 (M) |
| 2 | 6-bromo-N-(2-(4-(3-(trifluo-romethyl)pheny l)piperazi n-1-yl)ethyl)-2-naphthamide | ¹H NMR (400 MHz, CDCl₃) δ 8.25 (s, 1H), 8.01 (d, J = 1.4 Hz, 1H), 7.80 (ddd, J = 11.4, 8.7, 2.2 Hz, 3H), 7.58 (dd, J = 8.8, 1.9 Hz, 1H), 7.34 (t, J = 8.0 Hz, 1H), 7.10 (s, 1H), 7.07 (t, J = 7.3 Hz, 2H), 6.92 (s, 1H), 3.65 (dd, J = 11.2, 5.6 Hz, 2H), 3.33 - 3.18 (m, 4H), 2.76 - 2.63 (m, 6H). | 506.00 (M) |
| 3 | 6-bromo-N-(2-(4-(3,4-dichloro-phenyl)pipera zin-1-yl)ethyl)-2-naphthamide | ¹H NMR (400 MHz, CDCl₃) δ 8.25 (s, 1H), 8.02 (d, J = 1.6 Hz, 1H), 7.85 - 7.72 (m, 3H), 7.59 (dd, J = 8.8, 1.9 Hz, 1H), 6.95 (d, J = 2.9 Hz, 1H), 6.88 (s, 1H), 6.73 (dd, J = 8.9, 2.9 Hz, 1H), 3.63 (dd, J = 11.1, 5.6 Hz, 2H), 3.26 - 3.08 (m, 4H), 2.75 - 2.60 (m, 6H). | 507.95 (M) |
| 4 | 6-bromo-N-(2-(4-(2,3-dichloro-phenyl)pipera zin-1-yl)ethyl)-2-naphthamide | ¹H NMR (400 MHz, CDCl₃) δ 8.29 (s, 1H), 8.03 (d, J = 1.4 Hz, 1H), 7.85 (dd, J = 8.6, 1.5 Hz, 1H), 7.80 (d, J = 8.5 Hz, 2H), 7.60 (dd, J = 8.7, 1.9 Hz, 1H), 7.19 - 7.09 (m, 2H), 7.03 (s, 1H), 6.95 (dd, J = 7.5, 2.1 Hz, 1H), 3.66 (dd, J = 11.2, 5.4 Hz, 2H), 3.12 (s, 4H), 2.80 (t, J = 5.6 Hz, 6H). | 507.96 (M) |
| 5 | N-(2-(4-benzylpiperazin-1-yl)ethyl)-6-bromo-2-naphthamide | ¹H NMR (400 MHz, CDCl₃) δ 8.21 (s, 1H), 7.98 (s, 1H), 7.80 - 7.70 (m, 3H), 7.55 (dd, J = 8.7, 1.9 Hz, 1H), 7.25 (d, J = 4.4 Hz, 3H), 6.94 (s, 1H), 3.52 (dd, J = 11.2, 5.5 Hz, 2H), 3.47 (s, 2H), 2.58 (t, J = 6.0 Hz, 2H), 2.47 (d, J = 15.8 Hz, 8H). | 454.03 (M+H) |
| 6 | 6-bromo-N-(2-(4-(3,4-difluoro-benzyl)piperi din-1-yl)ethyl)-2-naphthamide | ¹H NMR (400 MHz, CDCl₃) δ 8.34 (s, 1H), 7.98 (d, J = 1.2 Hz, 1H), 7.87 (dd, J = 8.6, 1.6 Hz, 1H), 7.76 (t, J = 9.2 Hz, 3H), 7.56 (dd, J = 8.7, 1.8 Hz, 1H), 7.09 (dt, J = 10.1, 8.3 Hz, 1H), 7.00 - 6.91 (m, 1H), 6.89 - 6.80 (m, 1H), 3.74 (d, J = 4.2 Hz, 2H), 3.47 (d, J = 12.2 Hz, 2H), 3.24 - 3.13 (m, 2H), 2.73 (t, J = 11.7 Hz, 2H), 2.56 (d, J = 7.1 Hz, 2H), 1.87 (d, J = 13.9 Hz, 2H), 1.82 - 1.69 (m, 1H), 1.60 - 1.45 (m, 2H). | 488.04 (M+H) |
| 7 | 6-bromo-N-(2-(4-(3,4-difluoro-benzyl)pipera zin-1-yl)ethyl)-2-naphthamide | ¹H NMR (400 MHz, CDCl₃) δ 8.23 (s, 1H), 7.99 (s, 1H), 7.80 (dd, J = 8.5, 1.3 Hz, 1H), 7.74 (d, J = 8.8 Hz, 2H), 7.56 (dd, J = 8.7, 1.7 Hz, 1H), 7.18 - 7.11 (m, 1H), 7.06 (dt, J = 10.0, 8.3 Hz, 2H), 7.01 - 6.95 (m, 1H), 3.58 (dd, J = 11.0, 5.4 Hz, 2H), 3.44 (s, 2H), 2.71 (t, J = 5.8 Hz, 2H), 2.56 (d, J = 52.8 Hz, 8H). | 489.70 (M+H) |
| 8 | 6-bromo-N-(2-(3-(5-fluoro-pyridin-2-yl)-1H-pyrazol-1-yl)ethyl)-2-naphthamide | ¹H NMR (400 MHz, DMSO) δ 8.81 (t, J = 5.6 Hz, 1H), 8.55 (d, J = 2.9 Hz, 1H), 8.41 (s, 1H), 8.28 (d, J = 1.9 Hz, 1H), 8.01 - 7.90 (m, 4H), 7.83 (d, J = 2.3 Hz, 1H), 7.75 - 7.68 (m, 2H), 6.76 (d, J = 2.3 Hz, 1H), 4.41 (t, J = 6.2 Hz, 2H), 3.75 (q, J = 11.9, 5.9 Hz, 2H). | 439.14 (M) |
| 9 | 6-bromo-N-(2-(4-(3,4-dichloro-benzyl)piperi din-1-yl)ethyl)-2-naphthamide | ¹H NMR (400 MHz, MeOD) δ 8.27 (s, 1H), 8.05 (d, J = 1.6 Hz, 1H), 7.82 (d, J = 1.6 Hz, 1H), 7.80 (d, J = 8.1 Hz, 2H), 7.57 (dd, J = 8.8, 1.9 Hz, 1H), 7.31 (d, J = 8.2 Hz, 1H), 7.25 (d, J = 1.9 Hz, 1H), 7.01 (dd, J = 8.2, 2.0 Hz, 1H), 3.50 (t, J = 6.8 Hz, 2H), 2.97 (d, J = 11.6 Hz, 2H), 2.57 (t, J = 6.8 Hz, 2H), 2.46 (d, J = 6.8 Hz, 2H), 2.02 (t, J = 11.6 Hz, 2H), 1.57 (d, J = 13.6 Hz, 2H), 1.51 (dd, J = 7.3, 3.6 Hz, 1H), 1.30 - 1.23 (m, 2H). | 521.05 (M+H) |
| 10 | 3,5-dichloro-N-(2-(4-(3,4-dichloro-benzyl)piperi din-1-yl)ethyl)b enzamide | ¹H NMR (400 MHz, CDCl₃) δ 7.67 (d, J = 1.8 Hz, 2H), 7.49 (t, J = 1.8 Hz, 1H), 7.35 (d, J = 8.2 Hz, 1H), 7.24 (d, J = 2.0 Hz, 1H), 7.19 (s, 1H), 6.98 (dd, J = 8.2, 2.0 Hz, 1H), 3.59 (dd, J = 10.8, 5.2 Hz, 2H), 3.15 (d, J = 11.3 Hz, 2H), 2.82 (s, 2H), 2.53 (d, J = 7.2 Hz, 2H), 2.29 (t, J = 11.5 Hz, 2H), 1.75 (d, J = 13.6 Hz, 2H), 1.68 - 1.60 (m, 1H), 1.39 (td, J = 14.8, 3.2 Hz, 2H). | 460.98 (M) |
| 11 | 3,4-dichloro-N-(2-(4-(3,4-dichloro-benzyl)pipera zin-1-yl)ethyl)benzamide | ¹H NMR (400 MHz, CDCl₃) δ 7.89 (d, J = 2.0 Hz, 1H), 7.60 (dd, J = 8.3, 2.0 Hz, 1H), 7.51 (d, J = 8.3 Hz, 1H), 7.43 (d, J = 1.8 Hz, 1H), 7.38 (d, J = 8.2 Hz, 1H), 7.16 (dd, J = 8.2, 1.9 Hz, 1H), 6.96 (s, 1H), 3.53 (dd, J = 11.3, 5.4 Hz, 2H), 3.47 (d, J = 6.1 Hz, 4H), 2.61 (t, J = 5.9 Hz, 2H), 2.51 (d, J = 21.4 Hz, 6H). | 461.96 (M) |
| 12 | 3, 4-dichloro-N-(2-(4-(3-(trifluoromethyl)pheny l)piperazi n-1-yl)ethyl)b enzamide | ¹H NMR (400 MHz, CDCl₃) δ 7.89 (d, J = 2.0 Hz, 1H), 7.60 (dd, J = 8.3, 2.0 Hz, 1H), 7.52 (d, J = 8.3 Hz, 1H), 7.26 (s, 1H), 7.19 - 7.12 (m, 2H), 6.96 (dd, J = 7.2, 2.4 Hz, 1H), 6.74 (s, 1H), 3.58 (dd, J = 11.2, 5.6 Hz, 2H), 3.09 (s, 4H), 2.75 - 2.65 (m, 6H). | 447.92 (M+H) |
| 13 | 3, 4-dichloro-N-(2-(4-(2,3-dichloro-phenyl)pipera zin-1-yl)ethyl)b enzamide | ¹H NMR (400 MHz, CDCl₃) δ 7.88 (d, J = 1.9 Hz, 1H), 7.58 (dd, J = 8.3, 2.0 Hz, 1H), 7.51 (d, J = 8.3 Hz, 1H), 7.35 (t, J = 8.0 Hz, 1H), 7.12 (s, 1H), 7.08 (t, J = 8.6 Hz, 1H), 6.70 (s, 1H), 3.59 (dd, J = 11.2, 5.5 Hz, 2H), 3.30 - 3.22 (m, 4H), 2.72 - 2.63 (m, 6H). | 448.01 (M+H) |
| 14 | 3, 4-dichloro-N-(2-(4-(3,4-dichloro-phenyl)pipera zin-1-yl)ethyl)b enzamide | ¹H NMR (400 MHz, CDCl₃) δ 7.87 (d, J = 2.0 Hz, 1H), 7.57 (dd, J = 8.3, 2.0 Hz, 1H), 7.51 (d, J = 8.3 Hz, 1H), 7.28 (d, J = 9.0 Hz, 1H), 6.96 (d, J = 2.8 Hz, 1H), 6.74 (dd, J = 8.9, 2.8 Hz, 1H), 6.67 (s, 1H), 3.58 (dd, J = 11.3, 5.6 Hz, 2H), 3.22 - 3.17 (m, 4H), 2.66 (dd, J = 8.8, 5.5 Hz, 6H). | 447.94 (M) |
| 15 | N-(2-(4-benzylpiperazin-1-yl)ethyl)-3, 4-dichloro-benzamide | ¹H NMR (400 MHz, CDCl₃) δ 7.89 (d, J = 2.0 Hz, 1H), 7.59 (dd, J = 8.3, 2.0 Hz, 1H), 7.51 (d, J = 8.3 Hz, 1H), 7.36 - 7.29 (m, 3H), 7.29 - 7.23 (m, 2H), 6.96 (s, 1H), 3.58 - 3.51 (m, 4H), 2.70 (t, J = 5.8 Hz, 2H), 2.59 (d, J = 35.4 Hz, 8H). | 392.02 (M) |
| 16 | 3, 4-dichloro-N-(2-(4-(3,4-difluoro-benzyl)piperazin-1-yl)ethyl)b enzamide | ¹H NMR (400 MHz, CDCl₃) δ 7.87 (d, J = 1.9 Hz, 1H), 7.58 (dd, J = 8.3, 2.0 Hz, 1H), 7.52 (d, J = 8.3 Hz, 1H), 7.21 - 7.14 (m, 1H), 7.09 (dt, J = 10.0, 8.2 Hz, 1H), 7.04 - 6.98 (m, 1H), 6.79 (s, 1H), 3.52 (dd, J = 11.1, 5.6 Hz, 2H), 3.46 (s, 2H), 2.61 (t, J = 6.0 Hz, 2H), 2.51 (d, J = 21.6 Hz, 8H). | 428.00 (M) |
| 17 | 3,4-dichloro-N-(2-(4-(3,4-dichloro-benzyl)piperi din-1-yl)ethyl)b enzamide | ¹H NMR (400 MHz, MeOD) δ 7.90 (d, J = 2.0 Hz, 1H), 7.64 (dd, J = 8.4, 2.1 Hz, 1H), 7.53 (d, J = 8.4 Hz, 1H), 7.31 (d, J = 8.2 Hz, 1H), 7.24 (d, J = 1.9 Hz, 1H), 7.00 (dd, J = 8.2, 2.0 Hz, 1H), 3.44 (t, J = 6.8 Hz, 2H), 2.95 (d, J = 11.7 Hz, 2H), 2.53 (t, J = 6.8 Hz, 2H), 2.45 (d, J = 6.8 Hz, 2H), 2.00 (t, J = 11.2 Hz, 2H), 1.56 (d, J = 13.7 Hz, 2H), 1.52 - 1.46 (m, 1H), 1.27 - 1.19 (m, 2H). | 461.04 (M+H) |
| 18 | N- (2- (4-benzylpiperidin-1-yl)ethyl)-3,4-dichloro-benzamide | ¹H NMR (400 MHz, MeOD) δ 8.02 (d, J = 2.0 Hz, 1H), 7.76 (dd, J = 8.4, 2.1 Hz, 1H), 7.64 (d, J = 8.4 Hz, 1H), 7.27 (t, J = 7.5 Hz, 2H), 7.18 (dd, J = 10.4, 4.4 Hz, 3H), 3.55 (t, J = 6.8 Hz, 2H), 3.03 (d, J = 11.7 Hz, 2H), 2.61 (t, J = 6.8 Hz, 2H), 2.57 (d, J = 6.9 Hz, 2H), 2.06 (td, J = 11.8, 1.7 Hz, 2H), 1.68 (d, J = 14.0 Hz, 2H), 1.65 - 1.56 (m, J = 1H), 1.34 (qd, J = 12.7, 5.5 Hz, 2H). | 391.14 (M) |
| 19 | N-(2-(4-(3,4-dichloro-benzyl)piperi din-1-yl)ethyl)-1H-indol-2-carboxamide | ¹H NMR (400 MHz, MeOD) δ 7.62 (d, J = 8.1 Hz, 1H), 7.48 - 7.40 (m, 2H), 7.37 (d, J = 1.8 Hz, 1H), 7.23 (t, J = 7.6 Hz, 1H), 7.13 (dd, J = 8.2, 1.9 Hz, 1H), 7.09 (d, J = 7.3 Hz, 1H), 7.06 (s, 1H), 3.58 (t, J = 6.8 Hz, 2H), 3.07 (d, J = 10.8 Hz, 2H), 2.66 (t, J = 6.6 Hz, 2H), 2.58 (d, J = 6.8 Hz, 2H), 2.19 - 2.07 (m, 2H), 1.68 (d, J = 13.4 Hz, 2H), 1.66 - 1.55 (m, 1H), 1.44 - 1.33 (m, 2H). | 430.08 (M) |
| 20 | N- (2- (4-benzylpiperidin-1-yl)ethyl)-3-methyl-1H-indol-2-carboxamide | ¹H NMR (400 MHz, MeOD) δ 7.62 (d, J = 8.1 Hz, 1H), 7.39 (d, J = 8.3 Hz, 1H), 7.32 - 7.21 (m, 3H), 7.18 (dd, J = 7.2, 5.5 Hz, 3H), 7.09 (t, J = 7.5 Hz, 1H), 4.60 (s, 1H), 3.59 (t, J = 6.6 Hz, 2H), 3.05 (d, J = 11.4 Hz, 2H), 2.66 (t, J = 6.6 Hz, 2H), 2.56 (d, J = 4.2 Hz, 3H), 2.10 (t,J = 11.4 Hz, 2H), 1.70 (d, J = 13.8 Hz, 2H), 1.66 - 1.58 (m, 1H), 1.43 - 1.29 (m, 2H). | 376.15 (M+H) |
| 21 | N-(2-(4-(3-(trifluoromethyl)pheny l)piperazi n-1-yl)ethyl)-1H-indol-2-carboxamide | ¹H NMR (400 MHz, CDCl₃) δ 9.25 (s, 1H), 7.65 (d, J = 8.0 Hz, 1H), 7.44 (d, J = 8.3 Hz, 1H), 7.36 (t, J = 7.9 Hz, 1H), 7.32 - 7.26 (m, 1H), 7.14 (t, J = 7.2 Hz, 2H), 7.09 (t, J = 6.5 Hz, 2H), 6.86 (s, 1H), 6.84 (d, J = 1.3 Hz, 1H), 3.63 (dd, J = 11.2, 5.6 Hz, 2H), 3.30 (t, J = 4.9 Hz, 4H), 2.74 - 2.67 (m, 6H). | 417.11 (M+H) |
| 22 | N- (2- (4-(3,4-dichloro-benzyl)pipera zin-1-yl)ethyl)-1H-indol-2-carboxamide | ¹H NMR (400 MHz, CDCl₃) δ 9.21 (s, 1H), 7.67 (d, J = 8.0 Hz, 1H), 7.44 (d, J = 8.2 Hz, 2H), 7.38 (d, J = 8.2 Hz, 1H), 7.32 - 7.27 (m, 1H), 7.19 - 7.12 (m, 2H), 6.88 (s, 1H), 6.84 (d, J = 1.3 Hz, 1H), 3.56 (dd, J = 11.2, 5.5 Hz, 2H), 3.48 (s, 2H), 2.63 (t, J = 6.0 Hz, 2H), 2.53 (d, J = 14.7 Hz, 6H). | 431.07 (M) |
| 23 | N-(2-(4-(2, 3-dichloro-phenyl)pipera zin-1-yl)ethyl)-1H-indol-2-carboxamide | ¹H NMR (400 MHz, CDCl₃) δ 9.26 (s, 1H), 7.66 (d, J = 7.6 Hz, 1H), 7.47 - 7.42 (m, 1H), 7.32 - 7.27 (m, 1H), 7.20 - 7.11 (m, 3H), 6.99 (dd, J = 6.8, 2.8 Hz, 1H), 6.87 (s, 1H), 6.86 (d, J = 1.4 Hz, 1H), 3.63 (dd, J = 11.3, 5.5 Hz, 2H), 3.12 (s, 4H), 2.81 - 2.66 (m, 6H). | 417.06 (M) |
| 24 | N- (2- (4-(3,4-dichloro-phenyl)pipera zin-1-yl)ethyl)-1H-indol-2-carboxamide | ¹H NMR (400 MHz, CDCl₃) δ 9.17 (s, 1H), 7.65 (d, J = 8.0 Hz, 1H), 7.44 (d, J = 8.4 Hz, 1H), 7.32 - 7.26 (m, 3H), 7.14 (t, J = 7.5 Hz, 1H), 6.98 (d, J = 1 Hz, 4H), 6.83 (s, 1H), 6.76 (dd, J = 9.0, 2.9 Hz, 1H), 3.62 (dd, J = 10.7, 5.5 Hz, 2H), 3.25 - 3.18 (m, 4H), 2.69 (dd, J = 7.7, 4.9 Hz, 6H). | 417.03 (M) |
| 25 | N-(2-(4-benzylpiperazin-1-yl)ethyl)-1H-indol-2-carboxamide | ¹H NMR (400 MHz, CDCl₃) δ 9.30 (s, 1H), 7.67 (d, J = 8.0 Hz, 1H), 7.44 (d, J = 8.3 Hz, 1H), 7.33 (d, J = 4.5 Hz, 4H), 7.31 - 7.26 (m, 2H), 7.15 (t, J = 7.5 Hz, 1H), 6.92 (s, 1H), 6.84 (d, J = 1.2 Hz, 1H), 3.60 - 3.55 (m, 2H), 3.54 (s, 2H), 2.62 (t, J = 5.9 Hz, 2H), 2.55 (s, 8H). | 363.06 (M+H) |
| 26 | (4-benzylpiperidin-1-yl) (1H-indol-2-yl)methano ne | ¹H NMR (400 MHz, CDCl₃) δ 9.32 (s, 1H), 7.66 (d, J = 8.0 Hz, 1H), 7.44 (d, J = 8.3 Hz, 1H), 7.32 (dd, J = 14.5, 7.1 Hz, 3H), 7.29 - 7.21 (m, 2H), 7.21 - 7.16 (m, 2H), 7.14 (d, J = 7.7 Hz, 1H), 6.78 (d, J = 1.4 Hz, 1H), 4.73 (d, J = 13.3 Hz, 2H), 3.02 (s, 2H), 2.63 (d, J = 7.1 Hz, 2H), 1.91 (dtd, J = 14.9, 7.5, 3.7 Hz, 1H), 1.83 (d, J = 13.4 Hz, 2H), 1.36 (ddd, J = 25.0, 12.8, 4.1 Hz, 2H). | 319.02 3 (M+H) |
| 27 | N-(2-(4-(3,4-difluoro-benzyl)piperi din-1-yl)ethyl)-1H-indol-2-carboxamide | ¹H NMR (400 MHz, CDCl₃) δ 9.33 (s, 1H), 7.66 (d, J = 8.0 Hz, 1H), 7.43 (d, J = 8.2 Hz, 2H), 7.29 (d, J = 7.3 Hz, 1H), 7.14 (t, J = 7.5 Hz, 1H), 7.06 (dd, J = 18.5, 8.4 Hz, 1H), 6.99 - 6.90 (m, 2H), 6.84 (s, 1H), 3.62 (dd, J = 9.6, 4.5 Hz, 2H), 3.13 (d, J = 11.8 Hz, 2H), 2.78 (t, J = 5.3 Hz, 2H), 2.52 (d, J = 7.1 Hz, 2H), 2.23 (t, J = 12.2 Hz, 2H), 2.03 (d, J = 7.3 Hz, 2H), 1.71 (d, J = 12.8 Hz, 2H), 1.66 - 1.55 (m, 1H). | 398.08 (M+H) |
| 28 | N-(2-(4-(3,4-dichloro-phenyl)piperi din-1-yl)ethyl)-1H-indol-2-carboxamide | ¹H NMR (400 MHz, MeOD) δ 7.81 (s, 1H), 7.61 (d, J = 8.0 Hz, 1H), 7.44 (dd, J = 8.4, 0.5 Hz, 1H), 7.41 (d, J = 8.3 Hz, 1H), 7.39 (d, J = 1.9 Hz, 1H), 7.22 (t, J = 7.2 Hz, 1H), 7.17 (dd, J = 8.3, 2.0 Hz, 1H), 7.10 - 7.04 (m, 2H), 3.60 (t, J = 6.9 Hz, 2H), 3.17 (d, J = 11.7 Hz, 2H), 2.69 (t, J = 6.9 Hz, 2H), 2.25 (td, J = 11.8, 2.2 Hz, 2H), 1.88 (d, J = 12.5 Hz, 2H), 1.77 (ddd, J = 16.2, 12.9, 3.8 Hz, 2H). | 416.02 (M) |
| 29 | N-(2-(4-((tetrahydro-2H-pyran-4-yl)methyl) piperazin-1-yl)ethyl)-1H-indol-2-carboxamide | ¹H NMR (400 MHz, CDCl₃) δ 9.17 (s, 1H), 7.66 (d, J = 7.9 Hz, 1H), 7.44 (d, J = 8.2 Hz, 1H), 7.30 (d, J = 7.1 Hz, 1H), 7.15 (t, J = 7.2 Hz, 1H), 6.93 (s, 1H), 6.85 (d, J = 1.3 Hz, 1H), 3.97 (dd, J = 10.8, 3.5 Hz, 2H), 3.56 (dd, J = 11.2, 5.5 Hz, 2H), 3.44 - 3.33 (m, 2H), 2.62 (t, J = 5.9 Hz, 2H), 2.51 (d, J = 21.5 Hz, 6H), 2.22 (d, J = 7.1 Hz, 2H), 2.06 - 1.97 (m, 2H), 1.78 - 1.71 (m, 1H), 1.67 (dd, J = 15.5, 1.8 Hz, 2H), 1.34 - 1.20 (m, 2H) | 371.12 (M+H) |
| 30 | N- (2- (4-benzylpiperidin-1-yl)ethyl)-5-bromo-1H-indol-2-carboxamide | ¹H NMR (400 MHz, MeOD) δ 7.79 (d, J = 1.5 Hz, 1H), 7.40 (d, J = 8.8 Hz, 1H), 7.34 (dd, J = 8.8, 1.6 Hz, 1H), 7.30 (t, J = 7.6 Hz, 2H), 7.21 (t, J = 7.2 Hz, 3H), 7.04 (s, 1H), 3.73 (t, J = 6.1 Hz, 2H), 3.59 (d, J = 9.6 Hz, 2H), 3.26 - 3.16 (m, 2H), 2.85 (t, J = 10.0 Hz, 2H), 2.64 (d, J = 6.6 Hz, 2H), 1.91 (d, J = 11.8 Hz, 3H), 1.52 (dd, J = 25.2, 12.9 Hz, 2H). | 441.94 (M+H) |
| 31 | N- (2- (4-benzylpiperidin-1-yl)ethyl)-5-chloro-1H-indol-2-carboxamide | ¹H NMR (400 MHz, MeOD) δ 7.63 (d, J = 1.4 Hz, 1H), 7.45 (d, J = 8.8 Hz, 1H), 7.30 (t, J = 7.4 Hz, 2H), 7.21 (t, J = 7.5 Hz, 4H), 7.05 (s, 1H), 3.75 (t, J = 5.9 Hz, 2H), 3.63 (d, J = 9.5 Hz, 2H), 3.26 (s, 2H), 3.00 - 2.83 (m, 2H), 2.65 (d, J = 6.6 Hz, 2H), 1.92 (d, J = 12.5 Hz, 3H), 1.54 (dd, J = 25.2, 12.6 Hz, 2H). | 396.01 (M+H) |
| 32 | N- (2- (4-benzylpiperidin-1-yl)ethyl)-5, 6-difluoro-1H-indol-2-carboxamide | ¹H NMR (400 MHz, MeOD) δ 7.63 (d, J = 1.4 Hz, 1H), 7.45 (d, J = 8.8 Hz, 1H), 7.30 (t, J = 7.4 Hz, 2H), 7.21 (t, J = 7.5 Hz, 4H), 7.05 (s, 1H), 3.75 (t, J = 5.9 Hz, 2H), 3.63 (d, J = 9.5 Hz, 2H), 3.26 (s, 2H), 3.00 - 2.83 (m, 2H), 2.65 (d, J = 6.6 Hz, 2H), 1.92 (d, J = 12.5 Hz, 3H), 1.54 (dd, J = 25.2, 12.6 Hz, 2H). | 398.02 (M+H) |
| 33 | N- (2- (4-benzylpiperidin-1-yl)ethyl)-5-methoxy-1H-indol-2-carboxamide | ¹H NMR (400 MHz, MeOD) δ 7.36 (d, J = 8.9 Hz, 1H), 7.31 (t, J = 7.5 Hz, 2H), 7.22 (t, J = 7.1 Hz, 3H), 7.09 (d, J = 2.1 Hz, 1H), 7.02 (s, 1H), 6.93 (dd, J = 9.0, 2.2 Hz, 1H), 3.83 (s, 3H), 3.76 (t, J = 5.8 Hz, 2H), 3.72 (s, 2H), 3.00 (s, 2H), 2.66 (d, J = 6.6 Hz, 2H), 1.95 (d, J = 12.6 Hz, 3H), 1.56 (dd, J = 24.2, 12.2 Hz, 2H), 1.40 - 1.29 (m, 2H). | 392.10 (M+H) |
| 34 | N- (1- (4-benzylpiperidin-1-yl)-2-methylpropan-2-yl)-1H-indol-2-carboxamide | ¹H NMR (400 MHz, CDCl₃) δ 9.19 (s, 1H), 9.19 (s, 1H), 7.61 (d, J = 8.0 Hz, 1H), 7.37 (d, J = 8.3 Hz, 1H), 7.22 (t, J = 7.4 Hz, 3H), 7.17 - 7.12 (m, 2H), 7.08 (t, J = 7.4 Hz, 3H), 6.64 (s, 1H), 2.85 (d, J = 11.6 Hz, 2H), 2.50 (d, J = 7.0 Hz, 2H), 2.40 (s, 2H), 2.21 (t, J = 11.0 Hz, 2H), 1.59 (d, J = 13.0 Hz, 2H), 1.50 - 1.42 (m, 1H), 1.40 (s, 6H), 1.29 (ddd, J = 15.4, 12.4, 3.6 Hz, 2H). | 390.07 (M+H) |
| 35 | N- (2- (4-benzylpiperidin-1-yl)ethyl)-5, 6-dimethoxy-1H-indol-2-carboxamide | ¹H NMR (400 MHz, CDCl₃) δ 8.99 (s, 1H), 7.45 (t, J = 5.3 Hz, 1H), 7.30 (t, J = 7.4 Hz, 2H), 7.26 - 7.19 (m, 1H), 7.12 (d, J = 7.4 Hz, 2H), 7.06 (s, 1H), 7.00 (s, 1H), 6.83 (s, 1H), 3.88 (s, 6H), 3.66 (s, 2H), 3.51 (s, 2H), 3.19 (s, 2H), 2.77 (s, 2H), 2.59 (d, J = 7.0 Hz, 2H), 1.92 (d, J = 14.3 Hz, 2H), 1.81 (s, 1H). | 422.10 (M+H) |
| 36 | N- (2- (4-(hydroxy(phen yl)methyl) piperidin-1-yl)ethyl)-1H-indol-2-carboxamide | ¹H NMR (400 MHz, CDCl₃) δ 9.14 (s, 1H), 7.68 (d, J = 8.1 Hz, 1H), 7.43 (d, J = 8.3 Hz, 1H), 7.40 - 7.28 (m, 6H), 7.15 (t, J = 7.5 Hz, 1H), 6.90 (s, 1H), 6.83 (s, 1H), 4.43 (d, J = 7.5 Hz, 1H), 3.54 (dd, J = 11.1, 5.5 Hz, 2H), 3.01 (d, J = 12.4 Hz, 1H), 2.89 (d, J = 11.0 Hz, 1H), 2.57 (t, J = 5.9 Hz, 2H), 2.05 (s, 1H), 2.01 (d, J = 12.7 Hz, 1H), 1.94 (dd, J = 15.1, 10.5 Hz, 2H), 1.46 (dd, J = 21.7, 10.1 Hz, 1H), 1.33 (dd, J = 8.6, 5.2 Hz, 2H). | 377.99 (M) |
| 37 | N- (2- (4-(1H-indol-3-yl)piperidin-1-yl)ethyl)-1H-indol-2-carboxamide | ¹H NMR (400 MHz, CDCl₃) δ 9.16 (s, 1H), 7.98 (s, 1H), 7.67 (d, J = 7.9 Hz, 2H), 7.44 (d, J = 8.2 Hz, 1H), 7.39 (d, J = 8.1 Hz, 1H), 7.30 (d, J = 7.5 Hz, 2H), 7.21 (t, J = 7.6 Hz, 1H), 7.14 (dd, J = 17.3, 7.6 Hz, 2H), 7.02 (s, 1H), 6.98 (s, 1H), 6.87 (s, 1H), 3.61 (dd, J = 11.0, 5.5 Hz, 2H), 3.08 (d, J = 11.3 Hz, 2H), 2.68 (t, J = 6.0 Hz, 2H), 2.26 (dd, J = 19.8, 9.2 Hz, 2H), 2.10 (t, J = 20.4 Hz, 2H), 2.01 (d, J = 6.3 Hz, 1H), 1.85 (dd, J = 22.0, 12.3 Hz, 2H). | 386.95 (M) |
| 38 | N-(2-(3-benzylpiperidin-1-yl)ethyl)-1H-indol-2-carboxamide | ¹H NMR (400 MHz, MeOD) δ 7.62 (d, J = 8.1 Hz, 1H), 7.45 (d, J = 8.3 Hz, 1H), 7.24 (q, J = 8.1 Hz, 3H), 7.17 (d, J = 6.7 Hz, 3H), 7.08 (t, J = 7.5 Hz, 1H), 7.04 (s, 1H), 3.54 (t, J = 6.9 Hz, 2H), 3.01 (d, J = 11.5 Hz, 1H), 2.92 (d, J = 9.3 Hz, 1H), 2.62 (t, J = 6.8 Hz, 2H), 2.54 (d, J = 6.7 Hz, 2H), 2.12 - 2.01 (m, 1H), 1.91 (s, 1H), 1.88 - 1.78 (m, 1H), 1.75 (d, J = 11.2 Hz, 2H), 1.60 (d, J = 12.8 Hz, 1H), 1.03 (dd, J = 22.4, 9.8 Hz, 1H). | 361.98 (M) |
| 39 | N- (2- (4-benzylpiperidin-1-yl)ethyl)-4,6-dichloro-1H-indol-2-carboxamide | ¹H NMR (400 MHz, CDCl₃) δ 9.51 (s, 1H), 7.35 (s, 1H), 7.30 (d, J = 7.6 Hz, 2H), 7.21 (d, J = 7.0 Hz, 1H), 7.16 (d, J = 9.0 Hz, 3H), 7.04 (s, 1H), 6.91 (s, 1H), 3.55 (dd, J = 11.2, 5.5 Hz, 2H), 2.93 (d, J = 11.5 Hz, 2H), 2.57 (d, J = 7.1 Hz, 4H), 1.99 (t, J = 10.9 Hz, 2H), 1.69 (d, J = 13.5 Hz, 2H), 1.59 - 1.53 (m, 1H), 1.33 (dd, J = 21.0, 12.0 Hz, 2H). | 430 (M) |

**[Table 14]**

| | | | |
|---|---|---|---|
| | | | |
| 40 | N-(2-(4-benzylpiperidin-1-yl)ethyl) -5-(trifluoromethyl) -1H-indol-2-carboxamide | ¹H NMR (400 MHz, CDCl₃) δ 9.46 (s, 1H), 7.99 (s, 1H), 7.52 (s, 2H), 7.29 (t, J = 7.5 Hz, 2H), 7.21 (d, J = 7.0 Hz, 1H), 7.17 (t, J = 6.9 Hz, 2H), 7.09 (s, 1H), 6.93 (s, 1H), 3.55 (dd, J = 10.9, 5.4 Hz, 2H), 2.92 (d, J = 11.4 Hz, 2H), 2.58 (t, J = 5.8 Hz, 4H), 1.99 (t, J = 11.3 Hz, 2H), 1.70 (d, J = 13.5 Hz, 2H), 1.59 - 1.51 (m, 1H), 1.32 (ddd, J = 15.7, 12.8, 3.7 Hz, 2H). | 430.07 (M+H) |
| 41 | N-(2-(4-benzylpiperidin-1-yl)ethyl) -5-cyano-1H-indol-2-carboxamide | ¹H NMR (400 MHz, CDCl₃) δ 9.54 (s, 1H), 8.06 (s, 1H), 7.51 (s, 2H), 7.34 - 7.27 (m, 2H), 7.22 (d, J = 7.0 Hz, 1H), 7.17 (t, J = 7.9 Hz, 2H), 7.11 (s, 1H), 6.92 (s, 1H), 3.55 (dd, J = 11.2, 5.5 Hz, 2H), 2.92 (d, J = 11.6 Hz, 2H), 2.62 - 2.53 (m, 4H), 1.99 (t, J = 10.7 Hz, 2H), 1.70 (dd, J = 12.5, 1.5 Hz, 2H), 1.58 - 1.53 (m, 1H), 1.32 (ddd, J = 15.1, 12.4, 3.5 Hz, 2H). | 387.08 (M+H) |
| 42 | N-(2-(4-benzylpiperidin-1-yl)ethyl) -6-butoxy-1H-indol-2-carboxamide | ¹H NMR (400 MHz, CDCl₃) δ 9.88 (s, 1H), 8.87 (s, 1H), 7.47 (d, J = 8.7 Hz, 1H), 7.32 - 7.24 (m, 2H), 7.21 (t, J = 7.2 Hz, 1H), 7.14 - 7.05 (m, 3H), 6.80 - 6.73 (m, 2H), 3.90 (t, J = 6.3 Hz, 2H), 3.77 (s, 2H), 3.49 (s, 2H), 3.08 (s, 2H), 2.53 (s, 2H), 2.42 (s, 2H), 1.81 - 1.69 (m, 4H), 1.63 (s, 2H), 1.48 (dq, J = 14.7, 7.4 Hz, 2H), 0.97 (t, J = 7.4 Hz, 3H). | 434.06 (M+H) |
| 43 | N- (1- (4-benzylpiperidin-1-yl)propan -2-yl)-1H-indol-2-carboxamide | ¹H NMR (400 MHz, CDCl₃) δ 9.17 (s, 1H), 7.68 (d, J = 8.0 Hz, 1H), 7.44 (d, J = 8.2 Hz, 1H), 7.35 - 7.23 (m, 6H), 7.22 - 7.15 (m, 2H), 7.13 (d, J = 7.5 Hz, 2H), 6.86 (d, J = 4.2 Hz, 1H), 6.83 (s, 1H), 4.09 (dt, J = 14.9, 5.8 Hz, 1H), 3.49 (s, 3H), 2.86 (t, J = 10.1 Hz, 2H), 2.52 (d, J = 6.9 Hz, 2H), 2.49 - 2.33 (m, 2H), 2.11 (t, J = 10.7 Hz, 1H), 2.01 (dd, J = 12.2, 6.6 Hz, 1H), 1.85 (t, J = 11.0 Hz, 1H), 1.85 (t, J = 11.0 Hz, 1H), 1.63 (q, J = 15.3 Hz, 5H), 1.55 - 1.45 (m, 1H), 1.31 (d, J = 6.2 Hz, 5H). | 376.05 (M+H) |
| 44 | N- (4-benzylcyclohexyl) -1H-indol-2-carboxamide | ¹H NMR (400 MHz, DMSO) δ 11.52 (s, 1H), 8.24 (d, J = 7.6 Hz, 1H), 7.58 (d, J = 8.0 Hz, 1H), 7.41 (d, J = 8.2 Hz, 1H), 7.28 (t, J = 7.5 Hz, 2H), 7.18 (dd, J = 12.2, 4.6 Hz, 4H), 7.13 (d, J = 4.2 Hz, 1H), 7.01(t, J = 7.4 Hz, 1H), 3.74 (m, 1H), 1.85 (d, J = 11.1 Hz, 2H), 1.69 (d, J = 12.4 Hz, 2H), 1.47 (m, 1H), 1.30 (dd, J = 25.0, 13.6 Hz, 3H), 1.23 (s, 1H), 1.08 (q, J = 11.8 Hz, 2H). | 332.90 (M) |
| 45 | N- (2- (2-naphthamido)e thyl)-1H-indol-2-carboxamide | ¹H NMR (400 MHz, CDCl₃) δ 9.10 (brs, 1H), 8.35 (s, 1H), 7.96-7.80 (m, 4H), 7.68 (d, J = 8.0 Hz, 1H), 7.60-7.51 (m, 2H), 7.42 (d, J = 8.4 Hz, 1H), 7.36 (brs, 1H), 7.30 (d, J = 7.7 Hz, 1H), 7.21 (brs, 1H), 7.15 (t, J = 7.6 Hz, 1H), 6.99 (s, 1H), 3.86-3.73 (m, 4H) | 358.03 (M+H) |
| 46 | 5-(benzyloxy)-N-(2-(4-benzylpiperidin-1-yl)ethyl) -1H-indol-2-carboxamide | ¹H NMR (400 MHz, CDCl₃) δ 9.38 (s, 1H), 7.48 (d, J = 7.6 Hz, 2H), 7.40 (t, J = 7.4 Hz, 2H), 7.37 - 7.26 (m, 4H), 7.20 (t, J = 7.5 Hz, 1H), 7.16 (d, J = 7.6 Hz, 3H), 7.04 (d, J = 8.9 Hz, 1H), 6.91 (s, 1H), 6.74 (s, 1H), 5.11 (s, 2H), 3.54 (dd, J = 10.8, 5.3 Hz, 2H), 2.91 (d, J = 11.2 Hz, 2H), 2.57 (d, J = 6.8 Hz, 4H), 1.98 (t, J = 11.4 Hz, 2H), 1.68 (d, J = 12.6 Hz, 2H), 1.63 - 1.50 (m, 1H), 1.38 - 1.26 (m, 2H). | 468.15 (M+H) |
| 47 | N-(2-(4-benzylpiperidin-1-yl)ethyl) -5, 6-dichloro-1H-indol-2-carboxamide | ¹H NMR (400 MHz, CDCl₃) δ 9.40 (s, 1H), 7.76 (s, 1H), 7.56 (s, 1H), 7.29 (t, J = 7.5 Hz, 2H), 7.21 (t, J = 7.3 Hz, 1H), 7.16 (d, J = 7.6 Hz, 2H), 6.98 (s, 1H), 6.75 (s, 1H), 3.54 (dd, J = 11.0, 5.4 Hz, 2H), 2.91 (d, J = 11.2 Hz, 2H), 2.58 - 2.56 (m, 4H), 1.99 (t, J = 11.3 Hz, 2H), 1.69 (d, J = 12.7 Hz, 2H), 1.60 - 1.51 (m, 1H), 1.36 - 1.26 (m, 2H). | 430.03 (M) |
| 48 | N-(2-(4-(3,4-dichloro-benzyl)piper idin-1-yl)ethyl) -5,6-di-methoxy-1H-indol-2-carboxamide | ¹H NMR (400 MHz, CDCl₃) δ 9.20 (s, 1H), 7.34 (d, J = 8.2 Hz, 1H), 7.24 (d, J = 1.9 Hz, 1H), 7.05 (s, 1H), 6.98 (dd, J = 8.2, 2.0 Hz, 1H), 6.89 (s, 1H), 6.78 (s, 1H), 6.73 (d, J = 1.7 Hz, 1H), 3.93 (d, J = 3.7 Hz, 6H), 3.53 (dd, J = 11.2, 5.6 Hz, 2H), 2.92 (d, J = 11.6 Hz, 2H), 2.56 (t, J = 6.0 Hz, 2H), 2.52 (d, J = 7.1 Hz, 2H), 1.98 (td, J =11.5, 1.6 Hz, 2H), 1.65 (d, J = 12.2 Hz, 2H), 1.59 - 1.48 (m, 1H), 1.34 - 1.25 (m, 2H). | 492.13 (M+H) |
| 49 | N-(2-(4-benzylpiperidin-1-yl)ethyl) -5-fluoro-1H-indol-2-carboxamide | ¹H NMR (400 MHz, CDCl₃) δ 9.18 (s, 1H), 7.34 (ddd, J = 9.3, 8.1, 3.7 Hz, 2H), 7.28 (d, J = 7.7 Hz, 2H), 7.20 (t, J = 7.4 Hz, 1H), 7.16 (d, J = 7.2 Hz, 2H), 7.05 (td, J = 9.1, 2.3 Hz, 1H), 6.92 (s, 1H), 6.78 (d, J = 1.1 Hz, 1H), 3.53 (dd, J = 11.1, 5.6 Hz, 2H), 2.91 (d, J = 11.4 Hz, 2H), 2.56 (t, J = 6.7 Hz, 2H), 1.98 (t, J = 11.0 Hz, 2H), 1.69 (d, J = 12.7 Hz, 2H), 1.63 - 1.59 (m, 2H), 1.57 - 1.51 (m, 1H), 1.37 - 1.27 (m, 2H). | 380.13 (M+H) |
| 50 | N-(2-(4-benzylpiperidin-1-yl)-2-oxoethyl)-1H-indol-2-carboxamide | ¹H NMR (400 MHz, MeOD) δ 7.63 (d, J = 8.0 Hz, 1H), 7.46 (d, J = 8.3 Hz, 1H), 7.32 - 7.26 (m, 2H), 7.23 (d, J = 7.7 Hz, 1H), 7.19 (d, J = 7.8 Hz, 3H), 7.13 (s, 1H), 7.08 (t, J = 7.5 Hz, 1H), 4.52 (d, J = 13.8 Hz, 1H), 4.28 (q, J = 16.8 Hz, 2H), 3.96 (d, J = 14.1 Hz, 1H), 3.11 (dd, J = 23.4, 11.9 Hz, 1H), 2.67 (t, J = 11.6 Hz, 1H), 2.60 (d, J = 7.1 Hz, 2H), 1.87 (dd, J = 9.3, 5.2 Hz, 1H), 1.74 (t, J = 15.1 Hz, 2H), 1.32 (dd, J = 18.6, 6.4 Hz, 2H). | 376.13 (M+H) |
| 51 | N-(2-(4-(3,4-dichloro-benzyl)piper idin-1-yl)ethyl) -5-fluoro-1H-indol-2-carboxamide | ¹H NMR (400 MHz, CDCl₃) δ 9.30 (s, 1H), 7.38 - 7.33 (m, 2H), 7.30 (dd, J = 9.3, 2.5 Hz, 1H), 7.25 (d, J = 2.0 Hz, 1H), 7.05 (td, J = 9.1, 2.5 Hz, 1H), 6.98 (dd, J = 8.2, 2.0 Hz, 1H), 6.91 (s, 1H), 6.79 (d, J = 1.4 Hz, 1H), 3.54 (dd, J = 11.3, 5.5 Hz, 2H), 2.92 (d, J = 11.7 Hz, 2H), 2.58 (t, J = 6.0 Hz, 2H), 2.52 (d, J = 7.1 Hz, 2H), 2.00 (td, J = 11.7, 2.0 Hz, 2H), 1.68 (s, 2H), 1.60 - 1.49 (m, 1H), 1.35 - 1.28 (m, 2H). | 448.10 (M+H) |
| 52 | N-(2-(3-(5-fluoro-pyridin-2-yl)-1H-pyrazol-1-yl)ethyl) -1H-indol-2-carboxamide | ¹H NMR (400 MHz, DMSO) δ 11.57 (s, 1H), 8.63 (t, J = 5.7 Hz, 1H), 8.58 (d, J = 2.8 Hz, 1H), 7.95 (dd, J = 8.8, 4.6 Hz, 1H), 7.84 (d, J = 2.3Hz, 1H), 7.74 - 7.71 (m, 1H), 7.60 (d, J = 7.9 Hz, 1H), 7.41 (d, J = 8.2 Hz, 1H), 7.21 - 7.14 (m, 1H), 7.07 (d, J = 1.5 Hz, 1H), 7.05 - 6.99 (m, 1H), 6.75 (d, J = 2.3 Hz, 1H), 4.39 (t, J = 6.1 Hz, 2H), 3.73 (q, J = 11.9, 6.0 Hz, 2H). | 350.11 (M+H) |
| 53 | N-(1-((4-benzylpiperidin-1-yl)methyl )cyclopropyl) - 1H-indol-2-carboxamide | ¹H NMR (400 MHz, DMSO) δ 11.46 (s, 1H), 8.47 (s, 1H), 7.59 (d, J = 8.0 Hz, 1H), 7.42 (d, J = 8.2 Hz, 1H), 7.25 (t, J = 7.3 Hz, 2H), 7.17 (t, J = 6.4 Hz, 2H), 7.11 (dd, J = 11.5, 4.1 Hz, 3H), 7.03 (t, J = 7.5 Hz, 1H), 3.36 (d, J = 0.9 Hz, 2H), 2.98 (d, J = 11.1 Hz, 2H), 2.46 (d, J = 6.7 Hz, 2H), 1.88 (t, J = 11.0 Hz, 2H), 1.48 (d, J = 12.8 Hz, 2H), 1.41 (dd, J = 9.2, 5.2 Hz, 1H), 1.16 (dd, J = 22.7, 9.2 Hz, 2H), 0.78 (t, J = 5.6 Hz, 2H), 0.66 (t, J = 5.8 Hz, 2H). | 388.07 (M+H) |
| 54 | N- (2-((naphthalen-2-ylmethyl)amin o)ethyl)-1H-indol-2-carboxamide | ¹H NMR (400 MHz, CDCl₃) δ 9.07 (brs, NH), 8.24-8.20 (m, 1H), 7.93-7.88 (m, 1H), 7.80 (d, J = 7.7 Hz, 1H), 7.64 (d, J = 7.9 Hz, 1H), 7.58-7.51 (m, 2H), 7.48-7.37 (m, 3H), 7.31-7.27 (m, 1H), 7.15 (t, J = 7.5 Hz, 1H), 6.79 (brs, NH), 6.55 (s, 1H), 4.31 (s, 2H), 3.56 (dd, J = 11.1, 5.4 Hz, 2H), 3.00 (t, J = 5.7 Hz, 2H). | 344 [M+H] |
| 55 | N-(2-(4-benzylpiperidin-1-yl)ethyl) benzo[d]thia zol-2-carboxamide | ¹H NMR (400 MHz, CD₃OD) δ 8.12 (d of d, J = 8.1 Hz, 14.5 Hz, 2H), 7.64 - 7.53 (m, 2H), 7.28 - 7. 25 (m, 2H), 7.18 - 7.15 (m, 3H), 3.61 (t, J = 6.8 Hz, 2H), 3.04 (d, J = 11.7 Hz, 2H), 2.65 (t, J = 6.8 Hz, 2H), 2.57 (d, J = 6.8 Hz, 2H), 2.08 (d of t, J = 2.0 Hz, 11.7 Hz, 2H), 1.69 - 1.57 (m, 3H), 1.40 - 1.33 (m, 2H) | 380 (M+H) |
| 56 | N-(2-(4-benzylpiperidin-1-yl)ethyl)-1H-pyrrolo[2,3-b]pyridin -2-carboxamide | ¹H NMR (400 MHz, CD₃OD) δ 8.35 (d of d, J = 1.5 Hz, 4.7 Hz, 1H), 8.11 (d of d, J = 1.5 Hz, 8.0 Hz, 1H), 7.28 - 7.25 (m, 2H), 7.20 - 7.15 (m, 4H), 7.09 (s, 1H), 3.58 (t, J = 6.9 Hz, 2H), 3.04 (d, J = 11.7 Hz, 2H), 2.64 (t, J = 6.9 Hz, 2H), 2.57 (d, J = 6.9 Hz, 2H), 2.08 (t, J = 11.7 Hz, 2H), 1.70 - 1.57 (m, 3H), 1.33 (d of q, J = 3.8 Hz, 12.3 Hz, 2H) | 363 (M+H) |
| 57 | N-(2-(4-benzylpiperidin-1-yl)ethyl) indolin-2-carboxamide | ¹H NMR (400 MHz, CDCl₃) δ 7.41 (s, 1H), 7.31 (d, J = 7.4 Hz, 1H), 7.21 (t, J = 7.3 Hz, 1H), 7.17 - 7.07 (m, 4H), 6.84 (t, J = 7.4 Hz, 1H), 6.77 (d, J = 7.7 Hz, 1H), 4.43 (td, J = 10.5, 4.9 Hz, 1H), 4.20 (d, J = 4.4 Hz, 1H), 3.60 (dd, J = 16.3, 10.8 Hz, 1H), 3.38 (pd, J = 13.1, 6.1 Hz, 2H), 3.11 (dd, J = 16.3, 8.5 Hz, 1H), 2.94 - 2.74 (m, 2H), 2.54 (d, J = 7.0 Hz, 2H), 2.46 (td, J = 6.3, 1.6 Hz, 2H), 1.93 (q, J = 11.4 Hz, 2H), 1.60 (d, J = 15.5 Hz, 2H), 1.52 (ddd, J = 14.5, 7.3, 3.7 Hz, 1H), 1.33 - 1.13 (m, 2H). | 363.44 (M) |
| 58 | (R)-N-(2-(4-benzylpiperidin-1-yl)ethyl) indolin-2-carboxamide | ¹H NMR (400 MHz, CDCl₃) δ 7.42 (s, 1H), 7.31 (d, J = 7.4 Hz, 2H), 7.21 (t, J = 7.3 Hz, 1H), 7.18 - 7.09 (m, 4H), 6.84 (t, J = 7.4 Hz, 1H), 6.77 (d, J = 7.7 Hz, 1H), 4.43 (t, J = 11.5 Hz, 1H), 4.20 (s, 1H), 3.60 (dd, J = 16.3, 10.7 Hz, 1H), 3.46 - 3.29 (m, 2H), 3.10 (dd, J = 16.3, 8.6 Hz, 1H), 2.90 - 2.78 (m, 2H), 2.54 (d, J = 7.0 Hz, 2H), 2.46 (t, J = 6.4 Hz, 2H), 1.92 (q, J = 12.1 Hz, 2H), 1.60 (d, J = 15.4 Hz, 2H), 1.55 - 1.44 (m, 1H), 1.34 - 1.14 (m, 3H). | 364.03 (M+H) |
| 59 | N-(2-(4-benzylpiperidin-1-yl)ethyl) -5-methoxy-1H-benzo[d]imidazol-2-carboxamide | ¹H NMR (400 MHz, CDCl₃) δ 10.51 (s, 1H), 7.30 - 7.26 (m, 5H), 7.19 (t, J = 7.4 Hz, 1H), 7.16 - 7.14 (m, 2H), 6.98 (s, 1H), 3.87 (s, 3H), 3.59 (dd, J = 11.9, 6.1 Hz, 2H), 2.93 (d, J = 11.5 Hz, 2H), 2.59 (t, J = 6.2 Hz, 2H), 2.55 (d, J = 7.0 Hz, 2H), 1.97 (t, J = 10.7 Hz, 2H), 1.65 (s, 2H), 1.59 - 1.52 (m, 2H), 1.35 (ddd, J = 15.2, 12.3, 3.7 Hz, 2H). | 393.20 (M+H) |
| 60 | N-(2-(4-benzylpiperidin-1-yl)ethyl) -5-fluoro-benzo[d]thia zol-2-carboxamide | ¹H NMR (400 MHz, CDCl₃) δ 7.91 (dd, J = 8.9, 5.0 Hz, 1H), 7.82 (s, 1H), 7.78 (dd, J = 9.2, 2.4 Hz, 1H), 7.31 - 7.26 (m, 3H), 7.22 - 7.17 (m, 1H), 7.17 - 7.14 (m, 2H), 3.58 (dd, J = 11.8, 6.0 Hz, 2H), 2.93 (d, J = 11.6 Hz, 2H), 2.61 - 2.56 (m, 4H), 1.99 (td, J = 11.6, 2.2 Hz, 2H), 1.67 (d, J = 12.5 Hz, 2H), 1.56 - 1.51 (m, 1H), 1.35 (ddd, J = 15.3, 12.2, 3.6 Hz, 2H). | 398.15 (M+H) |
| 61 | N-(2-(4-benzylpiperidin-1-yl)ethyl) -1H-benzo[d]imid azol-2-carboxamide | ¹H NMR (400 MHz, CDCl₃) δ 10.6 (s, 1H), 7.88 - 7.81 (m, 2H), 7.54 (d, J = 6.8 Hz, 1H), 7.37 - 7.33 (m, 2H), 7.29 - 7.27 (m, 1H), 7.20 - 7.13 (m, 3H), 3.60 (q, J = 6.0 Hz, 2H), 2.93 (d, J = 11.5 Hz, 2H), 2.59 (t, J = 6.2 Hz, 2H), 2.54 (d, J = 7.0 Hz, 2H), 1.97 (d of t, J = 2.1 Hz, 11.5 Hz, 2H), 1.65 - 1.61 (m, 2H), 1.56 - 1.50 (m, 1H), 1.37 (d of q, J = 3.3 Hz, 12.1 Hz, 2H) | 363 (M+H) |
| 62 | N-((1R,4S)-4-(3,4-dichlorophenyl) - 1,2,3,4-tetrahydronaphthalen-1-yl) -1H-indol-2-carboxamide | ¹H NMR (400 MHz, CDCl₃) δ 12.0 (s, 1H), 8.13 (d, J = 9.0 Hz, 1H), 7.76 (d, J = 7.8 Hz, 1H), 7.56 (d, J = 7.8 Hz, 1H), 7.40 - 7.31 (m, 4H), 7.21 - 7.15 (m 3H), 6.93 - 6.85 (m, 2H), 5.59 - 5.53 (m, 1H), 4.17 (t, J = 5.7 Hz, 1H), 2.38 - 2.26 (m, 2H), 2.04 - 1.91 (m, 2H). | 436 (M+H) |
| 63 | N-(2-(4-benzylpiperidin-1-yl)ethyl) -5,6-dihydroxy-1H-indol-2-carboxamide | ¹H NMR (400 MHz, MeOD) δ 7.30 (t, J = 7.5 Hz, 2H), 7.20 (t, J = 7.2 Hz, 3H), 6.93 (s, 1H), 6.89 (s, 1H), 6.85 (s, 1H), 3.67 (t, J = 5.9 Hz, 2H), 3.48 (d, J = 11.5 Hz, 2H), 3.08 (s, 2H), 2.70 (t, J = 11.8 Hz, 2H), 2.62 (d, J = 6.5 Hz, 2H), 1.85 (d, J = 13.2 Hz, 2H), 1.82 - 1.74(m, 1H), 1.49 (dd, J = 24.8, 11.9 Hz, 2H). | 394.03 (M+H) |
| 64 | N-(2-(4-benzylpiperidin-1-yl)ethyl) -5-hydroxy-1H-benzo[d]imid azol-2-carboxamide | ¹H NMR (400 MHz, CDCl₃) δ 7.30 - 7.23 (m, 4H), 7.19 - 7.16 (m, 1H), 7.12 (d, J = 6.9 Hz, 2H), 6.85 (s, 1H), 3.61 (d, J = 5.7 Hz, 2H), 2.99 (d, J = 11.1 Hz, 2H), 2.63 (t, J = 5.8 Hz, 2H), 2.51 (d, J = 7.0 Hz, 2H), 2.04 - 1.96 (m, 2H), 1.64 (d, J = 11.6 Hz, 2H), 1.56 - 1.52 (m, 1H), 1.36 (ddd, J = 14.1, 12.3, 3.1 Hz, 2H). | 379.19 (M+H) |
| 65 | N-(2-(4-(3,4-dichloro-benzyl)piper idin-1-yl)ethyl) -5, 6-dihydroxy-1H-indol-2-carboxamide | ¹H NMR (400 MHz, MeOD) δ 7.42 (d, J = 8.2 Hz, 1H), 7.36 (d, J = 2.0 Hz, 1H), 7.12 (dd, J = 8.2, 2.0 Hz, 1H), 6.93 (s, 1H), 6.85 (d, J = 3.1 Hz, 2H), 3.54 (t, J = 6.9 Hz, 2H), 3.03 (d, J = 11.8 Hz, 2H), 2.61 (t, J = 6.9 Hz, 2H), 2.56 (d, J = 6.9 Hz, 2H), 2.09 (t, J = 10.9 Hz, 2H), 1.65 (t, J = 11.1 Hz, 2H), 1.62 - 1.56 (m, 1H), 1.40 - 1.28 (m, 2H). | 462.12 (M+H) |
| 66 | N-(2-(4-benzylpiperidin-1-yl)ethyl) -5-hydroxy-1H-indol-2-carboxamide | ¹H NMR (400 MHz, MeOD) δ 7.27 (t, J = 7.9 Hz, 3H), 7.19 - 7.16 (m, 3H), 6.95 (d, J = 2.1 Hz, 1H), 6.89 (s, 1H), 6.81 (dd, J = 8.7, 2.1 Hz, 1H), 3.56 (t, J = 6.6 Hz, 2H), 3.05 (d, J = 11.3 Hz, 2H), 2.63 (t, J = 6.7 Hz, 2H), 2.58 (d, J = 6.9 Hz, 2H), 2.08 (dd, J = 22.8, 10.2 Hz, 2H)), 1.69 (d, J = 13.7 Hz, 2H), 1.66 - 1.55 (m, 1H), 1.41 - 1.29 (m, 2H). | 378.00 (M+H) |
| 67 | N-(2-(4-benzylpiperidin-1-yl)ethyl) -5-butoxy-1H-indol-2-carboxamide | ¹H NMR (400 MHz, CDCl₃) δ 9.05 (s, 1H), 7.30 (dd, J = 14.8, 8.0 Hz, 3H), 7.20 (t, J = 7.4 Hz, 1H), 7.16 (d, J = 7.6 Hz, 2H), 7.08 (s, 1H), 6.96 (dd, J = 8.9, 2.0 Hz, 1H), 6.86 (s, 1H), 6.74 (s, 1H), 4.00 (t, J = 6.5 Hz, 2H), 3.53 (dd, J = 11.1, 5.6 Hz, 2H), 2.91 (d, J = 11.4 Hz, 2H), 2.56 (t, J = 6.6 Hz, 4H), 1.98 (t, J = 11.1 Hz, 2H), 1.85 - 1.76 (m, 2H), 1.68 (d, J = 15.6 Hz, 2H), 1.57 - 1.48 (m, 3H), 1.31 (ddd, J = 15.3, 12.6, 3.6 Hz, 2H), 1.00 (t, J = 7.4 Hz, 3H). | 434.14 (M+H) |
| 68 | N- (2-(methyl(naph thalen-2-ylmethyl)amin o)ethyl)-1H-indol-2-carboxamide | ¹H NMR (400 MHz, CDCl₃) δ 8.93 (brs, 1H), 8.32 (d, J = 7.7 Hz, 1H), 7.93 (d, J = 7.5 Hz, 1H), 7.80 (d, J = 8.1 Hz, 1H), 7.63 - 7.55 (m, 3H), 7.44 - 7.36 (m, 3H), 7.30 - 7.22 (m, 1H), 7.15 (t, J = 7.6 Hz, 1H), 6.39 (brs, 1H), 5.86 (s, 1H), 3.99 (s, 2H), 3.46 (dd, J = 10.4, 4.8 Hz, 2H), 2.68 (t, J = 5.6 Hz, 2H), 2.43 (s, 3H). | 358.03 (M+H) |
| 69 | N-(2-(4-benzylpiperidin-1-yl)ethyl) cyclopropanecarboxamide | ¹H NMR (400 MHz, CDCl₃) δ 7.29 - 7.26 (m, 2H), 7.20 - 7.12 (m, 3H), 6.29 (broad s, 1H), 3.34 (q, J = 5.7 Hz, 2H), 2.86 (d, J = 11.7 Hz, 2H), 2.54 (d, J = 7.0 Hz, 2H), 2.44 (t, J = 6.0 Hz, 2H), 1.95 - 1.89 (m, 3H), 1.66 - 1.50 (m, 3H), 1.40 - 1.23 (m, 3H), 0.97 - 0.93 (m, 2H), 0.73 - 0.69 (m, 2H) | 287 (M+H) |
| 70 | N-(2-(4-benzylpiperidin-1-yl)ethyl) -1-methylcyclopropanecarboxamide | ¹H NMR (400 MHz, CDCl₃) δ 7.30 - 7.27 (m, 2H), 7.20 - 7.13 (m, 3H), 6.52 (s, 1H), 3.32 (d of d, J = 6.0 Hz, 11.0 Hz, 2H), 3.18 - 3.14 (m, 2H), 2.85 (d, J = 11.5 Hz, 2H), 2.53 (d, J = 7.2 Hz, 2H), 2.46 (t, J = 6.0 Hz, 2H), 2.04 - 1.92 (m, 2H), 1.58 - 1.49 (m, 1H), 1.31 (s, 3H), 1.30 - 1.22 (m, 2H), 1.16 (d of d, J = 3.8 Hz, 6.4 Hz, 2H), 0.55 (d of d, J = 3.8 Hz, 6.4 Hz, 2H). | - |
| 71 | N-(2-(4-benzylpiperidin-1-yl)ethyl) picolinamide | ¹H NMR (400 MHz, CDCl₃) δ 8.56 (d, J = 4.6 Hz, 1H), 8.30 (s, 1H), 8.18 (d, J = 7.9 Hz, 1H), 7.83 (d of t, J = 1.6 Hz, 7.7 Hz, 1H), 7.43 - 7.39 (m, 1H), 7.29 - 7.25 (m, 2H), 7.20 - 7.13 (m, 3H), 3.56 (q, J = 5.8 Hz, 2H), 2.93 (d, J = 11.5 Hz, 2H), 2.59 - 2.53 (m, 3H), 2.00 -1.87 (m, 3H), 1.66 - 1.48 (m, 3H), 1.38 - 1.28 (m, 2H) | 324 (M+H) |
| 72 | N-(2-(4-(1H-indol-3-yl)piperi din-1-yl)ethyl) -4-butoxyben zamide | ¹H NMR (400 MHz, CDCl₃) δ 7.75 (d, J = 8.6 Hz, 1H), 7.66 (d, J = 7.8 Hz, 1H), 7.38 (d, J = 8.0 Hz, 1H), 7.20 (t, J = 7.6 Hz, 1H), 7.12 (t, J = 7.4 Hz, 1H), 7.00 (s, 1H), 6.93 (d, J = 8.5 Hz, 1H), 6.83 - 6.78 (m, 1H), 4.01 (t, J = 6.5 Hz, 1H), 3.57 (dd, J = 10.2, 5.2 Hz, 1H), 3.06 (d, J = 11.5 Hz, 1H), 2.88 (t, J = 13.2 Hz, 1H), 2.65 (t, J = 6.0 Hz, 1H), 2.23 (dd, J = 15.2, 8.7 Hz, 2H), 2.11 (d, J = 15.3 Hz, 1H), 2.02 (s, 1H), 1.84 - 1.74 (m, 2H), 1.50 (dd, J = 15.1, 7.4 Hz, 1H), 0.98 (t, J = 7.4 Hz, 2H). | 420.09 (M+H) |
| 73 | N-(2-(4-benzylpiperidin-1-yl)ethyl) -1H-indol-3-carboxamide | ¹H NMR (400 MHz, CDCl₃) δ 8.56 (s, 1H), 8.01 (d, J = 7.7 Hz, 1H), 7.86 (d, J = 2.4 Hz, 1H), 7.46 (d, J = 7.6 Hz, 1H), 7.31 - 7.27 (m, 3H), 7.21 (dd, J = 13.7, 6.4 Hz, 1H), 7.16 (d, J = 7.3 Hz, 2H), 6.93 (s, 1H), 3.58 (dd, J = 10.9, 5.5 Hz, 2H), 2.95 (d, J = 11.4 Hz, 2H), 2.59 (dd, J = 14.5, 6.6 Hz, 4H), 2.02 (t, J = 11.3 Hz, 3H), 1.70 (d, J = 13.0 Hz, 2H), 1.34 (dd, J = 22.4, 10.0 Hz, 4H). | 361.94 (M) |
| 74 | N-(2-(4-benzylpiperidin-1-yl)ethyl) -1-methyl-1H-indol-2-carboxamide | ¹H NMR (400 MHz, CDCl₃) δ 7.67 (d, J = 7.9 Hz, 1H), 7.39 (d, J = 8.4 Hz, 1H), 7.33 (d, J = 7.1 Hz, 1H), 7.31 - 7.26 (m, 4H), 7.21 (d, J = 7.1 Hz, 1H), 7.16 (t, J = 7.1 Hz, 3H), 6.88 (s, 1H), 4.07 (s, 3H), 3.53 (d, J = 5.2 Hz, 2H), 2.95 (d, J = 9.4 Hz, 2H), 2.60 (s, 2H), 2.56 (d, J = 7.0 Hz, 2H), 2.02 (t, J = 11.8 Hz, 2H), 1.68 (d, J = 12.4 Hz, 2H), 1.61 - 1.44 (m, 1H). | 376.65 (M+H) |
| 75 | N-(2-(4-benzylpiperidin-1-yl)ethyl) -1H-pyrrol-2-carboxamide | ¹H NMR (400 MHz, CDCl₃) δ 9.35 (s, 1H), 7.29 - 7.28 (m, 1H), 7.21 - 7.13 (m, 3H), 6.91 - 6.90 (m, 1H), 6.70 (broad s, 1H), 6.58 (apparent s, 1H), 6.25 - 6.23 (m, 1H), 3.49 (q, J = 5.7 Hz, 2H), 2.91 (d, J = 11.4 Hz, 2H), 2.55 - 2.53 (m, 4H),2.04 - 1.95 (m, 3H), 1.58 - 1.51 (m, 1H), 1.36 - 1.29 (m, 3H) | 312 (M+H) |
| 76 | 3- (4-benzylpiperidin-1-yl)-N-(1H-indol-2-yl)propan eamide | ¹H NMR (400 MHz, MeOD) δ 7.41 (d, J = 7.5 Hz, 1H), 7.32 (dd, J = 15.7, 7.5 Hz, 3H), 7.22 (t, J = 7.3 Hz, 4H), 7.02 (dt, J = 14.8, 7.0 Hz, 2H), 3.65 (d, J = 12.0 Hz, 2H), 3.49 (t, J = 6.7 Hz, 2H), 3.04 - 2.92 (m, 4H), 2.65 (d, J = 6.5 Hz, 2H), 1.94 (m, 3H), 1.61 - 1.46 (m, 2H). | 362.12 (M+H) |
| 77 | 4-(4-benzylpiperidin-1-yl)-N-(1H-indol-2-yl)-4-oxobutanamide | ¹H NMR (400 MHz, MeOD) δ 7.27 (d, J = 7.2 Hz, 1H), 7.21 (d, J = 7.8 Hz, 1H), 7.15 (t, J = 7.4 Hz, 2H), 7.05 (dd, J = 14.2, 7.2 Hz, 4H), 6.95 - 6.81 (m, 2H), 5.89 (s, 4H), 4.38 (d, J = 13.0 Hz, 1H), 3.91 (d, J = 13.5 Hz, 1H), 2.94 (t, J = 11.9 Hz, 1H), 2.75 - 2.64 (m, 2H), 2.62 - 2.56 (m, 2H), 2.50 (d, J = 13.7 Hz, 1H), 2.47 - 2.38 (m, 2H), 1.78 - 1.66 (m, 1H), 1.64 - 1.52 (m, 2H), 1.17 - 1.07 (m, 1H), 1.00 (dt, J = 12.5, 8.3 Hz, 1H). | 390.07 (M+H) |
| 78 | N-(2-(4-benzylpiperidin-1-yl)ethyl) benzo[d][1,3 ]dioxol-5-carboxamide | ¹H NMR (400 MHz, CDCl₃) δ 7.30 (dd, J = 6.3, 1.7 Hz, 1H), 7.29 - 7.24 (m, 3H), 7.19 (t, J = 7.3 Hz, 1H), 7.16 - 7.13 (m, 2H), 6.84 (d, J = 8.0 Hz, 1H), 6.74 (br, 1H), 6.03 (s, 2H), 3.48 (q, J = 5.4, 5.6 Hz, 2H), 2.88 (d, J = 11.5 Hz, 2H), 2.55 - 2.52 (m, 4H), 1.95 (td, J = 11.6, 1.5 Hz, 2H), 1.65 (s, 2H), 1.60 - 1.50 (m, 1H), 1.27 (qd, J = 12.1, 3.6 Hz, 2H). | 367.08 |
| 79 | N-(2-(4-benzylpiperidin-1-yl)ethyl) -2,3-dihydro-6H-[1,4]diox yno [2, 3-f]indol-7-carboxamide | ¹H NMR (400 MHz, CDCl₃) δ 8.89 (brs, 1H), 7.32 - 7.27(m, 2H), 7.24 - 7.13 (m, 3H), 7.10 (s, 1H), 6.89 (s, 1H), 6.82 (brs, 1H), 6.68 (d, J = 1.5 Hz, 1H), 4.29 (q, J = 5.4 Hz, 4H), 3.51 (q, J = 5.6 Hz, 2H), 2.90 (d, J = 11.5 Hz, 2H), 2.63 - 2.49 (m, 4H), 1.97 (t, J = 10.9 Hz, 2H), 173 - 1.65 (m, 2H), 1.60 - 1.49 (m, 1H), 1.37 - 1.25 (m, 2H). | 420.09 (M+H) |
| 80 | N-(2-(4-benzylpiperidin-1-yl)ethyl) -5H-[1,3]diox olo[4,5-f]indol-6-carboxamide | ¹H NMR (400 MHz, CDCl₃) δ 9.20 (brs, 1H), 7.32 - 7.27 (m, 2H), 7.23 - 7.13 (m, 3H), 6.99 (s, 1H), 6.86 (s, 1H), 6.80 (brs, 1H), 6.72 (d, J = 1.9 Hz, 1H), 5.96 (s, 2H), 3.52 (q, J = 5.5 Hz, 2H), 2.91 (d, J = 11.4 Hz, 2H), 2.61 - 2.50 (m, 4H), 1.97 (t, J = 10.7 Hz, 2H), 1.71 - 1.68 (m, 2H), 1.61 - 1.51 (m, 1H), 1.38 - 1.27 (m, 2H). | 406.10 (M+H) |
| 81 | 2-((2-(4-benzylpiperidin-1-yl)ethyl) carbamoyl)-1H-indol-5-carboxylic acid | ¹H NMR (400 MHz, MeOD) δ 8.30 (d, J = 0.9 Hz, 1H), 7.90 (dd, J = 8.6, 1.6 Hz, 1H), 7.39 (d, J = 8.6 Hz, 1H), 7.28 - 7.23 (m, 2H), 7.18 - 7.13 (m, 3H), 7.11 (d, J = 0.7 Hz, 1H), 3.58 (t, J = 6.8 Hz, 2H), 3.13 - 3.09 (m, 2H), 2.75 - 2.68 (m, 2H), 2.57 (d, J = 6.8 Hz, 2H), 2.22 - 2.13 (m, 2H), 1.73 - 1.66 (m, 2H), 1.66 - 1.58 (m, 1H), 1.42 - 1.32 (m, 2H). | 406.24 (M+H) |
| 82 | N2-(2-(4-benzylpiperidin-1-yl)ethyl) -1H-indol-2,5-dicarboxamide | ¹H NMR (400 MHz, MeOD) δ 8.24 (d, J = 1.2 Hz, 1H), 7.77 (dd, J = 8.7, 1.7 Hz, 1H), 7.49 (d, J = 8.7 Hz, 1H), 7.25 (dd, J = 9.5, 5.5 Hz, 2H), 7.16 (dd, J = 7.5, 5.1 Hz, 4H), 3.58 (t, J = 6.8 Hz, 2H), 3.08 (d, J = 11.4 Hz, 2H), 2.72 - 2.65 (m, 2H), 2.57 (d, J = 6.9 Hz, 2H), 2.19 - 2.10 (m, 2H), 1.70 (d, J = 13.3 Hz, 2H), 1.66 - 1.57 (m, 1H), 1.42 - 1.31 (m, 2H). | 405.25 (M+H) |

### <Comparative Example 1> Preparation of N-(2-(4-benzylpiperidin-1-yl)ethyl)-1H-indol-2-carboxamide

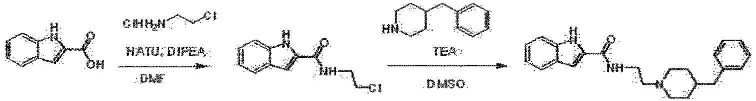

Step 1: 1H-indol-2-carboxylic acid (1 eq), 2-chloroethan-1-amine hydrochloride (1.5 eq), HATU (2.5 eq) and DIPEA (3 eq) were dissolved in DMF, and the reactants were stirred at room temperature for 12 hours. After finishing the reaction, the solvent was removed under a reduced pressure. The resultant was extracted with dichloromethane, an organic layer was washed with H₂O and brine in order, and dried over Na₂SO₄, and the solvent was removed under a reduced pressure. Then, the reaction mixture was separated and purified by MPLC to obtain N-(2-chloroethyl)-1H-indol-2-carboxamide.

Step 2: N-(2-chloroethyl)-1H-indol-2-carboxamide (1 eq) was dissolved in DMF, and TEA (4 eq) and 4-benzylpiperidine (2 eq) were added thereto, followed by stirring at 60°C for 12 hours. After finishing the reaction, the solvent was removed under a reduced pressure. The resultant was extracted with ethyl acetate, an organic layer was washed with H₂O and brine in order, and dried over Na₂SO₄, and the solvent was removed under a reduced pressure. Then, the reaction mixture was separated and purified by MPLC to obtain the compound of Comparative Example 1.

### <Experimental Example 1> Evaluation of Triple Reuptake Inhibitory Effect

In order to evaluate the reuptake inhibitory effect of the compounds of the Examples according to the present invention and the Comparative Example on dopamine, norepinephrine and serotonin, the experiments below were performed, and the results are shown in Table 15.

To evaluate the reuptake inhibitory effect on dopamine, the dopamine transporter cDNA was transfected using the HEK-293 cell line, and then sub-seeded in a 24 well plate the next day. After removing the medium, each compound of the Examples or Comparative Examples diluted to 1 µM in uptake buffer was added to a concentration of 200 µl/well and cultured at 37°C for 15 minutes. 100 µl of uptake buffer containing 20-30 nM [3H]-DA was added and cultured at 37°C for 5 minutes. The reaction solution was quickly removed and washed three times with 1 ml ice-cold uptake buffer. After dissolving the resultant in 1% SDS (0.5 ml), the degree of binding of radioisotope-labeled dopamine to the transporter was measured.

For norepinephrine (NE) and serotonin (5-HT), the degree of binding of radioisotope-labeled norepinephrine or serotonin to the transporter was measured in the same manner as above, except that cells transfected with NE or 5-HT transporter cDNA were used and [3H]-NE or [3H]-5-HT was used as a substrate.

In addition, when evaluating the reuptake inhibitory effect on dopamine, norepinephrine, and serotonin, the same method was performed without treating each compound of the Examples or Comparative Examples as a control, the degree of binding of radioisotope-labeled dopamine, norepinephrine, and serotonin to the transporter was measured, and by using this as a standard (100%), the triple reuptake inhibitory effect was evaluated as a relative ratio (Ratio%). That is, in the control group, the binding of dopamine, norepinephrine, and serotonin to transporters is not inhibited, but when each compound of the Examples is treated and thus the binding is inhibited, the Ratio% value decreases.

Meanwhile, the Ratio% values were compared, and in the case of the compounds of Examples 6, 9, 10, 19, 20, 22, 24, 28, 30, 31, 32, 33, 35, 41, 42, 48, 49, 51, 55, 56, 59, 61, 63, 64, 65, 66, 74 and 76 with excellent reuptake inhibitory effects on dopamine, norepinephrine and serotonin, the concentration at which the Ratio% is 50% was calculated using a total of 6 concentrations (1 nM, 5 nM, 10 nM, 50 nM, 100 nM, and 1 µM) and expressed as IC₅₀. In addition, in the case of the compound of Comparative Example 1, IC₅₀ was obtained by the same method as the Example Compounds.

**[Table 15]**

| | Triple reuptake inhibition (Ratio %) | | | Triple reuptake inhibition (IC₅₀, µM) | | |
|---|---|---|---|---|---|---|
| Example | SERT | NET | DAT | SERT | NET | DAT |
| 1 | 80.51 | 123.51 | 104.02 | | | |
| 2 | 78.42 | 120.40 | 107.77 | | | |
| 3 | 77.91 | 110.56 | 104.38 | | | |
| 4 | 73.53 | 121.24 | 108.51 | | | |
| 5 | 76.90 | 94.24 | 96.26 | | | |
| 6 | 73.84 | 34.16 | 57.66 | 13.96 | | 3.93 |
| 7 | 103.19 | 60.83 | 82.42 | | | |
| 8 | 86.83 | 95.78 | 97.06 | | | |
| 9 | 9.92 | 98.42 | 78.56 | 3.073 | | 2.907 |
| 10 | 37.57 | 76.85 | 44.72 | | 14.26 | 4.292 |
| 11 | 63.40 | 82.25 | 75.29 | | | |
| 12 | 93.46 | 94.42 | 103.97 | | | |
| 13 | 89.10 | 94.42 | 106.09 | | | |
| 14 | 88.61 | 105.49 | 94.20 | | | |
| 15 | 114.75 | 72.94 | 85.21 | | | |
| 16 | 113.34 | 82.44 | 85.54 | | | |
| 17 | 4.81 | 88.67 | 66.05 | | | |
| 18 | 47.45 | 67.88 | 68.93 | | | |
| 19 | 40.39 | 94.93 | 36.79 | 0.51 | >10 | 0.59 |
| 20 | 83.11 | 85.70 | 43.53 | | | 1.37 |
| 21 | 86.98 | 97.95 | 94.30 | | | |
| 22 | 52.47 | 91.21 | 63.72 | >10 | | |
| 23 | 85.13 | 99.59 | 70.42 | | | |
| 24 | 59.92 | 107.75 | 43.28 | >10 | | 8.03 |
| 25 | 97.46 | 76.45 | 76.50 | | | |
| 26 | 113.81 | 92.07 | 124.32 | | | |
| 27 | 75.39 | 69.72 | 71.77 | | | |
| 28 | 82.81 | 88.77 | 37.16 | | | 0.225 |
| 29 | 94.24 | 97.94 | 113.83 | | | |
| 30 | 75.29 | 94.09 | 49.44 | | 7.911 | 0.984 |
| 31 | 58.09 | 72.60 | 31.52 | | 4.347 | 1.726 |
| 32 | 54.81 | 66.78 | 28.03 | 2.044 | 1.474 | 0.824 |
| 33 | 48.73 | 65.51 | 25.55 | 1.328 | 1.697 | 0.331 |
| 34 | 90.64 | 70.66 | 55.54 | | | |
| 35 | 36.24 | 16.10 | 7.77 | 0.415 | 0.386 | 0.248 |
| 36 | 111.92 | 90.99 | 84.74 | | | |
| 37 | 32.42 | 76.15 | 38.64 | | | |
| 38 | 70.46 | 70.02 | 43.94 | | | |
| 39 | 76.27 | 63.99 | 40.9 | | | |
| 40 | 92.65 | 59.50 | 35.13 | | | |
| 41 | 48.50 | 31.85 | 12.3 | 0.525 | 0.371 | 0.171 |
| 42 | 46.64 | 44.53 | 45.67 | 0.680 | 0.171 | 0.338 |
| 43 | 81.76 | 56.98 | 30.29 | | | |
| 44 | 94.19 | 85.48 | 73.05 | | | |
| 45 | 91.05 | 102.98 | 110.91 | | | |
| 46 | 42.91 | 110.55 | 77.49 | | | |
| 47 | 75.98 | 94.59 | 41.80 | | | |
| 48 | 5.27 | 9.75 | 2.88 | 0.123 | 0.398 | 0.028 |
| 49 | 62.50 | 26.73 | 24.67 | 1.472 | 0.561 | 0.138 |
| 50 | 100.83 | 93.21 | 108.32 | | | |
| 51 | 27.71 | 83.07 | 41.61 | 0.331 | | 0.636 |
| 52 | 93.19 | 82.74 | 85.39 | | | |
| 53 | 108.82 | 69.01 | 86.48 | | | |
| 54 | 84.69 | 92.95 | 79.32 | | | |
| 55 | 53.01 | 57.47 | 39.32 | 3.014 | 2.483 | 2.64 |
| 56 | 76.79 | 81.84 | 37.06 | | | 1.48 |
| 57 | 77.95 | 124.77 | 60.52 | | | |
| 58 | 105.84 | 82.39 | 66.26 | | | |
| 59 | 45.80 | 43.43 | 17.60 | 0.198 | 0.257 | 0.198 |
| 60 | 53.40 | 50.81 | 68.95 | | | |
| 61 | 62.21 | 73.75 | 32.78 | 2.28 | 3.17 | 0.97 |
| 62 | 94.51 | 144.33 | 79.43 | | | |
| 63 | 38.25 | 48.43 | 13.34 | 0.369 | 0.092 | 0.087 |
| 64 | 47.72 | 57.21 | 22.84 | 0.237 | 0.543 | 0.362 |
| 65 | 10.79 | 73.83 | 7.57 | 0.044 | 5.73 | 0.049 |
| 66 | 38.79 | 21.59 | 8.78 | 0.265 | 0.167 | 0.036 |
| 67 | 53.46 | 92.60 | 56.05 | | | |
| 68 | 87.07 | 100.99 | 90.63 | | | |
| 69 | 113.80 | 86.02 | 92.83 | | | |
| 70 | 102.65 | 116.85 | 107.49 | | | |
| 71 | 67.98 | 105.84 | 117.41 | | | |
| 72 | 17.44 | 97.62 | 58.16 | | | |
| 73 | 88.39 | 57.01 | 33.58 | | | |
| 74 | 67.26 | 28.47 | 39.87 | 3.41 | 0.337 | 0.509 |
| 75 | 98.97 | 67.08 | 83.55 | | | |
| 76 | 68.60 | 32.60 | 36.87 | 0.811 | 0.496 | 0.278 |
| 77 | 98.61 | 91.05 | 111.32 | | | |
| 81 | 87.86 | 95.95 | 70.22 | | | |
| 82 | 41.20 | 37.73 | 47.70 | | | |
| Comp. Example 1 | | | | 5.347 | 0.524 | 4.18 |

In Table 16 above, SERT means a serotonin transporter, NET means a norepinephrine transporter, and DAT means a dopamine transporter.

As shown in Table 15 above,
it can be seen that the compounds of the Examples of the present invention and the Comparative Example can inhibit reuptake of serotonin, norepinephrine, and dopamine, and IC₅₀ value is lower for Examples 28, 35, 41, 42, 48, 51, 59, 63, 66 and 76 than Comparative Example 1.

Therefore, the compound represented by Formula 1 according to the present invention is excellent in the triple reuptake inhibitory effect of serotonin, norepinephrine, and dopamine, and thus can be useful in the treatment of mental illness.

### <Experimental Example 2> Evaluation on hyperactivity inhibitory effect

In order to evaluate the hyperactivity inhibitory effect of the compounds of Example 35 and Example 66 according to the present invention or Comparative Example 1, an experiment was performed as follows, and the results are shown in FIG. 1. An open field test is an experimental method utilized for searching for excess and decline in motor function, calmness, excitement, avoidance degree and toxicity.

The observation of the hyperactivity and behavior analysis of animals were conducted using an EthoVision System (Noldus IT b.v., Netherlands) that is a type of an open field test. The animals used for the experiment were male ICR mice of 4 weeks old. To induce hyperactivity, animal models intraperitoneally administered with 0.15 mg/kg of MK801 (drug name: dizocilpine) were used, and the compounds of Example 35, Example 66 or Comparative Example 1 was intraperitoneally administered by 5 mg/kg, 30 minutes ago the administration of MK801. Male ICR mice administered with 10% DMSO and 10% Tween-80 as the solvents dissolving a drug were considered a vehicle, and male ICR mice administered with MK801 without treatment with the Example Compound or Comparative Compound were considered a positive control. After that, in the center of a test box (40 x 40 x 30 cm) manufactured using black acryl, the mice under test were put and allowed to fit for 15 minutes, and behavior patterns were observed, recorded and analyzed for 15 minutes. Comparative analysis was conducted by measuring distance moved and movement duration.

The distance moved (cm) of the mice was increased by about two times for all groups treated with MK801 in contrast to the vehicle, and the movement was reduced for the group treated with the compound of Example 35 and the group treated with the compound of Example 66 in contrast to the positive control treated with only MK801 and the group treated with the compound of Comparative Example 1. Particularly, the distance moved was about 7000 cm for the vehicle, about 14000 cm for the positive control treated with only MK801, about 13400 cm for the group treated with the compound of Comparative Example 1, about 10000 cm for the group treated with the compound of Example 35, and about 8800 cm for the group treated with the compound of Example 66.

The movement duration (sec) of the mice was shown high in all groups treated with MK801 in contrast to the vehicle, and shown less in the group treated with the compound of Example 35 and the group treated with the compound of Example 66 in contrast to the positive group treated with only MK801 and the group treated with the compound of Comparative Example 1. Particularly, the vehicle showed about 880 seconds (s), the positive control treated with only MK801 showed about 1070 seconds, the group treated with the compound of Comparative Example 1 showed about 1100 seconds, the group treated with the compound of Example 35 showed about 950 seconds, and the group treated with the compound of Example 66 showed about 880 seconds (see FIG. 1).

The results show that the hyperactivity of the mice induced with MK801 was not inhibited by the treatment with the compound of Comparative Example 1 but was significantly inhibited by the treatment with the compound of Example 35 or Example 66. Through this, it could be found that the compound of Example 35 or 66 is excellent in the triple reuptake inhibitory effect of serotonin, norepinephrine and dopamine than the compound of Comparative Example 1, and can inhibit the hyperactivity of the mice.

Accordingly, the compound represented by Formula 1 according to the present invention is excellent in the triple reuptake inhibitory effect of serotonin, norepinephrine, and dopamine, and thus can be useful in the treatment of mental illness.

### <Experimental Example 3> Evaluation on causing sleep disorder or not

In order to evaluate the causing of sleep disorder or not as the side effect of the compound of Example 35, Example 66 according to the present invention or Comparative Example 1, an experiment was performed as follows, and the results are shown in FIG. 2. Pentobarbital sodium is a drug inducing sleep, and by observing the behavior of sleep-induced animals using the drug, the causing of sleep disorder or not can be evaluated. In addition, caffeine is well-known as a drug causing sleep disorder, and diazepam is well-known as a sleeping pill having sleep retention effect.

As experimental animals, male ICR mice of 3 weeks old were prepared and, 8 mice per group were prepared. A vehicle taking only water, groups intraperitoneally administered once a day for 5 days with 10 mg/kg of caffeine, 2 mg/kg of diazepam, 10 mg/kg of Comparative Example 1, and 10 mg/kg of Example 35 and 10 mg/kg of Example 66, were prepared. After 5 days, 42 mg/kg of pentobarbital sodium was intraperitoneally administered to the mice of each group to induce sleep.

After treatment with pentobarbital sodium, if a mouse was turned upside down showing its belly, it was considered sleep, and if a mouse changed posture while showing its back, it was considered to wake up completely. Sleep onset time and sleep duration were confirmed.

The sleep onset time of the mice was the lowest for the group treated with diazepam, the highest for the group treated with caffeine, higher for the group treated with Comparative Example 1 than the vehicle, and similar for the group treated with Example 35 or Example 66 as the vehicle. Particularly, the vehicle was about 225 seconds, the group treated with caffeine was about 400 seconds, the group treated with diazepam was about 170 seconds, the group treated with Comparative Example 1 was about 300 seconds, the group treated with Example 35 was about 220 seconds, and the group treated with Example 66 was about 230 seconds.

The sleep duration of the mice was the highest for the group treated with diazepam, the lowest for the group treated with caffeine, was lower for the group treated with Comparative Example 1 than the vehicle, and similar for the group treated with Example 35 or Example 66 as the vehicle. Particularly, the vehicle was about 2100 seconds, the group treated with caffeine was about 790 seconds, the group treated with diazepam was about 2900 seconds, the group treated with Comparative Example 1 was about 1100 seconds, the group treated with Example 35 was about 1950 seconds, and the group treated with Example 66 was about 2090 seconds (see FIG. 2).

From the results, it is shown that if treated with the compound of Comparative Example 1, the sleep onset time of the mice increased and the sleep duration decreased to show sleep disorder, but if treated with the compound of Example 35 or 66, no change was induced on the sleep onset time and the sleep duration not to show sleep disorder. Through this, it can be found that the compound of Comparative Example 1 induced sleep disorder as side effect, but the compound of Example 35 or 66 did not induce sleep disorder.

Accordingly, the compound represented by Formula 1 according to the present invention is excellent in the triple reuptake inhibitory effect of serotonin, norepinephrine, and dopamine, and thus can be useful in the treatment of mental illness.

### INDUSTRIAL APPLICABILITY

The compound represented by Formula 1 according to the present invention is excellent in the triple reuptake inhibitory effect of serotonin, norepinephrine, and dopamine, and thus can be useful in the treatment of mental illness.

## Claims

1. A compound represented by the following Formula 1, an isomer thereof, a solvate thereof, a hydrate thereof, or a pharmaceutically acceptable salt thereof: 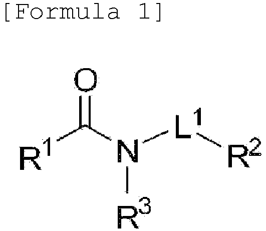 in Formula 1,
R¹ is unsubstituted or substituted cycloalkyl of C₃₋₆, unsubstituted or substituted aryl of C₆₋₁₀, unsubstituted or substituted heteroaryl of 5 to 14 atoms, containing one or more heteroatoms selected from the group consisting of N, S and O, substituted linear or branched C₁₋₈ alkyl, or substituted cycloalkenyl of C₄₋₆ to form aryl of C₆₋₁₀ together with substituted carbon atoms,
the substituted cycloalkyl is substituted with linear or branched C₁₋₈ alkyl, the substituted aryl is substituted with one or more substituents selected from the group consisting of linear or branched C₁₋₁₀ alkoxy and halogen, or substituted to form heterocycloalkenyl of C₃₋₆, containing one or more O together with substituted carbon atoms, the substituted heteroaryl is substituted with one or more substituents selected from the group consisting of unsubstituted or substituted linear or branched C₁₋₈ alkyl with one or more halogens, unsubstituted or substituted linear or branched C₁₋₁₀ alkoxy with aryl of C₆₋₁₀, C₁₋₁₀ carboxy, C₁₋₁₀ aminocarbonyl, benzyloxy, hydroxy, nitrile and halogen, or substituted to form heterocycloalkenyl of C₃₋₆, containing one or more O together with substituted carbon atoms, and the substituted alkyl is substituted with one or more substituents selected from the group consisting of substituted heterocycloalkyl with benzyl of 4 to 8 atoms, containing one or more heteroatoms selected from the group consisting of N, S and O, and oxo;
L¹ is a single bond, unsubstituted or substituted linear or branched C₁₋₈ alkylene or substituted cycloalkenylene of C₄₋₆,
the substituted alkylene is substituted with one or more substituents selected from the group consisting of oxo and linear or branched C₁₋₅ alkyl, or the substituted alkylene is substituted to form cycloalkyl of C₃₋₆ together with substituted carbon, and the substituted cycloalkenylene is substituted to form aryl of C₆₋₁₀ together with substituted carbon atoms;
R² is unsubstituted or substituted heterocycloalkyl of 4 to 8 atoms, containing one or more heteroatoms selected from the group consisting of N, S and O, unsubstituted or substituted heteroaryl of 5 to1 14 atoms, containing one or more heteroatoms selected from the group consisting of N, S and O, substituted aryl of C₆₋₁₀ with one or more halogens, unsubstituted or substituted cycloalkyl of C₃₋₆, or NR^{2a}R^{2b},
the substituted heterocycloalkyl, substituted heteroaryl and substituted cycloalkyl are each independently substituted with one or more substituents selected from the group consisting of unsubstituted or substituted heteroaryl of 5 to 14 atoms with halogen, containing one or more heteroatoms selected from the group consisting of N, S and O, substituted linear or branched C₁₋₁₀ alkyl and substituted aryl of C₆₋₁₀, where the substituted alkyl is substituted with one or more substituents selected from the group consisting of heterocycloalkyl of 4 to 8 atoms, containing one or more heteroatoms selected from the group consisting of N, S and O, hydroxy and unsubstituted or substituted aryl of C₆₋₁₀ with one or more halogens, and the substituted aryl is substituted with one or more substituents selected from the group consisting of substituted linear or branched C₁₋₈ alkyl with one or more halogens and halogen,
in this case, R^{2a} and R^{2b} are each independently hydrogen, unsubstituted or substituted linear or branched C₁₋₅ alkyl, where the substituted alkyl is substituted with one or more substituents selected from the group consisting of oxo and aryl of C₆₋₁₀; and
R³ is hydrogen or linear or branched C₁₋₅ alkyl;
or if L¹ is a single bond, R² and R³ may form unsubstituted or substituted heterocycloalkyl of 4 to 8 atoms, containing one or more heteroatoms selected from the group consisting of N, S and O together with a nitrogen atom bonded, and the substituted heterocycloalkyl is substituted with substituted linear or branched C₁₋₁₀ alkyl with aryl of C₆₋₁₀.

2. The compound, an isomer thereof, a solvate thereof, a hydrate thereof, or a pharmaceutically acceptable salt thereof according to claim 1, wherein
R¹ is unsubstituted or substituted cycloalkyl of C₃₋₅, unsubstituted or substituted aryl of C₆₋₁₀, unsubstituted or substituted heteroaryl of 5 to 10 atoms, containing one or more heteroatoms selected from the group consisting of N, S and O, substituted linear or branched C₁₋₃ alkyl, or substituted cycloalkenyl of C₅₋₆ to form aryl of C₆₋₈ together with substituted carbon atoms,
the substituted cycloalkyl is substituted with linear or branched C₁₋₃ alkyl, the substituted aryl is substituted with one or more substituents selected from the group consisting of linear or branched C₁₋₅ alkoxy and halogen, or substituted to form heterocycloalkenyl of C₄₋₆ containing one or more O together with substituted carbon atoms, the substituted heteroaryl is substituted with one or more substituents selected from the group consisting of unsubstituted or substituted linear or branched C₁₋₃ alkyl with one or more halogens, unsubstituted or substituted linear or branched C₁₋₅ alkoxy with aryl of C₆₋₈, C₁₋₅ carboxy, C₁₋₅ aminocarbonyl, benzyloxy, hydroxy, nitrile and halogen, or substituted to form heterocycloalkenyl of C₄₋₆, containing one or more O together with substituted carbon atoms, and the substituted alkyl is substituted with one or more substituents selected from the group consisting of substituted heterocycloalkyl with benzyl of 4 to 6 atoms, containing one or more heteroatoms selected from the group consisting of N, S and O, and oxo;
L¹ is a single bond, unsubstituted or substituted linear or branched C₁₋₃ alkylene or substituted cycloalkenylene of C₄₋₆,
the substituted alkylene is substituted with one or more substituents selected from the group consisting of oxo and linear or branched C₁₋₃ alkyl, or the substituted alkylene is substituted to form cycloalkyl of C₃₋₅ together with substituted carbon, and the substituted cycloalkenylene is substituted to form aryl of C₆₋₈ together with substituted carbon atoms;
R² is unsubstituted or substituted heterocycloalkyl of 4 to 6 atoms, containing one or more heteroatoms selected from the group consisting of N, S and O, unsubstituted or substituted heteroaryl of 5 to 10 atoms, containing one or more heteroatoms selected from the group consisting of N, S and O, substituted aryl of C₆₋₈ with one or more halogens, unsubstituted or substituted cycloalkyl of C₃₋₆, or NR^{2a}R^{2b},
the substituted heterocycloalkyl, substituted heteroaryl and substituted cycloalkyl are each independently substituted with one or more substituents selected from the group consisting of unsubstituted or substituted heteroaryl of 5 to 10 atoms with halogen, containing one or more heteroatoms selected from the group consisting of N, S and O, substituted linear or branched C₁₋₅ alkyl and substituted aryl of C₆₋₈, where the substituted alkyl is substituted with one or more substituents selected from the group consisting of heterocycloalkyl of 4 to 6 atoms, containing one or more heteroatoms selected from the group consisting of N, S and O, hydroxy and unsubstituted or substituted aryl of C₆₋₈ with one or more halogens, and the substituted aryl is substituted with one or more substituents selected from the group consisting of substituted linear or branched C₁₋₃ alkyl with one or more halogens and halogen,
in this case, R^{2a} and R^{2b} are each independently hydrogen, unsubstituted or substituted linear or branched C₁₋₃ alkyl, where the substituted alkyl is substituted with one or more substituents selected from the group consisting of oxo and aryl of C₆₋₁₀; and
R³ is hydrogen or linear or branched C₁₋₃ alkyl;
or if L¹ is a single bond, R² and R³ may form unsubstituted or substituted heterocycloalkyl of 4 to 6 atoms, containing one or more heteroatoms selected from the group consisting of N, S and O together with a nitrogen atom bonded, and the substituted heterocycloalkyl is substituted with substituted linear or branched C₁₋₅ alkyl with aryl of C₆₋₈.

3. The compound, an isomer thereof, a solvate thereof, a hydrate thereof, or a pharmaceutically acceptable salt thereof according to claim 1, wherein
R¹ is unsubstituted or substituted cyclopropyl, unsubstituted or substituted phenyl, unsubstituted or substituted naphthyl, unsubstituted or substituted heteroaryl of 5 to 9 atoms, containing one or more heteroatoms selected from the group consisting of N, S and O, substituted linear or branched C₁₋₃ alkyl, or substituted cyclopentenyl to form phenyl together with substituted carbon atoms,
the substituted cyclopropyl is substituted with methyl, the substituted phenyl and the substituted naphthyl are each independently substituted with one or more substituents selected from the group consisting of linear or branched C₁₋₄ alkoxy, chlorine and bromine, or the substituted phenyl is substituted to form heterocyclopentenyl containing one or more O together with substituted carbon atoms, the substituted heteroaryl is substituted with one or more substituents selected from the group consisting of unsubstituted or substituted methyl with one or more halogens, unsubstituted or substituted linear or branched C₁₋₄ alkoxy with phenyl, C₁₋₃ carboxy, C₁₋₃ aminocarbonyl, benzyloxy, hydroxy, nitrile and halogen, or substituted to form heterocycloalkenyl of C₅₋₆, containing one or more O together with substituted carbon atoms, and the substituted alkyl is substituted with one or more substituents selected from the group consisting of substituted piperidinyl with benzyl and oxo;
L¹ is a single bond, unsubstituted or substituted ethylene or substituted cyclohexenylene of C₄₋₆,
the substituted ethylene is substituted with one or more substituents selected from the group consisting of oxo and methyl, or the substituted ethylene is substituted to form cyclopropyl together with substituted carbon, and the substituted cyclohexenylene is substituted to form phenyl together with substituted carbon atoms;
R² is substituted piperidinyl, substituted piperazinyl, unsubstituted or substituted pyrazol, indole, substituted phenyl with one or more chlorine atoms, substituted cyclohexyl with benzyl, or NR^{2a}R^{2b},
the substituted piperidinyl, substituted piperazinyl and substituted pyrazole are each independently substituted with one or more substituents selected from the group consisting of indole, substituted pyridine with fluorine, substituted methyl and substituted phenyl, where the substituted methyl is substituted with one or more substituents selected from the group consisting of tetrahydropyran, hydroxy and unsubstituted or substituted phenyl with one or more halogens, and the substituted phenyl is substituted with one or more substituents selected from the group consisting of substituted methyl with one or more fluorine atoms and chlorine,
in this case, R^{2a} and R^{2b} are each independently hydrogen, unsubstituted or substituted methyl, where the substituted methyl is substituted with one or more substituents selected from the group consisting of oxo and naphthyl; and
R³ is hydrogen;
or if L¹ is a single bond, R² and R³ may form piperidinyl together with a nitrogen atom bonded, and the substituted piperidinyl is substituted with substituted phenyl.

4. The compound, an isomer thereof, a solvate thereof, a hydrate thereof, or a pharmaceutically acceptable salt thereof according to claim 1, wherein
R¹ is 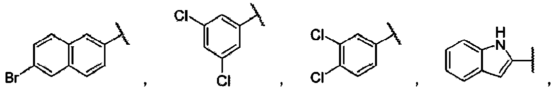 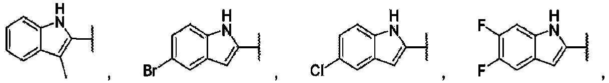 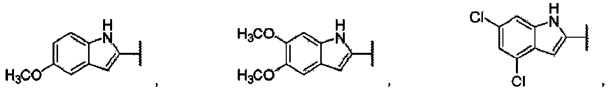 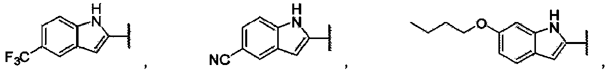 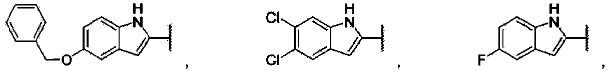 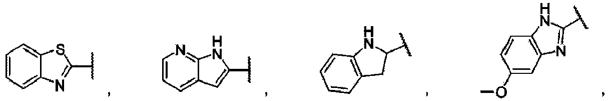 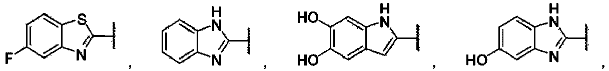 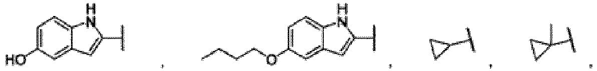 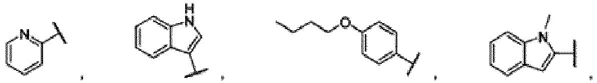 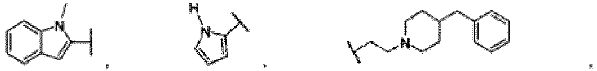 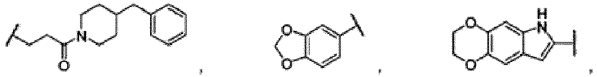 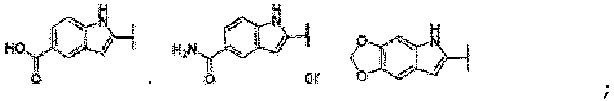
L¹ is a single bond, 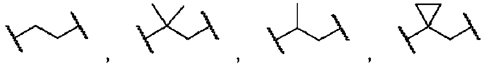 or 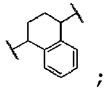
R² is 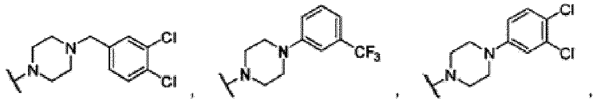 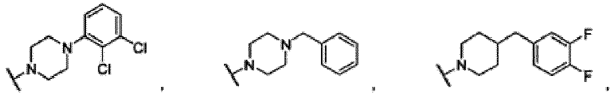 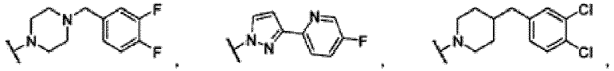 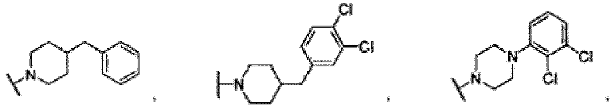 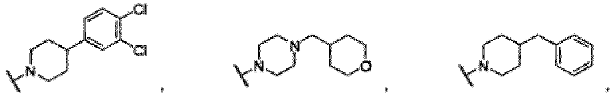 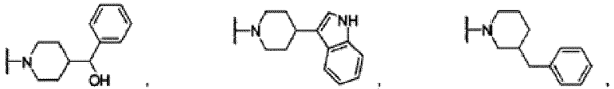 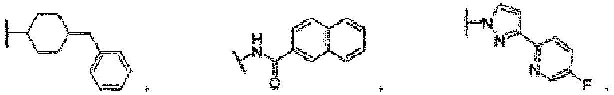 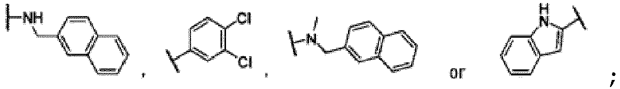 and
R³ is hydrogen; or
if L¹ is a single bond, -NR²R³ is

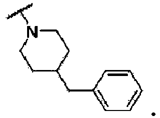

5. The compound, an isomer thereof, a solvate thereof, a hydrate thereof, or a pharmaceutically acceptable salt thereof according to claim 1, wherein the compound represented by Formula 1 is any one selected from the group consisting of the following compound group:
<1> 6-bromo-N-(2-(4-(3,4-dichlorobenzyl)piperazin-1-yl)ethyl)-2-naphthamide;
<2> 6-bromo-N-(2-(4-(3-(trifluoromethyl)phenyl)piperazin-1-yl)ethyl)-2-naphthamide;
<3> 6-bromo-N-(2-(4-(3,4-dichlorophenyl)piperazin-1-yl)ethyl)-2-naphthamide;
<4> 6-bromo-N-(2-(4-(2,3-dichlorophenyl)piperazin-1-yl)ethyl)-2-naphthamide;
<5> N-(2-(4-benzylpiperazin-1-yl)ethyl)-6-bromo-2-naphthamide;
<6> 6-bromo-N-(2-(4-(3,4-difluorobenzyl)piperidin-1-yl)ethyl)-2-naphthamide;
<7> 6-bromo-N-(2-(4-(3,4-difluorobenzyl)piperazin-1-yl)ethyl)-2-naphthamide;
<8> 6-bromo-N-(2-(3-(5-fluoropyridin-2-yl)-1H-pyrazol-1-yl)ethyl)-2-naphthamide;
<9> 6-bromo-N-(2-(4-(3,4-dichlorobenzyl)piperidin-1-yl)ethyl)-2-naphthamide;
<10> 3,5-dichloro-N-(2-(4-(3,4-dichlorobenzyl)piperidin-1-yl)ethyl)benzamide;
<11> 3,4-dichloro-N-(2-(4-(3,4-dichlorobenzyl)piperazin-1-yl)ethyl)benzamide;
<12> 3,4-dichloro-N-(2-(4-(3-(trifluoromethyl)phenyl)piperazin-1-yl)ethyl)benzamide;
<13> 3,4-dichloro-N-(2-(4-(2,3-dichlorophenyl)piperazin-1-yl)ethyl)benzamide;
<14> 3,4-dichloro-N-(2-(4-(3,4-dichlorophenyl)piperazin-1-yl)ethyl)benzamide;
<15> N-(2-(4-benzylpiperazin-1-yl)ethyl)-3,4-dichlorobenzamide;
<16> 3,4-dichloro-N-(2-(4-(3,4-difluorobenzyl)piperazin-1-yl)ethyl)benzamide;
<17> 3,4-dichloro-N-(2-(4-(3,4-dichlorobenzyl)piperidin-1-yl)ethyl)benzamide;
<18> N-(2-(4-benzylpiperidin-1-yl)ethyl)-3,4-dichlorobenzamide;
<19> N-(2-(4-(3,4-dichlorobenzyl)piperidin-1-yl)ethyl)-1H-indol-2-carboxamide;
<20> N-(2-(4-benzylpiperidin-1-yl)ethyl)-3-methyl-1H-indol-2-carboxamide;
<21> N-(2-(4-(3-(trifluoromethyl)phenyl)piperazin-1-yl)ethyl)-1H-indol-2-carboxamide;
<22> N-(2-(4-(3,4-dichlorobenzyl)piperazin-1-yl)ethyl)-1H-indol-2-carboxamide;
<23> N-(2-(4-(2,3-dichlorophenyl)piperazin-1-yl)ethyl)-1H-indol-2-carboxamide;
<24> N-(2-(4-(3,4-dichlorophenyl)piperazin-1-yl)ethyl)-1H-indol-2-carboxamide;
<25> N-(2-(4-benzylpiperazin-1-yl)ethyl)-1H-indol-2-carboxamide;
<26> (4-benzylpiperidin-1-yl)(1H-indol-2-yl)methanone;
<27> N-(2-(4-(3,4-difluorobenzyl)piperidin-1-yl)ethyl)-1H-indol-2-carboxamide;
<28> N-(2-(4-(3,4-dichlorophenyl)piperidin-1-yl)ethyl)-1H-indol-2-carboxamide;
<29> N-(2-(4-((tetrahydro-2H-pyran-4-yl)methyl)piperazin-1-yl)ethyl)-1H-indol-2-carboxamide;
<30> N-(2-(4-benzylpiperidin-1-yl)ethyl)-5-bromo-1H-indol-2-carboxamide;
<31> N-(2-(4-benzylpiperidin-1-yl)ethyl)-5-chloro-1H-indol-2-carboxamide;
<32> N-(2-(4-benzylpiperidin-1-yl)ethyl)-5,6-difluoro-1H-indol-2-carboxamide;
<33> N-(2-(4-benzylpiperidin-1-yl)ethyl)-5-methoxy-1H-indol-2-carboxamide;
<34> N-(1-(4-benzylpiperidin-1-yl)-2-methylpropan-2-yl)-1H-indol-2-carboxamide;
<35> N-(2-(4-benzylpiperidin-1-yl)ethyl)-5,6-dimethoxy-1H-indol-2-carboxamide;
<36> N-(2-(4-(hydroxy(phenyl)methyl)piperidin-1-yl)ethyl)-1H-indol-2-carboxamide;
<37> N-(2-(4-(1H-indol-3-yl)piperidin-1-yl)ethyl)-1H-indol-2-carboxamide;
<38> N-(2-(3-benzylpiperidin-1-yl)ethyl)-1H-indol-2-carboxamide;
<39> N-(2-(4-benzylpiperidin-1-yl)ethyl)-4,6-dichloro-1H-indol-2-carboxamide;
<40> N-(2-(4-benzylpiperidin-1-yl)ethyl)-5-(trifluoromethyl)-1H-indol-2-carboxamide;
<41> N-(2-(4-benzylpiperidin-1-yl)ethyl)-5-cyano-1H-indol-2-carboxamide;
<42> N-(2-(4-benzylpiperidin-1-yl)ethyl)-6-butoxy-1H-indol-2-carboxamide;
<43> N-(1-(4-benzylpiperidin-1-yl)propan-2-yl)-1H-indol-2-carboxamide;
<44> N-(4-benzylcyclohexyl)-1H-indol-2-carboxamide;
<45> N-(2-(2-naphthamido)ethyl)-1H-indol-2-carboxamide;
<46> 5-(benzyloxy)-N-(2-(4-benzylpiperidin-1-yl)ethyl)-1H-indol-2-carboxamide;
<47> N-(2-(4-benzylpiperidin-1-yl)ethyl)-5,6-dichloro-1H-indol-2-carboxamide;
<48> N-(2-(4-(3,4-dichlorobenzyl)piperidin-1-yl)ethyl)-5,6-dimethoxy-1H-indol-2-carboxamide;
<49> N-(2-(4-benzylpiperidin-1-yl)ethyl)-5-fluoro-1H-indol-2-carboxamide;
<50> N-(2-(4-benzylpiperidin-1-yl)-2-oxoethyl)-1H-indol-2-carboxamide;
<51> N-(2-(4-(3,4-dichlorobenzyl)piperidin-1-yl)ethyl)-5-fluoro-1H-indol-2-carboxamide;
<52> N-(2-(3-(5-fluoropyridin-2-yl)-1H-pyrazol-1-yl)ethyl)-1H-indol-2-carboxamide;
<53> N-(1-((4-benzylpiperidin-1-yl)methyl)cyclopropyl)-1H-indol-2-carboxamide;
<54> N-(2-((naphthalen-2-ylmethyl)amino)ethyl)-1H-indol-2-carboxamide;
<55> N-(2-(4-benzylpiperidin-1-yl)ethyl)benzo[d]thiazol-2-carboxamide;
<56> N-(2-(4-benzylpiperidin-1-yl)ethyl)-1H-pyrrolol[2,3-b]pyridin-2-carboxamide;
<57> N-(2-(4-benzylpiperidin-1-yl)ethyl)indolin-2-carboxamide;
<58> (R)-N-(2-(4-benzylpiperidin-1-yl)ethyl)indolin-2-carboxamide;
<59> N-(2-(4-benzylpiperidin-1-yl)ethyl)-5-methoxy-1H-benzo[d]imidazol-2-carboxamide;
<60> N-(2-(4-benzylpiperidin-1-yl)ethyl)-5-fluorobenzo[d]thiazol-2-carboxamide;
<61> N-(2-(4-benzylpiperidin-1-yl)ethyl)-1H-benzo[d]imidazol-2-carboxamide;
<62> N-((1R,4S)-4-(3,4-dichlorophenyl)-1,2,3,4-tetrahydronaphthalen-1-yl)-1H-indol-2-carboxamide;
<63> N-(2-(4-benzylpiperidin-1-yl)ethyl)-5,6-dihydroxy-1H-indol-2-carboxamide;
<64> N-(2-(4-benzylpiperidin-1-yl)ethyl)-5-hydroxy-1H-benzo[d]imidazol-2-carboxamide;
<65> N-(2-(4-(3,4-dichlorobenzyl)piperidin-1-yl)ethyl)-5,6-dihydroxy-1H-indol-2-carboxamide;
<66> N-(2-(4-benzylpiperidin-1-yl)ethyl)-5-hydroxy-1H-indol-2-carboxamide;
<67> N-(2-(4-benzylpiperidin-1-yl)ethyl)-5-butoxy-1H-indol-2-carboxamide;
<68> N-(2-(methyl(naphthalen-2-ylmethyl)amino)ethyl)-1H-indol-2-carboxamide;
<69> N-(2-(4-benzylpiperidin-1-yl)ethyl)cyclopropanecarboxamide;
<70> N-(2-(4-benzylpiperidin-1-yl)ethyl)-1-methylcyclopropanecarboxamide;
<71> N-(2-(4-benzylpiperidin-1-yl)ethyl)picolinamide;
<72> N-(2-(4-(1H-indol-3-yl)piperidin-1-yl)ethyl)-4-butoxybenzamide;
<73> N-(2-(4-benzylpiperidin-1-yl)ethyl)-1H-indol-3-carboxamide;
<74> N-(2-(4-benzylpiperidin-1-yl)ethyl)-1-methyl-1H-indol-2-carboxamide;
<75> N-(2-(4-benzylpiperidin-1-yl)ethyl)-1H-pyrrol-2-carboxamide;
<76> 3-(4-benzylpiperidin-1-yl)-N-(1H-indol-2-yl)propanamide;
<77> 4-(4-benzylpiperidin-1-yl)-N-(1H-indol-2-yl)-4-oxobutanamide;
<78> N-(2-(4-benzylpiperidin-1-yl)ethyl)benzo[d][1,3]dioxol-5-carboxamide;
<79> N-(2-(4-benzylpiperidin-1-yl)ethyl)-2,3-dihydro-6H-[1,4]dioxyno[2,3-f]indol-7-carboxamide;
<80> N-(2-(4-benzylpiperidin-1-yl)ethyl)-5H-[1,3]dioxo[4,5-f]indol-6-carboxamide;
<81> 2-((2-(4-benzylpiperidin-1-yl)ethyl)carbamoyl)-1H-indol-5-carboxylic acid; and
<82> N2-(2-(4-benzylpiperidin-1-yl)ethyl)-1H-indol-2,5-dicarboxamide.

6. A pharmaceutical composition for use in preventing or treating mental illness, comprising the compound represented by Formula 1 of claim 1, an isomer thereof, a solvate thereof, a hydrate thereof, or a pharmaceutically acceptable salt thereof, as an active ingredient.

7. The pharmaceutical composition according to claim 6, wherein the compound inhibits the reuptake of serotonin, norepinephrine, and dopamine.

8. The pharmaceutical composition according to claim 6, wherein the mental disorder is one or more selected from the group consisting of bulimia nervosa, mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substance-induced mood disorder, pseudo dementia, Ganger syndrome, obsessive-compulsive disorder, panic disorder, panic disorder without agoraphobia, panic disorder with agoraphobia, agoraphobia without history of panic disorder, panic attacks, memory deficit, memory loss, attention deficit hyperactivity disorder, obesity, anxiety, generalized anxiety disorder, eating disorder, Parkinson's disease, Parkinson's symptoms, dementia, aging dementia, senile dementia, Alzheimer's disease, Down syndrome, acquired immunodeficiency syndrome dementia complex, memory dysfunction in aging, specific phobia, social phobia, social anxiety disorder, post-traumatic stress disorder, acute stress disorder, chronic stress disorder, drug addiction, drug abuse, drug abuse tendency, cocaine abuse, nicotine abuse, tobacco abuse, alcohol addiction, alcoholism, pathological kleptomaniac, withdrawal syndrome due to withdrawal of intoxicating substances, pain, chronic pain, inflammatory pain, neuropathic pain, diabetic neuropathic pain, migraine, tension-type headache, chronic tension-type headache, depression-related pain, back pain, cancer pain, irritable bowel pain, irritable bowel syndrome, postoperative pain, postmastectomy pain syndrome (PMPS), poststroke pain, drug-induced neuropathy, diabetic neuropathy, pain sustained by the sympathetic nervous system, trigeminal neuralgia, toothache, facial muscle pain, phantom limb pain, anorexia, premenstrual syndrome, premenstrual dysphoric disorder, late luteal phase syndrome, posttraumatic syndrome, chronic fatigue syndrome, persistent vegetative state, urinary incontinence, stress incontinence, urge incontinence, nocturnal incontinence, sexual dysfunction, premature ejaculation, erectile difficulty, erectile dysfunction, restless legs syndrome, periodic limb movement disorder, eating disorder, anorexia nervosa, sleep disorder, pervasive developmental disorder, autism, Asperger's disorder, Rett disorder, childhood disintegrative disorder, learning disorder, motor ability disorder, mutism, trichotillomania, narcolepsy, post-stroke depression, stroke-induced brain injury, stroke-induced nerve injury, Tourette syndrome, tinnitus, tic disorder, body dysmorphic disorder, oppositional defiant disorder, and post-stroke disorder.

9. A triple reuptake inhibitor of serotonin, norepinephrine, and dopamine for use in preventing or treating mental illness, comprising the compound represented by Formula 1 of claim 1, an isomer thereof, a solvate thereof, a hydrate thereof, or a pharmaceutically acceptable salt thereof, as an active ingredient.

10. A health functional food composition for use in preventing or improving mental illness, comprising the compound represented by Formula 1 of claim 1, an isomer thereof, a solvate thereof, a hydrate thereof, or a pharmaceutically acceptable salt thereof, as an active ingredient.
